(19) Europäisches Patentamt
European Patent Office
Office européen des brevets

(11) EP 2 208 737 B1

(12) **EUROPEAN PATENT SPECIFICATION**

(45) Date of publication and mention of the grant of the patent:
**05.07.2017 Bulletin 2017/27**

(51) Int Cl.:
*C07K 14/47* (2006.01) *C12N 15/12* (2006.01)
*C12N 5/10* (2006.01) *A61K 38/17* (2006.01)
*A61K 39/395* (2006.01) *A61P 35/00* (2006.01)

(21) Application number: **10158162.7**

(22) Date of filing: **02.05.2008**

(54) **Complement factor H-derived short consensus repeat-antibody constructs**

Komplementierte Faktor-H-abgeleitete Kurzkonsenswiederholung-Antikörperkonstrukte

Constructions d'anticorps et de répétitions de faible consensus dérivées du facteur H du complément

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **03.05.2007 EP 07008954**

(43) Date of publication of application:
**21.07.2010 Bulletin 2010/29**

(62) Document number(s) of the earlier application(s) in accordance with Art. 76 EPC:
**08749298.9 / 2 152 736**

(73) Proprietor: **Lysomab GmbH**
**6130 Schwaz (AT)**

(72) Inventor: **Stoiber, Heribert**
**6130, Schwaz (AT)**

(74) Representative: **Vossius & Partner**
**Patentanwälte Rechtsanwälte mbB**
**Siebertstrasse 3**
**81675 München (DE)**

(56) References cited:
**EP-A- 0 854 150** **US-A1- 2002 068 059**
**US-A1- 2005 112 139** **US-A1- 2006 246 066**

- **REITER Y ET AL: "TARGETING OF COMPLEMENT TO TUMOR CELLS BY HETEROCONJUGATES COMPOSEDANTIBODIES AND OF THE COMPLEMENT COMPONENT C3B" JOURNAL OF IMMUNOLOGY, THE WILLIAMS AND WILKINS CO. BALTIMORE, vol. 142, no. 8, 15 April 1989 (1989-04-15), pages 2771-2777, XP001000393 ISSN: 0022-1767**
- **YEFENOF E ET AL: "Potentiation of NK cytotoxicity by antibody-C3b/iC3b heteroconjugates." JOURNAL OF IMMUNOLOGY (BALTIMORE, MD. : 1950) 15 FEB 1990, vol. 144, no. 4, 15 February 1990 (1990-02-15), pages 1538-1543, XP002494607 ISSN: 0022-1767**
- **REITER Y ET AL: "Killing of human tumor cells by antibody C3b conjugates and human complement" TARGETED DIAGNOSIS AND THERAPY, DEKKER, NEW YORK, NY, US, vol. 2, 1 January 1989 (1989-01-01), pages 119-135, XP009105210 ISSN: 1046-1906**
- **PINTÉR C ET AL: "Interference with complement regulatory molecules as a possible therapeutic strategy in HIV infection." EXPERT OPINION ON INVESTIGATIONAL DRUGS FEB 2000, vol. 9, no. 2, February 2000 (2000-02), pages 199-205, XP002494605 ISSN: 1354-3784**
- **JUHL H ET AL: "ADDITIVE CYTOTOXICITY OF DIFFERENT MONOCLONAL ANTIBODY-COBRA VENOM FACTOR CONJUGATES FOR HUMAN NEUROBLASTOMA CELLS" IMMUNOBIOLOGY, URBAN UND FISCHER VERLAG, DE, vol. 197, 1 January 1997 (1997-01-01), pages 444-459, XP002908499 ISSN: 0171-2985**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 208 737 B1

- BJORGE L ET AL: "Minimal residual disease in ovarian cancer as a target for complement-mediated mAb immunotherapy." SCANDINAVIAN JOURNAL OF IMMUNOLOGY MAY 2006 LNKD- PUBMED:16640659, vol. 63, no. 5, May 2006 (2006-05), pages 355-364, XP002584371 ISSN: 0300-9475
- STOIBER H ET AL: "Enhancement of complement-mediated lysis by a peptide derived from SCR 13 of complement factor H" IMMUNOBIOLOGY, FISCHER, STUTTGART, DE, vol. 203, no. 4, 1 May 2001 (2001-05-01), pages 670-686, XP009101727 ISSN: 0171-2985
- STOIBER H ET AL: "Role of complement in the control of HIV dynamics and pathogenesis" VACCINE, BUTTERWORTH SCIENTIFIC. GUILDFORD, GB, vol. 21, 1 June 2003 (2003-06-01), pages S77-S82, XP004425460 ISSN: 0264-410X
- STOIBER H ET AL: "Complement-opsonized HIV: the free rider on its way to infection" MOLECULAR IMMUNOLOGY, ELMSFORD, NY, US, vol. 42, no. 2, 1 February 2005 (2005-02-01), pages 153-160, XP004602274 ISSN: 0161-5890
- PANGBURN M K ET AL: "LOCALIZATION OF THE HEPARIN-BINDING SITE ON COMPLEMENT FACTOR H" JOURNAL OF BIOLOGICAL CHEMISTRY, AMERICAN SOCIETY OF BIOLOCHEMICAL BIOLOGISTS, BIRMINGHAM,; US, vol. 266, no. 25, 5 September 1991 (1991-09-05), pages 16847-16853, XP002035067 ISSN: 0021-9258

## Description

The disclosure generally relates to a complement activating construct comprising a complement factor H-derived short consensus repeat (fH-derived SCR) and a binding molecule which specifically recognizes a pathogen. More specifically, the fH-derived SCR is selected from the group consisting of SCR7, SCR9, SCR13 SCR18-20 and artificial SCR (aSCR). Furthermore, an in vivo method for screening complement-based approaches for the treatment of the prevention, treatment or amelioration of an infection with a pathogen or a pathological condition associated with an infection with a pathogen is described. The invention relates to a complement activating construct comprising a complement factor H-derived short consensus repeat (fH-derived SCR) and a binding molecule which specifically recognizes or binds to a cancer cell, malignant cell or tumor cell, wherein said fH-derived SCR comprises a polypeptide that is capable of binding heparin.
Animal viruses such as animal RNA viruses can be inactivated and lysed by human serum (see: Takeuchi (1994) J Virol. 68: 8001-7; Cooper (1976) J Exp Med. 144: 970-84; Bartholomew (1978) J Immunol. 12.1: 1748-51; Bartholomew (1978) J Exp Med. 147: 844-53; Sherwin (1978) Int J Cancer. 21(1): 6-11; Jensen (1979) Hamatol Bluttransfus. 23: 501-3; Kobilinsky (1980) Infect Immun. 29(1): 165-70); Dierich (1996) in: Immunology of HIV infection, editor: S. Gupta, New York, Plenum Press, 365-376). This neutralising property is mediated by human complement. Also human retroviruses such as human T-lymphotropic virus (HTLV) or human immunodeficiency virus (HIV) activate the complement system. These viruses trigger the classical pathway already during the acute phase of infection, resulting in a deposition of C3-fragments on the viral surface (Ebenbichler (1991) J Exp Med. 174: 1417-24; Stoiber (2001) Immunol Reviews 180:168-76; Saifuddin (1995) AIDS Res Hum Retroviruses 11: 115-22; Stoiber (1997) Annu Rev Immunol. 15: 649-674).

Although inactivated by animal sera, activation of the complement cascade by HTLV or HIV seems to result only in partial virolysis when incubated with human serum (Stoiber (1997) loc. cit.; Sullivan (1996) J Immunol 157: 1791-1798; Dierich (1996) Nature Med 2: 153-155). Responsible for this intrinsic resistance against human complement are host cell-derived proteins, which are acquired by HIV during the budding process (Frank (1996) AIDS 10: 1611-20). Among them are regulators of complement activation (RCA) such as CD46 (MCP), CD55 (DAF) or CD59 which down-regulate the complement system (Montefiori (1994) Virology 205: 82-92; Saifuddin (1995) J Exp Med. 182: 501-509; Schmitz (1995) J Clin Invest. 96: 1520-6; Marschang (1995) Eur J Immunol. 25: 285-290; Takefman (1998) Virology 46: 370-378).

The intrinsic resistance of retroviruses against complement of their natural host seems to represent a general phenomenon. A mouse retrovirus is resistant to mouse serum, but is efficiently destroyed by complement of other species, such as human, feline or sheep (Spear (1991) Immunology 73: 377-82 and own unpublished observations). Similarly, as discussed above, HIV is not affected by human complement, but is lysed by animal sera within minutes. Thus, retroviruses have adapted similar but species specific protection mechanisms to keep complement activation in their natural host under the threshold necessary to induce virolysis. Therefore, opsonised virions accumulate in retrovirus-infected hosts.

Previously, it has been shown that viruses bind complement factor H (fH) in fluid phase. Since fH is a negative regulator of complement activation (RCA), said fH binding promotes significant protection against complement-induced lysis of virus particles such as HIV (Stoiber (1996) J Exp Med. 183: 307-310). The crucial role of fH for protection of the virus is evident, since incubation of HIV with fH-depleted sera results in up to 80% of complement-dependent virolysis in the presence of HIV-specific antibodies (Stoiber (1996) loc. cit.).

A common RCA motif is a repeat of about 60 amino acids (aa), i.e. short consensus repeats (SCRs) or complement consensus repeats (CCR) (Takeuchi (1994) loc.cit.). fH is organised in 20 SCR units (Prodinger (2004) in: Fundamental Immunology, editor: WE. Paul, Lippincott-Raven Publishers). The first 5 SCRs of fH have "decay-accelerating activity" serving as a cofactor for C3b inactivation (Prodinger (2004) loc. cit.). SCR 7, 9, 18-20 and probably SCR 13 mediate the binding of fH to negative charged surface elements such as heparin (Prodinger (2004) loc. cit.; Cheng (2006) Mol Immunol. 43: 972-9). Binding of fH to negatively charged host cells contributes to the protection of host cells against damage induced by the host's own complement. Reiter (1989) Journal of Immunology 142: 2771-2777 and Reiter (1989) Targeted Diagnosis and Therapy 2: 119-135 describe a heteroconjugate composed of complement factor C3b and a murine monoclonal antibody directed to transferrin.

Yefenof (1990) Journal of Immunology 144: 1538-1543 discloses heteroconjugates composed either C3b or iC3b, each coupled to an anti-transferrin antibody, respectively.

US-A1 2002/68059 describes the preparation of modified C3 convertases which are insensitive against, inter alia, inactivation by complement regulators H and I.

US-A1 2006/246066 describes the preparation of a fusion protein consisting of complement regulators such as DAF or factor H and the Fc-fragment of an 1g.

Pintér (2000) Expert Opinion of Investigational Drugs 9: 199-205 shows that the N-terminal moiety of SCR13 (derived from factor H) binds competitively to HIV-infected cells and that it leads to the lysis of the infected cells.

EP-A1 0 854 150 describes reagents for the treatment of pathogen-induced disorders, whereby said reagents are peptide chains of less than 100 amino acids and are able to bind to a CFH (fH)-binding region. However, it could be shown that these peptides have still certain disadvantages and better treatment options are desired.

The technical problem underlying the present invention is to provide efficacious means and methods for prevention, treatment or amelioration of an infection with a pathogen or of a pathological condition associated with an infection with a pathogen (not claimed) or of a condition associated with a proliferative disease, like cancer. The solution to the above technical problem is achieved by providing the embodiments characterized in the claims. Accordingly, this disclosure generally relates to a short consensus repeat-antibody construct (SCR-Ab) comprising:

(a) a complement factor H-derived short consensus repeat (fH-derived SCR); and
(b) a binding molecule that specifically recognizes a pathogen,

wherein said fH-derived SCR comprises a polypeptide that is capable of binding heparin.

In one embodiment, said fH-derived SCR is selected from the group consisting of SCR7, SCR9, SCR13, SCR18-20 and artificial SCR (aSCR)or a functional fragment of said fH-derived SCR7, SCR9, SCR13, SCR18-20 and aSCR.

The SCR-Ab constructs described herein comprise a binding molecule that specifically recognizes a pathogen additionally to the herein defined complement factor H-derived short consensus repeat. As documented herein below and in the appended examples it was, in accordance with this disclosure, surprisingly found that the inventive SCR-Ab constructs are capable of lysing pathogens in vitro in a highly unexpected manner. In contrast thereto, SCR-derived polypeptides that do not comprise a pathogen-specific binding molecule failed to induce lysis of the pathogen under the same conditions. Accordingly, the embodiments of the present disclosure overcome the disadvantages of the prior art, like EP-A1 0 854 150. It was also found that the herein described SCR-Ab constructs are useful in the treatment of an infection with a pathogen in vivo. As documented in the examples SCR constructs that lacked pathogen-specific binding molecule or SCR constructs that are not linked to a pathogen-specific binding molecule are much less efficacious and partially non-functional. The inventive constructs are also useful in the medical intervention of cancer and/or of proliferative disorders, whereby in these embodiments binding molecules are to be employed that specifically bind to or recognize a cancer cell, tumour cell or a malignant cell; see also items below. Therefore, it is also envisaged that the inventive pharmaceutical concept described herein may be employed in a medical setting where it is desired to inhibit and/or eliminate pathogen infected host cells or malignant cells, like cancer cells, malignant cells or tumour cells.

Therefore, the invention relates to short consensus repeat-antibody construct (SCR-Ab) comprising:

(a) a complement factor H-derived short consensus repeat (fH-derived SCR); and
(b) an antibody molecule that specifically recognizes a cancer cell,

wherein said fH-derived SCR comprises a polypeptide that is capable of binding heparin, wherein said complement factor H-derived short consensus repeat (fH-derived SCR) and said antibody molecule are covalently linked or comprised in a single chain multi-functional polypeptide and wherein said fH-derived SCR is selected from the group consisting of:

(i) a polypeptide encoded by the amino acid sequence comprised in SEQ ID NO: 4, SEQ ID NO: 6, SEQ ID NO: 8, SEQ ID NO: 22, SEQ ID NO: 24, SEQ ID NO: 26, SEQ ID NO: 30 or SEQ ID NO: 32;
(ii) a polypeptide encoded by the amino acid sequence that is at least 60% identical to the amino acid sequence comprised in SEQ ID NO: 4, SEQ ID NO: 6, SEQ ID NO: 8, SEQ ID NO: 22, SEQ ID NO: 24, SEQ ID NO: 26, SEQ ID NO: 30 or SEQ ID NO: 32 and wherein said polypeptide is capable of binding heparin;
(iii) a polypeptide encoded by the polynucleotide sequence as comprised in SEQ ID NO: 3, SEQ ID NO: 5, SEQ ID NO: 7, SEQ ID NO: 21, SEQ ID NO: 23, SEQ ID NO: 25, SEQ ID NO: 29 or SEQ ID NO: 31;
(iv) a polypeptide encoded by the complementary sequence of a polynucleotide that is able to hybridize with the polynucleotide as comprised in SEQ ID NO: 3, SEQ ID NO: 5, SEQ ID NO: 7, SEQ ID NO: 21, SEQ ID NO: 23, SEQ ID NO: 25, SEQ ID NO: 29 or SEQ ID NO: 31 and wherein said polypeptide is capable of binding heparin; and.
(v) a polypeptide encoded by a nucleic acid molecule which is at least 60% identical to the nucleic acid sequence as comprised in SEQ ID NO: 3, SEQ ID NO: 5, SEQ ID NO: 7, SEQ ID NO: 21, SEQ ID NO: 23, SEQ ID NO: 25, SEQ ID NO: 29 or SEQ ID NO: 31 and wherein said polypeptide is capable of binding heparin.

In one embodiment, said fH-derived SCR is selected from the group consisting of SCR7, SCR9, SCR13 and SCR18-20 or a functional fragment of said fH-derived SCR7, SCR13, SCR18-20 and artificial SCR (aSCR).

Monoclonal Antibodies (mAbs) offer a promising perspective for treatment of certain types of tumours (Reichert (2007) Nat Rev Drug Discov 6:349-356). mAbs targeting angiogenesis-regulating growth factors, such as VEGF or tumour antigens, enable a more potent therapeutic efficacy when combined with conventional drugs, thereby unifying classical chemotherapeutic schemes with new immunotherapeutic concepts (i.e. chemoimmunotherapy). Targeting Her2/Neu in breast cancer, CD20 in lymphoma as well as EGFR and VEGF in gastrointestinal cancer has already been proven to prolong survival as compared to chemotherapy alone and therefore represents an important additional treatment option for these cancer types (Reichert (2007) loc.cit.). Moreover, data from clinical studies testing an antibody directed against an extracellular epitope of Her2/Neu (Trastuzumab) in the adjuvant setting on Her2/neu overexpressing breast cancers after surgical resection of the primary tumour demonstrated a ~30 percent reduction in the risk of death, which was highly significant when compared to adjuvant chemotherapy alone.

These data support the concept that mAbs are most promising therapeutics when applied in patients with minimal tumour load (i.e. with micro-metastases or residual tumour cells after resection of the solid primary tumour). Despite the outstanding potency of mAb, their clinical efficacy in human cancer is far from optimal (Holz (1998) Recent Results Cancer Res 146:214-218). Therefore, several strategies including the application of radioimmuno-conjugates or bispecific antibodies are currently tested in pre-clinical models as well as in clinical trials to enhance their clinical efficacy (Reichert (2007) loc.cit.).

The binding of mAbs to tumour cells is thought to induce complement activation which may result in the destruction of tumour cells by complement-dependent cellular cytotoxicity (CDCC) and complement-mediated lysis (Durrant (2001). Curr Opin Investig Drugs 2:959-966). However these complement-mediated effector functions are limited, as tumours, similar to normal cells, are protected from complement-induced damage by regulators of complement activation (RCAs), which are over-expressed by certain tumours (Durrant (2001) loc.cit.). Among these RCAs are membrane anchored regulators such as CD46 (MCP), CD55 (DAF) or CD59 and in addition, RCAs in fluid phase, like factor H (fH) (Bjørge (2005) Br J Cancer 9:895-905; Prodinger (2004) Complement. In: Fundamental Immunology. Ed.: Paul WE. Lippincott-Raven Publishers).

The crucial role of fH for protection of several tumours is shown for ovarian, lung and colonic cancer cells (Prodinger (2004) loc.cit.; Ajona (2007) J Immunol 178:5991-5997; Fedarko (2000) J Biol Chem 275(22):16666-16672; Kinders (1998) Clin Cancer Res 4:2511-2520).

As described, a common motif of RCAs is a repeat of about 60 amino acids (aa), called short consensus repeats (SCRs) or complement consensus repeats (Prodinger (2004) loc.cit.). fH is organised in 20 SCR units (Prodinger (2004) loc.cit.). The first 5 SCRs of fH exhibit "decay - accelerating activity" and promote C3b inactivation (Prodinger (2004) loc.cit.). SCR 7, 9, 18-20 and probably SCR 13 mediate the binding of fH to negative charged surface elements such as heparin sulfates (Prodinger (2004) loc.cit.). Binding of fH to negatively charged host cells contributes to the protection against damage induced by the host's own complement.

As not only RCAs but also negatively charged surface structures are up-regulated in certain tumours (Fedarko (2000) loc.cit.), fH binds with high preference to the tumour surface and therefore contributes to the protection against complement-induce lysis. As exemplified herein, blocking of RCAs such as fH increases the efficacy of mAb therapy.

As used herein, the term "short consensus repeat", "SCR" "complement consensus repeat" or "CCR" relates to the consensus repeat motifs which can be deduced from polynucleotide or amino acid sequences encoding regulators of complement activity (RCA). Particularly, SCR which mediate the binding of the RCA to negatively charged elements such as heparin may be of relevance in the context of the current disclosure. In one embodiment, the SCR comprised in the SCR-Ab construct described herein may be a fH-derived SCR selected from the group consisting of SCR7, SCR9, SCR13, SCR18-20 and artificial SCR (aSCR) or a functional fragment thereof. Yet, it is of note that SCRs also may have "decay-accelerating activity" which is associated with the down-regulating of complement activation.

As used herein, the term "artificial short consensus repeat", "artificial SCR" or "aSCR" relates to amino acid sequences (or polynucleotide sequences encoding such amino acid sequences) that are non-naturally occurring regulators of complement activity (RCA). "Artificial SCRs" as used herein mediate the binding of a non-naturally occurring RCA to negatively charged elements such as heparin. Accordingly, "aSCR" represents, inter alia, an artificial derivate of fH or a related protein of the complement regulator protein family, which is capable of binding to heparin. Amino acid motifs which function as heparin binding sites and which contribute to the binding an "artificial SCR" to negatively charged surface elements are known in the art; see inter alia Smith (2000) J Virol 74:5659-5666 and Ghebremariam (2005) Ann N Y Acad Sci 1056:113-122. Accordingly, "artificial SCR"/"aSCR" may comprise a repetitive sequence which contains clusters of basic amino acid residues such as R or K resulting in the sequence motif "R/K-X-R/K", wherein "X" is any naturally occurring amino acid. Furthermore, it is described in the art that fH-derived proteins capable of binding to heparin are made up of more than 9% positively charged amino acids and have an overall isoelectric point (pI) of greater than 7.0 (Smith (2000) J Virol 74:5659-5666). The capability of heparin-binding artificial SCRs or fragments thereof to induce lysis of a pathogen can be tested by using the in vitro lysis assays as described herein below and in particular in the appended examples.

Taken together, artificial SCRs (or fragments thereof) according to the present disclosure and comprised in the inventive short consensus repeat-antibody construct (SCR-Ab) may comprise (i) repetitive surface-exposed K/R-X-K/R motifs (wherein "X" is any naturally occurring amino acid); (ii) comprise more than 9% positively charged amino acids; and/or (iii) have an overall isoelectric point (pI) of greater than 7.0. One example of an artificial SCR described herein may comprise the amino acid sequence as shown herein below in SEQ ID NO: 32 or may be encoded by a polynucleotide comprised in SEQ ID NO: 31. Yet, the person skilled in the art is readily in the position to use and to obtain further artificial SCR useful in the SCR-Ab constructs as provided herein, for instance by employing the herein described in vitro assays for testing heparin binding and/or the herein described in vitro lysis assays.

As used herein, the term "binding molecule" as comprised in the inventive short consensus repeat-antibody construct (SCR-Ab) described herein relates to a molecule that is able to specifically interact with (a) potential binding partner(s) so that is able to discriminate between said potential binding partner(s) and a plurality of different molecules as said potential binding partner(s) to such an extent that, from a pool of said plurality of different molecules as potential binding partner(s), only said potential binding partner(s) is/are bound, or is/are significantly bound. Methods for the measurement of binding of a binding molecule to a potential binding partner are known in the art and can be routinely performed e.g. by using a Biacore apparatus.

In EP-A1 0 854 150 it is taught that fH-derived pathogen-binding peptide chains preferably comprise a SCR13 region-derived sequence, whereas the SCR7 region of fH is to be deleted. EP-A1 0 854 150 does not provide for or hint to SCR-Ab constructs as described herein. Surprisingly (and in contrast to this prior teaching), the SCR-Ab constructs described herein may also comprise other fH-derived SCR sequences, including, inter alia, SCR7, SCR9, SCR18-20 and artificial SCR (aSCR).

In one embodiment, the fH-derived SCR as comprised in the SCR-Ab construct described herein may comprise the amino acid sequence as shown herein below in SEQ ID NOs: 4, 22, 24, 6, 26, 8, 30 or 32 or a functional fragment thereof:

STKVRKCVFHYVENGDSAYWEKVYVQGQSLKVQCYNGYSLQNGQDTMTCTENGW SPPPKCIIL (SEQ ID NO:4; mouse SCR7);

EFLRKCYFPYLENGYNQNYGRKFVQGKSIDVACHPGYALPKAQTTVTCMENGWSPT PRCIPL (SEQ ID NO: 22; human SCR7);

EFKSCDIPVFMNARTKNDFTWFKLNDTLDYECHDGYESNTGSTTGSIVCGYNGWSDL PICYPL (SEQ ID NO: 24; human SCR9);

STKATDQLEKCRVLKSTGIEAIKPKLTEFTHNSTMDYKCRDKQEYERSICINGKWDPE PNCTIL (SEQ ID NO:6; mouse SCR13);

GTDKLKKCKSSNLIILEEHLKNKKEFDHNSNIRYRCRGKEGWIHTVCINGRWDPEVN CSMGL (SEQ ID NO: 26; human SCR13);

KDNSCVDPPHVPNATIVTRTKNKYLHGDRVRYECNKPLELFGQVEVMCENGIWTEK PKCRDSTGKCGPPPPIDNGDITSLSLPVYEPLSSVEYQCQKYYLLKGKKTITCTNGKW SEPPTCLHACVIPENIMESHNIILKWRHTEKIYSHSGEDIEFGCKYGYYKARDSPPFRT KCINGTINYPTCV (SEQ ID NO:8; mouse SCR18-20)

EFDTSCVNPPTVQNAYIVSRQMSKYPSGERVRYQCRSPYEMFGDEEVMCLNGNWTEPPQCKDSTGKCGPPPPIDNGDITSFPLSVYAPASSVEYQCQNLYQLEGNKRITCRNGQWSEPPKCLHPCVISREIMENYNIALRWTAKQKLYSRTGESVEFVCKRGYRLSSRSHTLRTTCWDGKLEYPTCAKRPL (SEQ ID NO:30; human SCR18-20); and

SMLINLGAHKSGSSSGRKKYGSKRKKSGSSSGRKKYGSKRKKSGSSSGSTRKKYGSKRKKSGSSSGRKKYGSKRKKSGSSSGSTRSTSSRIEGRGSGHHHHHHGSGHHHHHHAA

(SEQ ID NO:32; illustrative artificial SCR).

Said amino acid sequences SEQ ID NOs: 4, 22, 24, 6, 26, 8, 30 and 32 may be encoded by a polynucleotide comprised in SEQ ID NOs: 3, 21, 23, 5, 25, 7, 29 and 31, respectively:

gaattctcgaccaaagtgcgcaaatgtgtgttccactacgtggaaaacggtgatagcgcgtactgggaaaaagtgtatgttcagggccagagcctgaaagtgcagtgctataacggctatagcctgcagaatggccaggataccatgacctgcaccgaaaatggttggagcccgccgccgaaatgtattattctcgatctaga (SEQ ID NO:3; mouse SCR7);

gaattcctcagaaaatgttattttccttatttggaaaatggatataatcaaaattatggaagaaagtttgtacagggtaaatctatagacgttgcctgccatcctggctacgctcttccaaaagcgcagaccacagttacatgtatggagaatggctggtctcctactcccagatgcatccctctaga (SEQ ID NO: 21; human SCR7);

gaattcaaatcttgtgatatcccagtatttatgaatgccagaactaaaaatgacttcacatggtttaagctgaatgacacattggactatgaatgccatgatggttatgaaagcaatactggaagcaccactggttccatagtgtgtggttacaatggttggtctgatttacccatatgttatcctctaga (SEQ ID NO: 23; human SCR9);

gaattctcgaccaaagcgaccgatcagctggaaaaatgccgcgttctgaaaagcaccggcatcgaagcgattaaaccgaaactgaccgaatttacccacaacagcaccatggattacaaatgccgcgataaacaggaatatgaacgcagcatttgcatcaacggcaaatgggatccggaaccgaattgcaccattctcgatctaga (SEQ ID NO:5; mouse SCR13);

ggtaccgataaacttaagaagtgcaaatcatcaaatttaattatacttgaggaacatttaaaaaacaagaaggaattcgatcataattctaacataaggtacagatgtagaggaaaagaaggatggatacacacagtctgcataaatggaagatgggatccagaagtgaactgctcaatgggtctaga (SEQ ID NO: 25; human SCR13);

atcgatgaaagataacagctgcgttgatccgccgcatgttccgaatgcgaccattgtgacccgcaccaaaaacaaatatctgcacggc
gatcgtgtgcgttatgaatgcaacaaaccgctggaactgtttggtcaggttgaagtgatgtgcgaaaacggcatctggaccgaaaaacc
gaaatgccgtgatagcaccggtaaatgtggtccgccgccgccgattgataatggcgatatcaccagcctgagcctgccggtttatgaac
cgctgagcagcgtggaatatcagtgccagaaatattatctgctgaaaggcaaaaaaaccatcacctgcaccaacggtaaatggagcga
accgccgacctgtctgcatgcgtgtgtgattccggaaaacatcatggaaagccacaacatcattctgaaatggcgccacaccgaaaaa
atctatagccacagcggcgaagatattgaattcggctgtaaatatggctattacaaagcgcgtgatagcccgccgtttcgtaccaaatgc
atcaacggcaccattaactatccgacctgcgtgcgtctaga (SEQ ID NO:7; mouse SCR18-20);

gaattcgacacctcctgtgtgaatccgcccacagtacaaaatgcttatatagtgtcgagacagatgagtaaatatccatctggtgagaga
gtacgttatcaatgtaggagcccttatgaaatgtttggggatgaagaagtgatgtgtttaaatggaaactggacggaaccacctcaatgc
aaagattctacaggaaaatgtgggccccctccacctattgacaatggggacattacttcattcccgttgtcagtatatgctccagcttcatc
agttgagtaccaatgccagaacttgtatcaacttgagggtaacaagcgaataacatgtagaaatggataatggtcagaaccaccaaaat
gcttacatccgtgtgtaatatcccgagaaattatggaaaattataacatagcattaaggtggacagccaaacagaagctttattcgagaac
aggtgaatcagttgaatttgtgtgtaaacggggatatcgtctttcatcacgttctcacacattgcgaacaacatgttgggatgggaaactgg
agtatccaacttgtgcaaaaagacctctaga (SEQ ID NO:29; human SCR18-20); and

atcgatgttaattaacctaggtgcgcacaagtctggttcttcctccggtagaaagaaatatggttccaagagaaagaagtctggatcctctt
ctggaagaaagaaatacggtagtaagagaaaaaaatccggttcctcctccggaagtactagaaaaaagtacggaagtaaaagaaaga
agtccggtagttcctcaggaagaaagaagtacggttcaaaaagaaaaaagagtggatcctcctctggtagtactcgatcgactagttcg
cgaattgaaggtagaggttctggtcatcatcatcaccatcacggttctggacatcaccaccatcatcatgcggccgc (SEQ ID NO:31; illustrative artificial SCR).

It is evident for the skilled artisan that the disclosure is not limited to the specific short consensus repeat-antibody construct (SCR-Ab) sequences as provided herein.

In one embodiment, the fH-derived SCR as comprised in the SCR-Ab construct described herein may comprise a polypeptide encoded by the complementary sequence of a polynucleotide that is able to hybridize, preferably under stringent conditions with the polynucleotide as comprised in the above described SEQ ID NOs: 3, 21, 23, 5, 25, 7, 29 and 31 and wherein said SCR is capable of binding heparin.

In another embodiment, the fH-derived SCR as comprised in the SCR-Ab construct described herein may comprise a polypeptide encoded by the amino acid sequence that is at least 60% identical to the amino acid sequence as comprised in SEQ ID NO: 4, 22, 24, 6, 26, 8, 30 or 32 or is encoded by a nucleic acid molecule which is at least 60% identical to the nucleic acid sequence as comprised in SEQ ID NO: 3, 21, 23, 5, 25, 7, 29 or 31 and wherein said polypeptide is capable of binding heparin.

In yet another embodiment, the fH-derived SCR as comprised in the SCR-Ab construct described herein may comprise an ortholog of the polypeptide encoded by the amino acid as comprised in SEQ ID NO: 4, 22, 24, 6, 26, 8 or 30 or is encoded by a nucleic acid molecule which is an ortholog of the nucleic acid sequence as comprised in SEQ ID NO: 3, 21, 23, 5, 25, 7 or 29 and wherein said polypeptide is capable of binding heparin.

Methods for identifying orthologs of a given polypeptide are well known in the art including the herein described hybridization methods.

In yet another embodiment, the fH-derived SCR as comprised in the SCR-Ab construct described herein may comprise a polypeptide encoded by the amino acid sequence comprised in SEQ ID NO: 4 or SEQ ID NO: 22 or a functional fragment thereof.

In a most preferred embodiment, the basic amino acid residues as comprised in the amino acid sequence encoding the fH-derived SCR as comprised in the SCR-Ab construct described herein are not exchanged. Basic amino acid residues are lysine, arginine and histidine.

Methods which are suitable for testing whether a SCR or a fragment thereof is capable of binding heparin are known in the art. Binding of fH is mediated, inter alia, through binding of the SCRs to negative surface elements such as heparin (Prodinger (2004) loc. cit.; Cheng (2006) loc. cit.). The identification of fH-derived SCRs or functional fragments that are useful in the context of the present,disclosure, therefore, may be achieved by using the heparin binding assay as described herein below in the Examples. An example of a further fH derived SCR that is capable of binding heparin is SCR9. The capability of heparin-binding SCRs or fragments thereof to induce lysis of a pathogen can be tested by using the in vitro lysis assays as described herein below in the examples. Yet, the person skilled in the art is aware that other SCR binding assays or in vitro lysis assays may also be useful for testing fH-derived SCR polypeptide sequences for their use in the inventive SCR-Ab constructs as described herein.

The term "hybridization" or "hybridizes" as used herein may relate to hybridizations under stringent or non-stringent conditions. If not further specified, the conditions are preferably non-stringent. Said hybridization conditions may be established according to conventional protocols described, for example, in Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as 0.1 x SSC, 0.1% SDS at 65°C. Non-stringent hybridization conditions for the detection of homologous or not exactly complementary sequences may be set at 6 x SSC, 1% SDS at 65°C. As is well known, the length of the probe and the composition of the nucleic acid to be determined constitute further parameters of the hybridization conditions. Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Hybridizing nucleic acid molecules also comprise fragments of the above described molecules. Such fragments may represent nucleic acid sequences which code for a fH-derived SCR or a functional fragment thereof which have a length of at least 12 nucleotides, preferably at least 15, more preferably at least 18, more preferably of at least 21 nucleotides, more preferably at least 30 nucleotides, even more preferably at least 40 nucleotides and most preferably at least 60 nucleotides. Furthermore, nucleic acid molecules which hybridize with any of the aforementioned nucleic acid molecules also include complementary fragments, derivatives and allelic variants of these molecules. Additionally, a hybridization complex refers to a complex between two nucleic acid sequences by virtue of the formation of hydrogen bonds between complementary G and C bases and between complementary A and T bases; these hydrogen bonds may be further stabilized by base stacking interactions. The two complementary nucleic acid sequences hydrogen bond in an anti-parallel configuration. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed). The terms "complementary" or "complementarity" refer to the natural binding of polynucleotides under permissive salt and temperature conditions by base-pairing. For example, the sequence "A-G-T" binds to the complementary sequence "T-C-A". Complementarity between two single-stranded molecules may be "partial", in which only some of the nucleic acids bind, or it may be complete when total complementarity exists between single-stranded molecules. The degree of complementarity between nucleic acid strands has significant effects on the efficiency and strength of hybridization between nucleic acid strands. This is of particular importance in amplification reactions, which depend upon binding between nucleic acids strands.

The term "hybridizing sequences" preferably refers to sequences which display a sequence identity of at least 40%, preferably at least 50%, more preferably at least 60%, even more preferably at least 70%, particularly preferred at least 80%, more particularly preferred at least 90%, even more particularly preferred at least 95% and most preferably at least 97% identity with a nucleic acid sequence as described above encoding an antibody molecule. Moreover, the term "hybridizing sequences" preferably refers to sequences encoding an fH-derived SCR or a functional fragment thereof having a sequence identity of at least 40%, preferably at least 50%, more preferably at least 60%, even more preferably at least 70%, particularly preferred at least 80%, more particularly preferred at least 90%, even more particularly preferred at least 95% and most preferably at least 97% identity with an amino acid sequence of the fH-derived SCR sequences as described herein above.

In accordance with the present disclosure, the term "identical" or "percent identity" in the context of two or more nucleic acid or amino acid sequences, refers to two or more sequences or subsequences that are the same, or that have a specified percentage of amino acid residues or nucleotides that are the same (e.g., 60% or 65% identity, preferably, 70-95% identity, more preferably at least 95% identity), when compared and aligned for maximum correspondence over a window of comparison, or over a designated region as measured using a sequence comparison algorithm as known in the art, or by manual alignment and visual inspection. Sequences having, for example, 60% to 95% or greater sequence identity are considered to be substantially identical. Such a definition also applies to the complement of a test sequence. Preferably the described identity exists over a region that is at least about 15 to 25 amino acids or nucleotides in length, more preferably, over a region that is about 50 to 100 amino acids or nucleotides in length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Although the FASTDB algorithm typically does not consider internal non-matching deletions or additions in sequences, i.e., gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul, Nucl. Acids Res. 25 (1997), 3389-3402; Altschul, J. Mol. Evol. 36 (1993), 290-300; Altschul, J. Mol. Biol. 215 (1990), 403-410). The BLASTN program for nucleic acid sequences uses as defaults a word length (W) of 11, an expectation (E) of 10, M=5, N=4, and a comparison of both strands. For amino acid sequences, the BLASTP program uses as defaults a wordlength (W) of 3, and an expectation (E) of 10. The BLOSUM62 scoring matrix (Henikoff Proc. Natl. Acad. Sci., USA, 89, (1989), 10915) uses alignments (B) of 50, expectation (E) of 10, M=5, N=4, and a comparison of both strands.

Moreover, the present disclosure also relates to nucleic acid molecules whose sequence is being degenerate in comparison with the sequence of an above-described hybridizing molecule. When used in accordance with the present disclosure the term "being degenerate as a result of the genetic code" means that due to the redundancy of the genetic code different nucleotide sequences code for the same amino acid.

In order to determine whether an amino acid residue or nucleotide residue in a given fH-derived SCR sequence corresponds to a certain position in the amino acid sequence or nucleotide sequence of any of e.g. SEQ ID NOs: 4, 22, 24, 6, 26, 8, 30 and 32, the skilled person can use means and methods well-known in the art, e.g., alignments, either manually or by using computer programs such as those mentioned further down below in connection with the definition of the term "hybridization" and degrees of homology.

For example, BLAST 2.0, which stands for Basic Local Alignment Search Tool BLAST (Altschul (1997), loc. cit.; Altschul (1993), loc. cit.; Altschul (1990), loc. cit.), can be used to search for local sequence alignments. BLAST, as discussed above, produces alignments of both nucleotide and amino acid sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cut-off score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.

Analogous computer techniques using BLAST (Altschul (1997), loc. cit.; Altschul (1993), loc. cit.; Altschul (1990), loc. cit.) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\text{\% sequence identity} \times \text{\% maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules. Another example for a program capable of generating sequence alignments is the CLUSTALW computer program (Thompson, Nucl. Acids Res. 2 (1994), 4673-4680) or FASTDB (Brutlag Comp. App. Biosci. 6 (1990), 237-245), as known in the art.

The binding molecule as comprised in the SCR-Ab construct of the disclosure may be selected from the group consisting of antibody molecules, receptor molecules, aptamers, DARPins and the like. The person skilled in the art is readily in the position to use and to obtain specific binding molecules, useful in the SCR-Ab constructs as provided herein.

In the context of the present disclosure, the term "antibody molecule(s)" or "antibody(ies)" comprises antibody molecule(s) like full immunoglobulin molecules, e.g. IgM, IgD, IgE, IgA or IgG, like IgG1, IgG2, IgG2b, IgG3 or IgG4 as well as to parts of such immunoglobulin molecules, like Fab-fragments, Fab'-fragments, F(ab)2-fragements, chimeric F(ab)2 or chimeric Fab' fragments, chimeric Fab-fragments or isolated VH- or CDR-regions (said isolated VH- or CDR-regions being, e.g. to be integrated or engineered in corresponding "framework(s)"). Accordingly, the term "antibody molecule" also comprises known isoforms and modifications of immunoglobulins, like single-chain antibodies or single chain Fv fragments (scAB/scFv) or bispecific antibody constructs, said isoforms and modifications being characterized as comprising at least one antigen binding site which specifically recognizes an antigen on the surface of a virus particle. A specific example of the above described isoform or modification may be a sc (single chain) antibody in the format VH-VL or VL-VH. Also bispecific scFvs are envisaged, e.g. in the format VH-VL-VH-VL, VL-VH-VH-VL, VH-VL-VL-VH. Also comprised in the term "antibody molecule(s)" are diabodies and molecules that comprise an antibody Fc domain as a vehicle attached to at least one antigen binding moiety/peptide, e.g. peptibodies as described in WO 00/24782. The term "Antibody fragments" also comprises such fragments which are engineered to provide modified antibody effector functions such as antibody dependent cell-mediated cytotoxicity (ADCC) and/or complement dependent cytotoxicity (CDC).

In one embodiment, the SCR-Ab of the disclosure comprises an antigen binding domain that is derived from the antibody molecules as comprised in the following Table 1.

| virus family | virus species | viral antigen | antibody clone designation | reference (paper, patent, Acc. Nr. etc.) |
|---|---|---|---|---|
| **Flaviviridae** | hepatitis C virus | hepatitis C virus E2 glycoprotein | HCV-AB 68 | ECACC Nr.: 00051714 as described in US 2004/0071710 A1 |
| (positive sense single-stranded RNA virus) | yellow fever virus | yellow fever virus (vaccine strains and Asibi strain) | 2D12; IgG2a Kappa light chain | ATCC No.: CRL-1689 as described in Schlesinger (1983) Virology 125: 8-17 |
| **Retroviridae** | human immunodeficiency virus | gp41; Epitope : ELDKWA | 2F5 ; IgG1 (κ) | ECACC Nr.: 90091704 as described in WO 03/022879 A2 |
| (reverse transcribing RNA virus) | | gp120 | 2G12; IgG1 (κ) | ECACC Nr.: 93091517 as described in WO 03/022879 A2 |
| | | gp41 ; Epitope: GCSGKLICTTAVP W | 3D6 ; IgG1 (κ) | ECACC Nr.: 87110301 as described in EP 0 355 140 B1 |
| | | gp41 ; Epitope : NWFDIT | 4E10 ; IgG1 (κ) | ECACC Nr. : 90091703 as described in WO 03/022879 A2 |
| | | gp41 | 4E10 ; IgG3 | ECACC Nr.: 01110665 as described in WO 03/022879 A2 |
| | Friend murine leukaemia virus | FV envelope protein | clone #48 | Chesebro et al. (1981) Virology 112(1): 131-44 |
| **Paramyxoviridae** (negative sense single-stranded RNA virus) | measles virus | measles virus haemagglutinin | HA cl. 55 | ECACC Nr.: 95040311 as described in Giraudon and Wild (1981) J. Gen Virol;54:325 |
| **Rhabdoviridae** (negative sense single-stranded RNA virus) | rabies virus | glycoprotein of various rabies virus strains | MAb 57; IgG2 | US 2003/0157112 A1 Antibody encoded by specific amino acid sequences |
| **Herpesviridae** (double stranded DNA virus) | Epstein-Barr virus | 350/220 kDa viral envelope glycoprotein | 72A1 ; Mouse IgG1 | ATCC No.:HB-168 as described in Hoffman (1980) PNAS 77: 2979-2983 |

(continued)

| virus family | virus species | viral antigen | antibody clone designation | reference (paper, patent, Acc. Nr. etc.) |
|---|---|---|---|---|
| **Hepadnaviridae** (reverse transcribing DNA virus) | hepatitis B virus | hepatitis B virus surface antigen (HBsAg) | H25B10; Mouse IgG 1 | ATCC No.: CRL-8017 as described in US 4,271,145 |

Yet, the person skilled in the art is readily in the position to use and to obtain other suitable antibody molecules. For instance, antibody molecules known to specifically bind surface antigens of pathogens may be used as binding molecule comprised in the SCR-Ab construct described herein. Alternatively, suitable antibody molecules may be raised using standard methods known in the art, see, inter alia, Harlow and Lane "Antibodies: a laboratory manual" Cold Spring Harbor Laboratory Press (1988).

In another embodiment, the SCR-Ab of the disclosure comprises an antigen-binding domain as comprised in the antibody molecule selected from the group consisting of 2F5, 2G12, 3D6, 4E10 IgG1 and 4E10 IgG3 as defined in Table 1 that is provided herein above.

The term "receptor molecule" as used herein relates to proteins or fragments thereof which are capable of binding specific ligands. Binding of a ligand to a receptor molecule in its normal cellular context initiates a cellular response to the ligand. In the present disclosure, however, useful receptor molecules may also only comprise the ligand-binding portion of a receptor which is not capable of initiating a cellular response. The skilled person is aware that receptor molecules capable of binding pathogen-associated ligands are specifically useful in the context of the present disclosure. In a non-limiting example, a receptor molecule capable of binding a pathogen-associated ligand may be the CD4 receptor, preferably the human CD4 receptor. Other receptor molecules which are capable of binding pathogen-associated ligands include chemokine receptors such as the (human) CXCR4 receptor or the (human) β-chemokine receptor CCR5. Yet, the person skilled in the art is readily capable of identifying other examples of receptor molecules which are useful in the SCR-Ab construct as described herein.The term "aptamer" as used herein relates to nucleic acid molecules that are capable of specifically binding target molecules. Aptamers commonly comprise RNA, single stranded DNA, modified RNA or modified DNA molecules. The preparation of aptamers is well known in the art and may involve, inter alia, the use of combinatorial RNA libraries to identify binding sites (Gold (1995), Ann. Rev. Biochem 64, 763-797).

The term "DARPin" as used herein relates to designed binding proteins comprising ankyrin repeats as known in the art (Kohl (2003), PNAS 100, 1700-1705, Forrer (2003), FEBS letters 539, 2-6). Highly specific DARPins may be generated by screening DARPin libraries as described in WO 02/20565, e.g. by using ribosome display technology (Hanes (1997), PNAS 94, 4937-4942). Yet, the person skilled in the art is aware that other designed repeat protein libraries (DRP libraries), such as Leucine-rich repeat (LRR) libraries, may be used for screening binding molecules which are useful in the context of the present disclosure.

As used herein, "specifically recognizing" or "specifically binding" refers to the binding of a binding molecule to a target molecule, such molecules may be antibody molecules binding to a given antigen or receptor molecule binding to a given ligand and the like. According the law of mass action, the binding-equilibrium is dependent on the concentration of target molecule [t], the concentration of binding molecule [b] and the concentration of the binding molecule-target molecule complex [bt]. The equilibrium constant Kd, therefore, is defined as [t] x [b] / [bt]. Ligand binding of the binding molecule comprised in the SCR-Ab constructs as described herein may occur e.g. with an affinity (Kd) of about $10^{-13}$ to $10^{-6}$ M, e.g. with an affinity of about $10^{-13}$ to $10^{-7}$ M, e.g. with an affinity of about $10^{-13}$ to $10^{-8}$ M, e.g. with an affinity of about $10^{-13}$ to $10^{-9}$ M. As discussed before, the term "specifically recognizing/binding" in the context of the binding molecules for a given pathogen comprises in particular corresponding antibodies (or fragments or derivatives thereof).

In contrast thereto, the Kd for endogenous fH on cells is relatively high. Thus, only small amounts of endogenous fH are bound on the cell surface and the exchange from fH on the surface is fast. The Kd-value of fH-derived SCRs is expected to be in the same range as for the whole fH molecule. Previously described SCR constructs such as those described in EP-A1 0 854 150 do not comprise the pathogen-specific binding molecule as defined herein and, accordingly; show a substantial lower affinity (Kd) for pathogens or pathogen infected cells. Consequently, relatively high amounts of the respective SCR constructs are required in order to compete with the endogenous fH for binding on the cells and to sufficiently block fH-pathogen interactions; see, inter alia, the appended Figures 5 and 7.

The SCR-Ab constructs as described herein selectively bind pathogen-associated proteins exposed on pathogens and/or pathogen-infected cells with a high affinity via the comprised binding molecule. Accordingly, the fH-derived SCR constructs will only dissociate with the Kd of the binding molecule and are thought be "arrested" at the surface of pathogens and/or pathogen-infected cells. Without being bound by theory, the fH-derived SCR region comprised in the SCR-Ab constructs of the disclosure are thought to displace the host-derived fH from the surface of the pathogen and/or pathogen infected cells. This is thought to lead to the termination of the fH mediated inhibition of complement activation which finally will result in the complement-mediated lysis of the pathogens and/or pathogen infected cells.

The fH-derived SCR and the binding molecule as comprised in the SCR-Ab construct of the present disclosure may be covalently linked. The covalent linking of a fH-derived SCR with a binding molecule results in a molecule in which said fH-derived SCR and said binding molecule are connected by (a) covalent bond(s). A covalent bond is a chemical bonding that is characterized by the sharing of pairs of electrons between atoms, as, inter alia, obtained by the herein exemplified cross-binding via chemical compounds. However, also the recombinant production of constructs as disclosed herein, i.e. SCR-Ab comprising a complement factor derived short consensus repeat and a covalently bound binding molecule specifically recognizing/binding to a pathogen is envisaged.

Alternatively, the fH-derived SCR and the binding molecule as comprised in the SCR-Ab construct described herein may be non-covalently linked. Non-covalent bonds are known in the art and include, but are not limited to the association of protein molecules as a result of protein-protein interaction. Non-limiting examples of non-covalent bonds that may be useful for linking a fH-derived SCR and a binding molecule in the context of the present disclosure include the biotin/streptavidin complex, lectin/glycoprotein complexes and antibody-antigen complexes. Yet, the person skilled in the art is readily capable of identifying other non-covalent bonds/complexes which are useful for non-covalently linking a fH-derived SCR with a binding molecule for generating SCR-Ab constructs as described herein.

In one embodiment, the fH-derived SCR and the binding molecule are comprised in a single-chain multi-functional polypeptide. A single-chain SCR-Ab construct e.g. may consist of (a) polypeptide(s) comprising (a) SCR-derived domain(s) and (a) binding-molecule domain(s). Said domains are connected by a polypeptide linker, wherein said linker is disposed between said SCR-derived domain(s) and said binding-molecule domain(s).

The SCR-Ab construct as described herein specifically recognizes a pathogen, wherein said pathogen may be a virus or a bacterium. Said virus may be selected from the group consisting of a double-stranded DNA virus, single-stranded DNA virus, double-stranded RNA virus, positive-sense single-stranded RNA virus, negative-sense single-stranded RNA virus, reverse transcribing RNA virus and reverse transcribing DNA virus. Said double-stranded DNA virus may include, but is not limited to herpes simplex virus, cytomegalo virus, varicella zoster virus, Epstein-Barr virus, roseolo virus, human herpesvirus-7 or Kaposi’s sarcoma-associated virus. The positive-sense single-stranded RNA viruses as defined above include, but are not limited to corona virus, hepatitis C virus, dengue fever virus, polio virus, rubella virus, yellow fever virus or tick-borne encephalitis virus. The negative-sense single-stranded RNA viruses as defined above include, but are not limited to influenza virus, Ebola virus, Marburg virus, measles virus, mumps virus, rabies virus, parainfluenza virus, Lassa virus or lymphocytic choriomeningitis virus. Said reverse transcribing RNA virus may be a retrovirus, wherein said retrovirus e.g. may be selected from the group consisting of Rous sarcoma virus (RSV); mouse mammary tumour virus (MMTV); Friend murine leukaemia virus (FV); feline leukaemia virus; feline sarcoma virus; bovine leukaemia virus; human T-lymphotropic virus (HTLV); bovine immunodeficiency virus; equine infectious anaemia virus; feline immunodeficiency virus; human immunodeficiency virus (HIV); simian immunodeficiency virus (SIV) and spumavirus. The reverse transcribing DNA virus as defined herein above includes, but is not limited to hepatitis B virus.

In another embodiment, the present disclosure relates to polynucleotides encoding the SCR-Ab constructs as described herein. Said SCR-Ab encoding polynucleotide e.g. may comprise, but is not limited to, a polynucleotide encoding a fH-derived SCR and a binding molecule that are comprised in a single chain multi-functional polypeptide. The term "polynucleotide", as used herein, is intended to include nucleic acid molecules such as DNA molecules and RNA molecules. Said nucleic acid molecule may be single-stranded or double-stranded, but preferably is double-stranded DNA. Preferably, said polynucleotide may be comprised in a vector.

Furthermore, it is envisaged to transfect cells with the polynucleotides or vectors as described herein. Yet, in a further embodiment, the present disclosure relates to polynucleotides which upon expression encode the above-described polypeptides. Said polynucleotides may be fused to suitable expression control sequences known in the art to ensure proper transcription and translation of the polypeptide. Such vectors may comprise further genes such as marker genes which allow for the selection of said vector in a suitable host cell and under suitable conditions.

Preferably, the polynucleotide described herein, is comprised in a recombinant vector in which a polynucleotide encoding the herein described fH-SCR-constructs is operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression in prokaryotic host cells comprise, e.g., the PL, lac, trp or tac promoter in E. coli, and examples for regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. Methods which are well known to those skilled in the art can be used to construct recombinant vectors; see, for example, the techniques described in Sambrook (1989), Molecular Cloning: A Laboratory Manual, Cold Spring Harbor Laboratory N.Y. and Ausubel (1989), Current Protocols in Molecular Biology, Green Publishing Associates and Wiley Interscience, N.Y. In this context, suitable expression vectors are known in the art such as Okayama-Berg cDNA expression vector pcDV1 (Pharmacia), pCDM8, pRc/CMV, pcDNA1, pcDNA3, pPICZalpha A (Invitrogen), or pSPORT1 (GIBCO BRL). Furthermore, depending on the expression system used leader sequences capable of directing the polypeptide to a cellular compartment or secreting it into the medium may be added to the coding sequence of the polynucleotide described herein.

In accordance with the above, the present disclosure relates to vectors, particularly plasmids, cosmids, viruses and bacteriophages used conventionally in genetic engineering that comprise a polynucleotide encoding a polypeptide described herein. Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector described herein into targeted cell populations. The vectors containing the polynucleotides described herein can be transferred into the host cell by well-known methods, which vary depending on the type of cellular host. Such methods, for example, include the techniques described in Sambrook (1989), loc. cit. and Ausubel (1989), loc. cit. Accordingly, calcium chloride transfection is commonly utilized for prokaryotic cells, whereas calcium phosphate treatment or electroporation may be used for other cellular hosts; see Sambrook, supra. As a further alternative, the polynucleotides and vectors described herein can be reconstituted into liposomes for delivery to target cells. The polynucleotide or vector described herein which is present in host cell may either be integrated into the genome of the host cell or it may be maintained extra-chromosomally.

In a further aspect, the disclosure comprises methods for the preparation of the SCR-Ab construct as described herein. The inventive SCR-Ab construct may be recombinantly produced, e.g. by cultivating a cell comprising the described polynucleotides or vectors which encode the inventive SCR-Ab constructs and isolating said constructs from the culture. The inventive SCR-Ab construct may be produced in any suitable cell-culture system including, but not limited to eukaryotic cells, e.g. pichia pastoris yeast strain X-33 or CHO cells. Further suitable cell lines known in the art are obtainable from cell line depositories, like the American Type Culture Collection (ATCC). The term "eukaryotic" is meant to include yeast, higher plant, insect and mammalian cells. The transformed hosts can be grown in fermentors and cultured according to techniques known in the art to achieve optimal cell growth. In a further embodiment, the disclosure thus relates to a process for the preparation of a polypeptide described above comprising cultivating a cell described herein under conditions suitable for the expression of the polypeptide and isolating the polypeptide from the cell or the culture medium.

The polypeptides described herein, accordingly, can be isolated from the growth medium, cellular lysates or cellular membrane fractions. The isolation and purification of the expressed polypeptides described herein may be by any conventional means, including ammonium sulphate precipitation, affinity columns, column chromatography, gel electrophoresis and the like and may involve the use of monoclonal or polyclonal antibodies directed, e.g., against a tag of e.g. the polypeptides described herein; see, Scopes (1982), "Protein Purification", Springer-Verlag, N.Y.. The protein e.g. can be purified via its His-tag by using a Ni-NTA-column (Mack (1995), PNAS 92, 7021-7025) as described in the appended examples. Substantially pure polypeptides of at least about 90 to 95% homogeneity are preferred, and 98 to 99% or more homogeneity are most preferred, for pharmaceutical uses. Depending upon the host employed in a recombinant production procedure, the polypeptides described herein may be glycosylated or may be non-glycosylated.

The method for the preparation of the short consensus repeat-antibody construct (SCR-Ab) as described herein may also comprise coupling of the herein described fH-derived SCR with a binding molecule. For instance, the fH-derived SCR may be coupled with a binding molecule by using sulfosuccinimidyl 4-[p-maleimidophenyl]butyrate (Sulfo-SMPB) chemical cross-linking as described in the appended examples. Yet, the person skilled in the art is readily capable of identifying other chemical cross-linkers which are useful in generating the SCR-Ab construct as described herein. Non-limiting examples of such cross-linking reagents are listed in the following Table 2.

| **Abbreviation** | **Chemical Name** |
|---|---|
| **ABH** | *p*-Azidobenzoyl hydrazide |
| **AMAS** | *N*-(〈-Maleimidoacetoxy)-succinimide ester |
| **ANB-NOS** | *N*-5-Azido-2-nitrobenzyloxy-succinimide |
| **APDP*** | *N*-(4-[*p*-Azidosalicylamido]butyl)- 3’-(2’-pyridyldithio) propionamide |
| **APG******** | *p*-Azidophenyl glyoxal monohydrate |

(continued)

| Abbreviation | Chemical Name |
|---|---|
| **ASBA*** | 4-(*p*-Azidosalicylamido)-butylamine |
| **BASED*** | *Bis* ([ß-[4-azidosalicylamido]ethyl) disulfide |
| **BMB** | 1,4-*Bis*-Maleimidobutane |
| **BMDB** | 1,4-*Bis*-Maleimidyl-2,3-dihydroxybutane |
| **BMH** | *Bis*-Maleimidohexane |
| **BMOE** | *Bis*-Maleimidoethane |
| **BMPA** | *N*-ß-Maleimidopropionic acid |
| **BMPH** | *N*-(ß-Maleimidopropionic acid)hydrazide•TFA |
| **BMPS** | *N*-(ß-Maleimidopropyloxy)succinimide ester |
| **BM[PEO]$_2$** | 1,8-*Bis*-Maleimidodiethylene-glycol |
| **BM[PEO]$_3$** | 1,11-*Bis*-Maleimidotriethyleneglycol |
| **BS$_2$G-d0** | *Bis* (sulfosuccinimidyl)glutarate-d0 |
| **BS$_2$G-d4** | *Bis* (sulfosuccinimidyl)2,2,4,4-glutarate-d4 |
| **BS$_3$ (Sulfo-DSS)** | *Bis* (sulfosuccinimidyl)suberate |
| **BS$_3$-d0** | *Bis* (sulfosuccinimidyl)suberate-d0 |
| **BS$_3$-d4** | *Bis* (sulfosuccinimidyl)2,2,7,7-suberate-d4 |
| **BS[PEG]$_5$** | *Bis* (NHS)PEO$_5$ |
| **BSOCOES** | *Bis* (2-[succinimidoxycarbonyloxy]ethyl)sulfone |
| **C6-SANH***** | C6-Succinimidyl 4-hydrazinonicotinate acetone hydrazone |
| **C6-SFB****** | C6-Succinimidyl 4-formylbenzoate |
| **DCC** | *N,N*-Dicyclohexylcarbodiimide |
| **DFDNB** | 1-5-Difluoro-2,4-dinitrobenzene |
| **DMA** | Dimethyl adipimidate•2HCl |
| **DMP** | Dimethyl pimelimidate•2HCl |
| **DMS** | Dimethyl suberimidate•2HCl |
| **DPDPB** | 1,4-Di-(3'-[2'pyridyldithio]propionamido) butane |
| **DSG** | Disuccinimidyl glutarate |
| **DSP** | Dithiobis(succimidylpropionate) (Lomant's Reagent) |
| **DSS** | Disuccinimidyl superate |
| **DST** | Disuccinimidyl tartarate |
| **DTBP** | Dimethyl 3,3'-dithio*bis*propionimidate•2HC |
| **DTME** | Dithio*bis*-maleimidoethane |
| **DTSSP (Sulfo-DSP)** | 3,3'-Dithio*bis*(sulfosuccinimidylpropionate) |
| **EDC** | 1-Ethyl-3-(3-dimethylaminopropyl) carbodiimide hydrochloride |
| **EGS** | Ethylene glycol *bis*(succinimidylsuccinate) |
| **EMCA** | *N* -e-Maleimidocaproic acid |
| **EMCH** | *N* -(e-Maleimidocaproic acid)hydrazide |
| **EMCS** | *N*-(e-Maleimidocaproyloxy)succinimide ester |

(continued)

| Abbreviation | Chemical Name |
|---|---|
| **GMBS** | *N*-(g-Maleimidobutyryloxy)succinimide ester |
| **HBVS** | 1,6-Hexane-*bis*-vinylsulfone |
| **KMUA** | *N*-\| -Maleimidoundecanoic acid |
| **KMUH** | *N*-(\| -Maleimidoundecanoic acid)hydrazide |
| **LC-SMCC** | Succinimidyl 4-(*N*-maleimidomethyl) cyclohexane-1-carboxy-(6-amidocaproate) |
| **LC-SPDP** | Succinimidyl 6-(3'-[2-pyridyl- dithio]propionamido)hexanoate |
| **MBS** | *m*-Maleimidobenzoyl-*N*-hydroxysuccinimide ester |
| **MPBH** | 4-(4-*N*-Maleimidophenyl)- butyric acid hydrazide•HCl |
| **Mts-Atf-Biotin**** | 2-[*N* 2-(4-Azido-2,3,5,6-tetrafluorobenzoyl)-*N* 6-(6-biotinamidocaproyl)-L-lysinyl]ethylmethanethiosulfate |
| **Mts-Atf-LC-Biotin**** | 2-{*N* 2-[N6-(4-Azido-2,3,5,6-tetrafluorobehzoyl)-*N* 6-(6-biotinamidocaproyl)-L-lysinyl]} ethylmethanethiosulfate |
| **NHS-ASA*** | *N*-Hydroxysuccinimidyl-4-azidosalicylic acid |
| **PDPH** | 3-(2-Pyridyldithio)propionylhydrazide |
| **PMPI** | *N*-(*p*-Maleimidophenyl)isocyanate |
| **SADP** | 3'-dithiopropionate |
| **SANH***** | Succinimidyl 4-hydrazinonicotinate acetone hydrazone |
| **SANPAH** | *N*-Succinimidyl 6-(4'-azido-2'-nitrophenylamino)hexanoate |
| **SBAP** | Succinimdyl 3-(bromoacetamido)propionate |
| **SFB****** | Succinimidyl 4-formylbenzoate |
| **SHTH***** | Succinimidyl 4-hydrazidoterephthalate hydrochloride |
| **SIA** | *N*-succinimidyl iodoacetate |
| **SIAB** | *N*-Succinimidyl(4-iodoacetyl)aminobenzoate |
| **SMCC** | Succinimidyl 4-(*N*-maleimido- methyl)cyclohexane-1-carboxylate |
| **SM[PEG]$_2$** | NHS-PEO$_2$-Maliemide |
| **SM[PEG]$_4$** | NHS-PEO$_4$-Maliemide |
| **SM[PEG]$_8$** | NHS-PEO$_8$-Maliemide |
| **SM[PEG]$_{12}$** | NHS-PEO$_{12}$-Maliemide |
| **SMPB** | Succinimidyl 4-(*p*-maleimido-phenyl)butyrate |
| **SMPH** | Succinimidyl-6-(ß-maleimidopropionamido)hexanoate |
| **SMPT** | 4-Succinimidyloxycarbonyl- methyl-〈-(2-pyridyldithio)toluene |
| **SPB** | Succinimidyl-(4-psoralen-8-yloxy)butyrate |
| **SPDP** | *N*-Succinimidyl 3-(2-pyridyldithio)propionate |
| **Sulfo-DSS** | *See BS$_3$* |
| **Sulfo-EGS** | Ethylene glycol *bis* (sulfo-succinimidyl succinate) |
| **Sulfo-EMCS** | *N*-(e-Maleimidocaproyloxy)sulfosuccinimide ester |
| **Sulfo-GMBS** | *N*-(g-Maleimidobutryloxy)sulfosuccinimide ester |
| **Sulfo-HSAB** | *N*-Hydroxysulfosuccinimidyl-4-azidobenzoate |

(continued)

| Abbreviation | Chemical Name |
|---|---|
| **Sulfo-KMUS** | *N*-(k-Maleimidoundecanoyloxy)sulfosuccinimide ester |
| **Sulfo-LC-SMPT** | Sulfosuccinimidyl 6-(〈-methyl-〈-[2-pyridyldithio]-toluamido)hexanoate |
| **Sulfo-LC-SPDP** | Sulfosuccinimidyl 6-(3'-[2-pyridyl- dithio]propionamido)hexanoate |
| **Sulfo-MBS** | *m*-Maleimidobenzoyl-*N*-hydroxysulfosuccinimide ester |
| **Sulfo-NHS-LC-ASA*** | Sulfosuccinimidyl(4-azido-salicylamido)hexanoate |
| **Sulfo-SADP** | Sulfosuccinimidyl(4-azido-phenyldithio)propionate |
| **Sulfo-SAED** | Sulfosuccimidyl 2-[7-azido-4-methylcoumarin-3-acetamido]ethyl-1,3'-dithiopropionate |
| **Sulfo-SAND** | Sulfosuccinimidyl-2-(*m*-azido-*o*-nitrobenzamido) ethyl 1,3'-dithiopropionate |
| **Sulfo-SANPAH** | Sulfosuccinimidyl 6-(4'-azido-2'- nitrophenylamino)hexanoate |
| **Sulfo-SASD*** | Sulfosuccinimidyl 2-(*p*-azido- salicylamido)ethyl 1,3'-dithiopropionate |
| **Sulfo-SBED**** | Sulfo-NHS-(2-6-[Biotinamido]-2-(*p*-azidobezamido) hexanoamido)ethyl-1,3'-dithiopropionate *(Trifunctional)* |
| **Sulfo-SFAD** | Sulfosuccinimidyl(perfluoroazidobenzamido) ethyl 1,3'-dithiopropionate |
| **Sulfo-SIAB** | Sulfosuccinimidyl(4-iodo-acetyl)aminobenzoate |
| **Sulfo-SMCC** | Sulfosuccinimidyl 4-(*N*-maleimido- methyl)cyclohexane-1-carboxylate |
| **Sulfo-SMPB** | Sulfosuccinimidyl 4-(*p*-maleimidophenyl)butyrate |
| **THPP** | β-(Tris[hydroxymethyl]phosphine)propionic acid (betaine) |
| **TMEA***** | *Tris*-(2-Maleimidoethyl)amine *(Trifunctional)* |
| **TSAT***** | *Tris*-(succimimidyl aminotricetate) *(Trifunctional)* |

Accordingly, any conventional cross-linking procedure may be applied for preparing the herein described SCR-Ab constructs, including, but not limited to, by forming protein-protein interactions such as biotin-streptavidin complexes or the antibody-antigen complexes.

In a further embodiment, the short consensus repeat-antibody (SCR-Ab) described herein is comprised in a composition. Said composition may comprise one or more SCR-Ab constructs as provided herein. Said composition may be a pharmaceutical composition, optionally further comprising a pharmaceutically acceptable carrier and/or diluent. The use of the herein described SCR-Ab constructs for the preparation of a pharmaceutical composition for the prevention, treatment or amelioration of an infection with a pathogen or a pathological condition associated with an infection with a pathogen is also envisaged. Said pathogen may be a virus or a bacterium as defined herein above.

It is evident for the skilled artisan that for short consensus repeat-antibody construct (SCR-Ab) in which the comprised complement factor H-derived short consensus repeat (fH-derived SCR) and the binding molecule are non-covalently bound, said fH-derived SCR and said binding molecule may be administered concomitantly or sequentially. As exemplified herein below, e.g., the binding molecule may be administered first followed by the administration of the fH-derived SCR. Accordingly, the binding molecule that, e.g. specifically binds to a pathogen specifically associates to the fH-SCR forming the herein described non-covalently linked short consensus repeat-antibody construct (SCR-Ab). Therefore, the compositions described herein also comprise compositions in which both the complement factor H-derived short consensus repeat (fH-derived SCR) and the binding molecule are present independently.

The present disclosure also relates to the use of the nucleic acid molecules (polynucleotides), vectors, as well as transfected cells comprising said nucleic acid molecules (polynucleotides), vectors in medical approaches, like, e.g. cell based gene therapy approaches or nucleic acid based gene therapy approaches.

Said viral vectors are particularly suitable for gene therapy. Gene therapy, which is based on introducing therapeutic genes into cells by ex-vivo or in-vivo techniques is one of the most important applications of gene transfer. Suitable vectors, methods or gene-delivering systems for in-vitro or in-vivo gene therapy, as well as vector systems, are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813, Isner, Lancet 348 (1996), 370-374; Muhlhauser, Circ. Res. 77 (1995), 1077-1086; Onodua, Blood 91 (1998), 30-36; Verzeletti, Hum. Gene Ther.

9 (1998), 2243-2251; Verma, Nature 389 (1997), 239-242; Anderson, Nature 392 (Supp. 1998), 25-30; Wang, Gene Therapy 4 (1997), 393-400; Wang, Nature Medicine 2 (1996), 714-716; WO 94/29469; WO 97/00957; US 5,580,859; US 5,589,466; US 4,394,448, US2007/086985, US2007/082388, US2007/071770, US2007/003522 US2007/048285 (or corresponding EP1757703) or WO2004/111248 and references cited therein. Suitable vehicles/delivery vehicles, are inter alia, disclosed in WO2007/022030, WO2007/018562. Further suitable gene therapy constructs for use especially in lymphatic cells and/or tissues are known in the art; see Li, Ann NY Acad Sci 1082 (2006) 172-9.

In yet another embodiment, the present disclosure relates to method for the prevention, treatment or amelioration of an infection with a pathogen or a pathological condition associated with an infection with a pathogen can, in further embodiments using different binding molecules recognizing or binding to tumour cells, cancer cells and/or malignant cells for the treatment of an proliferative disorder like cancer, comprising administering the short consensus repeat-antibody construct (SCR-Ab) as described herein above or in the items below to a mammal in need of such prevention or treatment, wherein said mammal is a human. In a preferred embodiment, said virus may be a human immunodeficiency virus (HIV). Examples of a pathological condition associated with an infection with a pathogen may include, but are not limited to, acquired immune deficiency syndrome (AIDS) which is associated with a HIV infection, severe acute respiratory syndrome (SARS) which is associated with a corona virus infection, acute/chronic hepatitis C which is associated with hepatitis C virus infection or influenza which is associated with influenza virus infection.

As described, it is also envisaged that the inventive pharmaceutical concept described herein may be employed in a medical setting where it is desired to inhibit and/or eliminate pathogen infected host cells or malignant cells, like cancer cells. In said embodiment, the binding molecule comprised in the SCR-Abs of the invention specifically recognizes or binds to a cancer cell, malignant cell and/or tumour cell.

The SCR-Ab constructs to be employed in accordance with this invention may therefore (and alternatively) comprise a binding molecule that specifically recognizes or binds to a cancer cell, a malignant cell or a tumour cell additionally to the herein defined complement factor H-derived short consensus repeat. As documented herein below and in the appended examples it was, in accordance with this invention, surprisingly found that certain inventive SCR-Ab constructs are capable of lysing cancer cells in vitro in a highly unexpected manner.

Said cancer cell, malignant cell or tumour cell may be derived from any cancer or tumour type. In one embodiment the cancer cell or malignant cell is selected from the group consisting of but not limited to breast cancer cells, Burkitt's lymphoma cells, multiple myeloma cells, colorectal cancer cells, metastatic colorectal cancer cells, Non-Hodgkin's Lymphoma cells, lung cancer cells, chronic lymphocytic leukaemia cells, micro-metastases or residual tumour cells. Preferably, the cancer cell or malignant cell the tumour cells may be derived from micro-metastases or residual tumours.

Short consensus repeat-antibody construct (SCR-Ab) comprising an antibody molecule that specifically recognizes a cancer cell as described herein is useful for the prevention, treatment or amelioration of a cancerous disease in a subject. Preferably, said subject is a human.

As used herein, the cancerous disease may be selected from but not limited to breast cancer, Burkitt's lymphoma, multiple myeloma, colorectal cancer, metastatic colorectal cancer, Non-Hodgkin's Lymphoma, lung cancer, chronic lymphocytic leukaemia. Preferably, the cancerous disease originates from micro-metastases or residual tumours.

The antibody molecule comprised in the herein described short consensus repeat-antibody construct (SCR-Ab) useful for the treatment of a cancerous disease may be selected from the group consisting of but not limited to monoclonal antibodies recognizing an epitope selected from the group consisting of: CD9, CD19, CD20, CD22, CD30, CD33, CD40, CD46, CD55, CD56, CD138, erbB1, HER2/neu, IGFR, MUC-1, TAG-72, TAL-6, TRAILR and VEGFR. Antibody molecules that specifically recognize the above-described antigens are well known in the art. The compositions of the invention may be in solid or liquid form and may be, inter alia, in a form of (a) powder(s), (a) tablet(s), (a) solution(s) or (an) aerosol(s). Furthermore, it is envisaged that the pharmaceutical composition described herein might comprise further biologically active agents, depending on the intended use of the pharmaceutical composition.

Such agents might be antibiotics, antiviral drugs or drugs acting on the gastro-intestinal system.

Administration of the suitable (pharmaceutical) compositions may be effected by different ways, e.g., by parenteral, subcutaneous, intraperitoneal, topical, intrabronchial, intrapulmonary and intranasal administration and, if desired for local treatment, intralesional administration. Parenteral administrations include intraperitoneal, intramuscular, intradermal, subcutaneous intravenous or intraarterial, administration. It is particularly preferred that said administration is carried out by injection and/or delivery, e.g., to a site in a brain artery or directly into brain tissue. The compositions described herein may also be administered directly to the target site, e.g., by biolistic delivery to an external or internal target site, like a specific organ which is infected with a pathogen.

Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. Suitable carriers may comprise any material which, when combined with the SCR-Ab constructs described herein, retains the biological activity of the comprised SCR-Ab construct; see Remington's Pharmaceutical Sciences (1980) 16th edition, Osol, A. Ed. Preparations for parenteral administration may include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles may include sodium chloride solution, Ringer's dextrose, dextrose and sodium chloride, lactated Ringer's, or fixed oils. Intravenous vehicles may include fluid and nutrient replenishes, electrolyte replenishers (such as those based on Ringer's dextrose), and the like. Preservatives and other additives may also be present including, for example, antimicrobials, anti-oxidants, chelating agents, and inert gases and the like. In addition, the pharmaceutical composition described herein might comprise proteinaceous carriers, like, e.g., serum albumine or immunoglobuline, preferably of human origin.

These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. Pharmaceutically active matter may be present in amounts between 1 $\mu$g and 20 mg/kg body weight per dose, e.g. between 0.1 mg to 10 mg/kg body weight, e.g. between 0.5 mg to 5 mg/kg body weight. If the regimen is a continuous infusion, it should also be in the range of 1 $\mu$g to 10 mg per kilogram of body weight per minute. Yet, doses below or above the indicated exemplary ranges also are envisioned, especially considering the aforementioned factors.

The pharmaceutical compositions as described herein may be formulated to be short-acting, fast-releasing, long-acting, or sustained-releasing. Hence, the pharmaceutical compositions may also be suitable for slow release or for controlled release. Sustained-release preparations may be prepared using methods well known in the art. Suitable examples of sustained-release preparations include semipermeable matrices of solid hydrophobic polymers containing the antibody in which the matrices are in the form of shaped articles, e.g. films or microcapsules. Examples of sustained-release matrices include polyesters, copolymers of L-glutamic acid and ethyl-L-glutamate, non-degradable ethylene-vinyl acetate, hydrogels, polylactides, degradable lactic acid-glycolic acid copolymers and poly-D-(-)-3-hydroxybutyric acid. Possible loss of biological activity and possible changes in the binding properties of SCR-Ab constructs comprised in sustained-release preparations may be prevented by using appropriate additives, by controlling moisture content and by developing specific polymer matrix compositions.

Furthermore, it is envisaged that the pharmaceutical composition described herein might comprise further biologically active agents, depending on the intended use of the pharmaceutical composition. For example in patients suffering from an HIV-infection, such agents might be drugs acting on the immunological system, drugs used in anti-viral treatment, in particular in HIV-treatment (for example, HAART) and AIDS management and/or antiinflammatory drugs. HAART therapy consists of a cocktail of three classes anti-viral drugs. The classes are nucleosidal reverse transcriptase inhibitors (NRTI), non-nucleosidal reverse transcriptase inhibitors (NNRTI) and protease inhibitors (PI). Usually 2 to 4 drugs from preferentially more than one class are combined to reduce viral load to almost non-detectable levels. Early treatment of infected patients with HAART prevents the transition of viral strains from usage of CCR5 to other chemokine receptors, like CXCR4 (Connor (1997) J. Exp. Med. 185, 621-628). Constructs as disclosed herein can be administered in addition to HAART intravenously, subcutaneously, and/or into the cerebral-spinal fluid. Other agents for combination with the inventive constructs could comprise, inter alia, or integrase inhibitors such as raltegravir.

In a further embodiment, the composition as comprised herein may be a diagnostic composition, optionally further comprising suitable means for detection.

In yet another embodiment, the present disclosure relates to a kit comprising at least one SCR-Ab construct as defined. Advantageously, the kit described herein further comprises, optionally (a) buffer(s), storage solutions and/or remaining reagents or materials required for the conduct of medical, scientific or diagnostic assays and purposes. Furthermore, parts of the kit described herein can be packaged individually in vials or bottles or in combination in containers or multicontainer units. In one embodiment, said kit may comprise the short consensus repeat-antibody construct (SCR-Ab) according to the present disclosure, wherein both the complement factor H-derived short consensus repeat (fH-derived SCR) and the binding molecule are present independently in one container. In another embodiment, said complement factor H-derived short consensus repeat (fH-derived SCR) and the binding molecule are present independently in more than one container and wherein a covalently bound SCR-Ab or a non-covalently bound SCR-Ab is formed after the contacting the comprised fH-derived SCR with the comprised binding molecule.

The kit described herein may be advantageously used, inter alia, for carrying out the method described herein and could be employed in a variety of applications referred herein, e.g., as diagnostic kits, as research tools or medical tools. Additionally, the kit described herein may contain means for detection suitable for scientific, medical and/or diagnostic purposes. The manufacture of the kits follows preferably standard procedures which are known to the person skilled in the art.

In addition to the above description, the disclosure also relates to the following items which are particularly useful in the medical intervention of cancerous diseases, tumour diseases and/or hyperplastic diseases:

Item 1. A short consensus repeat-antibody construct (SCR-Ab) comprising

(a) a complement factor H-derived short consensus repeat (fH-derived SCR); and
(b) a binding molecule that specifically recognizes or binds to a cancer cell, malignant cell or tumour cell,

wherein said fH-derived SCR comprises a polypeptide that is capable of binding heparin.

Item 2. The short short consensus repeat-antibody construct (SCR-Ab) according to item 1 wherein said fH-derived SCR is selected from the group consisting of SCR7, SCR9, SCR13 and SCR18-20 or a functional fragment of said fH-derived SCR7, SCR9, SCR13 and SCR18-20 or is an artificial SCR (aSCR).

Item 3. The short consensus repeat-antibody construct (SCR-Ab) of item 1 or 2, wherein said cancer cell, malignant cell or tumour cell is selected from the group consisting of breast cancer cells, Burkitt's lymphoma cells, multiple myeloma cells, colorectal cancer cells, metastatic colorectal cancer cells, Non-Hodgkin's Lymphoma cells, lung cancer cells, chronic lymphocytic leukaemia cells, micro-metastases or residual tumour cells.

Item 4. The short consensus repeat-antibody construct (SCR-Ab) of any item 1 to 3, wherein said binding molecule comprises an antibody molecule, receptor molecule, aptamer or DARPin or a ligand binding fragment thereof.

Item 5. The short consensus repeat-antibody construct (SCR-Ab) of item 4, wherein said antibody molecule is a monoclonal antibody recognizing an epitope selected from the group consisting of: CD9, CD19, CD20, CD22, CD30, CD33, CD40, CD46, CD55, CD56, CD138, erbB1, HER2/neu, IGFR, MUC-1, TAG-72, TAL-6, TRAILR and VEGFR.

Item 6. The short consensus repeat-antibody construct (SCR-Ab) of any one of items 1 to 5, wherein said complement factor H-derived short consensus repeat (fH-derived SCR) and said binding molecule are covalently or non-covalently linked.

Item 7. The short consensus repeat-antibody construct (SCR-Ab) of any one of items 1 to 5, wherein said complement factor H-derived short consensus repeat (fH-derived SCR) and said binding molecule are comprised in a single chain multi-functional polypeptide.

Item 8. The short consensus repeat-antibody construct (SCR-Ab) of any one of items 1 to 7 wherein said fH-derived SCR is selected from the group consisting of

(a) a polypeptide encoded by the amino acid sequence comprised in SEQ ID NO: 4, SEQ ID NO: 6, SEQ ID NO: 8, SEQ ID NO: 22, SEQ ID NO: 24, SEQ ID NO: 26, SEQ ID NO: 30 or SEQ ID NO: 32 or a functional fragment thereof;
(b) a polypeptide encoded by the amino acid sequence that is at least 60% identical to the amino acid sequence comprised in SEQ ID NO: 4, SEQ ID NO: 6, SEQ ID NO: 8, SEQ ID NO: 22, SEQ ID NO: 24, SEQ ID NO: 26, SEQ ID NO: 30 or SEQ ID NO: 32 and wherein said polypeptide is capable of binding heparin;
(c) a polypeptide encoded by the polynucleotide sequence as comprised in SEQ ID NO: 3, SEQ ID NO: 5, SEQ ID NO: 7, SEQ ID NO: 21, SEQ ID NO: 23, SEQ ID NO: 25, SEQ ID NO: 29 or SEQ ID NO: 31 or a functional fragment thereof;
(d) a polypeptide encoded by the complementary sequence of a polynucleotide that is able to hybridize with the polynucleotide as comprised in SEQ ID NO: 3, SEQ ID NO: 5, SEQ ID NO: 7, SEQ ID NO: 21, SEQ ID NO: 23, SEQ ID NO: 25, SEQ ID NO: 29 or SEQ ID NO: 31 and wherein said polypeptide is capable of binding heparin; and
(e) a polypeptide encoded by a nucleic acid molecule which is at least 60% identical to the nucleic acid sequence as comprised in SEQ ID NO: 3, SEQ ID NO: 5, SEQ ID NO: 7, SEQ ID NO: 21, SEQ ID NO: 23, SEQ ID NO: 25, SEQ ID NO: 29 or SEQ ID NO: 31 and wherein said polypeptide is capable of binding heparin.

Item 9. The short consensus repeat-antibody construct (SCR-Ab) of any one of items 1 to 8, wherein said fH-derived SCR comprises the polypeptide encoded by the amino acid sequence comprised in SEQ ID NO: 4 or SEQ ID NO: 22 or a functional fragment thereof.

Item 10. A polynucleotide encoding the short consensus repeat-antibody construct (SCR-Ab) of any one of items 1 to 9.

Item 11. A vector comprising the polynucleotide of item 10.

Item 12 . A cell transfected with the polynucleotide of item 10 or the vector of item 11.

Item 13. A method for the preparation of the short consensus repeat-antibody construct (SCR-Ab) of any one of items 1 to 9 which comprises cultivating a cell of item 11 and isolating said polypeptide from the culture.

Item 14. A method for the preparation of the short consensus repeat-antibody construct (SCR-Ab) of any one of items 1 to 9 which comprises coupling said fH-derived SCR with said binding molecule.

Item 15. The method of item 14, wherein said fH-derived SCR is coupled with said binding molecule by using sulfosuccinimidyl 4-[p-maleimidophenyl]butyrate (Sulfo-SMPB) chemical cross-linking.

Item 16. The method of item 14, wherein said fH-derived SCR is coupled with said binding molecule by forming a biotin-streptavidin complex.

Item 17. A composition comprising the short consensus repeat-antibody construct (SCR-Ab) of any one of items 1 to 9.

Item 18. The composition of item 17 which is a pharmaceutical composition, optionally further comprising a pharmaceutically acceptable carrier.

Item 19. The composition of item 17 which is a diagnostic composition, optionally further comprising suitable means for detection.

Item 20. Use of the short consensus repeat-antibody construct (SCR-Ab) of any one of items 1 to 9 for the preparation of a pharmaceutical composition for the prevention, treatment or amelioration of a cancerous disease.

Item 21. The short consensus repeat-antibody construct (SCR-Ab) of any one of items 1 to 9 for the use as a pharmaceutical composition for the prevention, treatment or amelioration of a cancerous disease.

Item 22. Method for the prevention, treatment or amelioration of a cancerous disease, comprising administering the short consensus repeat-antibody construct (SCR-Ab) of any of items 1 to 9 or the composition of item 18 or 19 to a mammal in need of such prevention or treatment.

Item 23. The method according to item 22, wherein said mammal is a human.

Item 24. The use of item 20, the short consensus repeat-antibody construct of item 20 or the method of item 22 or 23, wherein said a cancerous disease is breast cancer, Burkitt's lymphoma, multiple myeloma, colorectal cancer, metastatic colorectal cancer, Non-Hodgkin's Lymphoma, lung cancer or chronic lymphocytic leukaemia or is derived from micro-metastases or residual tumours.

Item 25. Kit comprising the short consensus repeat-antibody construct (SCR-Ab) of any one of items 1 to 9, the polynucleotide of item 10, the vector of item 10 or the composition of any one of items 17 to 19.

The embodiments provided herein above in relation to binding molecule directed against a pathogen and the corresponding short consensus repeat-antibody construct apply, mutatis mutandis, to the above-provided items relating to short consensus repeat-antibody constructs (SCR-Abs) comprising a binding molecule that specifically recognizes and/or binds to a cancer cell/malignant cell/tumour cell.

In all embodiments, it is envisaged that the SCR-Abs of this disclosure are particular useful in medical/pharmaceutical intervention. Accordingly, subjects in need of such a medical/pharmaceutical intervention may be treated with the constructs/compounds of this disclosure. The subject to be treated may be mammalian, in particular a human.

## FIGURES

**Figure 1:** Detection of isolated fH-derived SCR2, 7 and 13 by dot blot analysis.

**Figure 2:** Following the coupling procedure with SCR7, Ab-SCR constructs comprising the binding molecules 2G12, 4E10, 3D6 and 2F5 as defined in Table 1 were examined using Western blot analysis on a 12% SDS-page gel under reducing conditions. The constructs were visualized by a HRP-labeled anti-HIS-tag-mAb, which recognizes the SCR. Both, the heavy and the light chain shifted from 50kD or 25kD, respectively to about 60 kD and 35kD respectively corresponding to the molecular weight of the binding molecules plus the coupled SCR.

**Figure 3:** Slot blot analysis of fractions eluted from a heparin column by a salt gradient

**Figure 4:** Analysis of fractions eluted from a heparin column by a salt gradient by Silver staining of a SDS-page electrophoresis gel.

**Figure 5:** Lysis of MMTV by fH depleted serum. Isolated virus was incubated with normal mouse serum (NMS; dark green and cyan line), in RPMI 1640 without any supplement as input control (red and green line) or in NMS containing less than 5% fH, designated as fH-depleted serum (brown and blue line). All sera were diluted 1:10 in RPMI 1640. The RNA of remaining MMTV (which was not lysed by mouse complement) was isolated and amplified by reverse transcriptase real time-PCR. An increase of about 3.3 in the Ct value corresponds to a reduction of about 1 log in viral titer. Thus fH depleted serum (Δ-fH serum) reduces the amount of MMTV at more than 5 log, since the Ct value of the control sera increased from about 20.2 (red and pink line, Ct values 20.0, 20.4) to 36.5 (pink and blue line, Ct values 37.3 and 35.7).

**Figure 6:** Lysis of FV by SCR-Ab construct. Isolated virus was incubated with normal mouse serum (NMS; red and brown line), in RPMI 1640 without any supplement as input control (cyan and pink line) or in NMS containing SCR7 coupled to the Ab#48 (SCR7Ab#48; brown and blue line). All sera were diluted 1:10 in RPMI 1640. The RNA of remaining FV (which was not lysed by mouse complement) was isolated and amplified by reverse transcriptase real time-PCR. An increase of about 3.3 in the Ct value corresponds to a reduction of about 1 log in viral titer. Thus the SCR-Ab construct reduces the amount of FV at more than 2 log, since the Ct value of the control sera increased from about 17.0 (red and brown line, Ct values 17.0, 17.0) to 23.3 (brown and blue line, Ct values 23.2 and 23.4).

**Figure 7:** The amount of infected cells in the spleen of FV-infected mice was determined by FACS analysis. While in infected animals about 15% of the cells are FV-antigen positive, is the amount of infected cells drastically reduced when mice were treated in addition with SCR7, 13 and 18-20 coupled to the virus-specific antibody Ab#48. The control construct (SCR2-Ab#48) showed a slight reduction of infected cells too, probably mediated by the Ab.

**Figure 8:** Lysis of HIV by fH-derived SCR 7. HIV was incubated with NHS, SCR7 coupled to the HIV-specific mAb 3D6 (constr), with not-coupled mixed SCR and the antibody 3D6 (SCR7 + 3D6) or with the isolated antibody (3D6). A detergent (Igepal) indicates 100% lysis.

**Figure 9:** Infection assay in the presence of an fH-derived construct. Following lysis, samples were applied to peripheral blood mononuclear cells (PBMCs) and incubated for 5 days. While NHS induced vigorous infection of the cells, the construct reduced the amount of produced HIV drastically. The mix of the uncoupled compounds had no effect. The viral replication was quantified by a standard p24 ELISA at day 5 post infection. Again, coupling of the SCR to the antibody was a pre-requisite for an efficient reduction of the viral titre.

**Figure 10:** Lysis of HIV-1 by SCR9 or SCR7. HIV-1 was incubated with NHS (1:10 diluted in RPMI1640) containing SCR7 or SCR9 or NHS (1:10 diluted in RPMI1640) without any further supplement. As further control Igepal (100% lysis) and HIV in heat-inactivated NHS (HIV, background lysis) was used. Compared to HIV or HIV incubated with NHS, the addition of SCR7 or SCR9 induced vigorous virolysis of HIV. Thus, also SCR9 is capable of improving the effector function of antibodies.

**Figure 11:** Lysis of MHV by fh-derived SCR 13. Mouse Hepatitis Virus (MHV), a member of the coronavirus family, was incubated with normal mouse serum (NMS; line 4,5), or in NHS containing a control SCR (line 6,7) or NMS with SCR13 (line 8,9). After incubation for 45min, the RNA of remaining MHV (which was not lysed by mouse complement) was isolated and amplified by RT-PCR to detect the MHV specific PCR product at around 380bp. While control construct or NMS were unable to reduce the viral RNA, SCR13 induced the complete destruction of MHV. This experiment shows that SCR13 is effective not only by retroviruses (HIV, SIV, FV, MMTV) but also with viruses from other families.

**Figure 12:** Lysis of tumour cells by fH-derived SCR 7. SOK3-cells (human ovarian adenocarcinoma cells) were incubated with human serum in the absence (left) or presence (right) of an fH-derived SCR (SCR7). C3-deposition (FL-1) and PI-staining (FL-2) revealed that the SCR induced more C3-deposition and increased the lysis of the tumour cells from 13% to 24%.

**Figure 13:** Reduction of the B cell counts by the presence of fH-derived SCRs. The amount of Raji cells, a Human B lymphocyte Burkitt's lymphoma cell line, was drastically reduced in the presence of fH-derived SCRs already in the absence of an anti-CD20 antibody. The effect was additionally enhanced when anti-CD20 was cross-linked with an anti-IgG antibody.

**Figure 14:** Purification of an illustrative artifical SCR (aSCR*i*). An optimized SCR was expressed in the P. pastoris system and purified via Heparin colums as by standard methods. The purified product was identifie by Western blot analysis and vizualized via the binding of POX-labelled antibodies against the His-tag of the illustrative aSCR. The blot shows two broad band representing concentrated monomeric and dimeric illustrative aSCRs, the smear is the due to the glycosylation of the protein. The bound protein was released from the heparin column after elution with high salt buffer (eluate), no aSCR was found in the flow trough (FT).

**Figure 15:** Lysis of HIV-1 by aSCR. HIV-1 was incubated with NHS (1:10 diluted in RPMI1640) containing illustrative artificial SCR (aSCR*i*) in different concentrations or NHS (1:10 diluted in RPMI1640). All samples contained in addition the HIV-1 specific mAb 2G12 in a 1:500 dilution to enhance complement activation. As further control Igepal (100% lysis) and HIV in heat-inactivated NHS (hiNHS, background lysis) was used. Compared to HIV or HIV incubated with NHS, the addition of aSCR*i* in an equimolar amount did not enhance CoML (aSCR-1), while a 120-fold excess (in relation to fH) induced vigorous virolysis of HIV and eliminated the virus nearly to 100% (aSCR-120). Thus, also artificial SCRs are capable of improving the effector function, when coupled to antibodies.

The present invention is additionally described by way of the following illustrative non-limiting examples that provide a better understanding of the present invention and of its many advantages.
The following examples illustrate the invention:

**EXAMPLES**

**Example 1: Preparation of short consensus repeat-antibody construct**

Codon-optimized plasmids comprising the genetic sequences encoding for the complement factor H-derived short consensus repeats (fH-SCRs) SCR2, SCR7, SCR9, SCR13 and SCR18-20 were purchased from GeneArt.
The SCR polynucleotide sequences encoding SCR2, SCR7, SCR9, SCR13 and SCR18-20 were amplified using the following primer sets (primers manufactured by Metabion):

5'Primer mouse SCR2: aaaaagaattctcgaccaaaaaac (SEQ ID NO:9)
3'Primer mouse SCR2: aaaaagtctagaccctcgagg (SEQ ID NO:10)
5'Primer mouse SCR7: aaaaagaattctcgaccaaagtg (SEQ ID NO:11)
3'Primer mouse SCR7: aaaactctagatcgagaataatac (SEQ ID NO:12)
5'Primer human SCR7: aaaaagaattcctcagaaaatgt (SEQ ID NO:33)
3'Primer human SCR7: aaaatctagagggatgcatc (SEQ ID NO:34)
5'Primer human SCR9: aaagaattcaaatcttgtgatatc (SEQ ID NO:35)
3'Primer human SCR9: aaaatctagaggataacatatgg (SEQ ID NO:36)
5'Primer mouse SCR13: aaaaagaattctcgaccaaagcg (SEQ ID NO:13)
3'Primer mouse SCR13: aaaaatctagatcgagaatggtg (SEQ ID NO:14)
5'Primer human SCR13: gattgggtaccgacaagtt (SEQ ID NO:37)
3'Primer human SCR13: ggagctctagacccatgg (SEQ ID NO:38)
5'Primer mouse SCR18-20: aaatcgatgaaagataacagctgcgttg (SEQ ID NO:15)
3'Primer mouse SCR18-20: aatctagacgcacgcaggtcggatag (SEQ ID NO:16)5'Primer human SCR18-20: aaaagaattcgacacctcctg (SEQ ID NO:41)
3'Primer human SCR18-20: aaaatctagaggtctttttgcac (SEQ ID NO:42)

The synthetic SCRs mSCR2, mSCR7, hSCR7, hSCR9, mSCR13, hSCR13, mSCR18-20 and hSCR18-20 (all GeneArt) were amplified by Real-Time PCR (BioRad) with the following protocol (Brillant SYBR-Green Q-PCR, BioRad):

| | |
|---|---|
| DNA | 5 µl ($10^{-4}$ dilution of the original vector sample) |
| Primer for | 1 µl |
| Primer rev | 1 µl |
| 2x Supermix | 20 µl |
| $H_2O$ | 13 µl |
| | 40µl |

| | |
|---|---|
| PCR profile: | |
| initial denaturing | 95°C 8,5' |
| 10 cycles | 95°C 15" |
| | 45°C 55" |
| 35 cycles | 95°C 15" |
| | 62°C 55" |
| 70 cycles | 60°C 8" (melting point analysis) |

The PCR products were analysed by agarose gel electrophoresis (2%TAE) and the specific bands were extracted and purified (Gel extraction Kit, Qiagen). Samples were digested with specific enzymes (Fermentas) and ligated into their destined vector (also digested with suitable enzymes). The list below gives an overview of cloning details:

| | |
|---|---|
| mSCR2 | in pPICZalphaA, restriction sites: EcoRI (5-prime) und XbaI (3-prime) |
| mSCR7 | in pPICZalphaA, restriction sites: EcoRI (5-prime) und XbaI (3-prime) |
| hSCR7 | in pPICZalphaA, restriction sites: EcoRI (5-prime) und XbaI (3-prime) |
| hSCR9 | in pPICZalphaA, restriction sites: EcoRI (5-prime) und XbaI (3-prime) |
| mSCR13 | in pPICZalphaA, restriction sites: EcoRI (5-prime) und XbaI (3-prime) |
| hSCR13 | in pPICZalphaA, restriction sites: KpnI (5-prime) und XbaI (3-prime) |
| mSCR18-20 | in pPICZalphaC, restriction sites: ClaI (5-prime) und XbaI (3-prime) |
| hSCR18-20 | in pPICZalphaA, restriction sites: EcoRI (5-prime) und XbaI (3-prime) |

The PCR resulted in the synthesis of DNA molecules consisting of the following polynucleotide sequences, comprising an EcoRI restriction site at the 5' end (underlined) and a XbaI (SCR2, 7, 9, 13 and human SCR18-20) or a ClaI restriction site (mouse SCR18-20) at the 3' end (double underlined):

mouse SCR2:

gaattctcgaccaaaaaaccgtgtggtcatccgggtgataccccgtttggtagctttcgtctggcggttggtagccagtttgaatttggcg
cgaaagtggtgtatacctgcgatgatggctatcagctgctgggcgaaattgattatcgtgaatgcggtgcggatggctggattaacgata
ttccgctgtgcgaaatcctcgagggtctaga (SEQ ID NO:1);

mouse SCR7:

gaattctcgaccaaagtgcgcaaatgtgtgttccactacgtggaaaacggtgatagcgcgtactgggaaaaagtgtatgttcagggcca
gagcctgaaagtgcagtgctataacggctatagcctgcagaatggccaggataccatgacctgcaccgaaaatggttggagcccgcc
gccgaaatgtattattctcgatctaga (SEQ ID NO:3);

human SCR7:

gaattcctcagaaaatgttattttccttatttggaaaatggatataatcaaaattatggaagaaagtttgtacagggtaaatctatagacgttgcctgccatcctggctacgctcttccaaaagcgcagaccacagttacatgtatggagaatggctggtctcctactcccagatgcatccctctaga (SEQ ID NO:21);

human SCR9:

gaattcaaatcttgtgatatcccagtatttatgaatgccagaactaaaaatgacttcacatggtttaagctgaatgacacattggactatgaatgccatgatggttatgaaagcaatactggaagcaccactggttccatagtgtgtggttacaatggttggtctgatttacccatatgttatcctctaga (SEQ ID NO:23);

mouse SCR13:

gaattctcgaccaaagcgaccgatcagctggaaaaatgccgcgttctgaaaagcaccggcatcgaagcgattaaaccgaaactgaccgaatttacccacaacagcaccatggattacaaatgccgcgataaacaggaatatgaacgcagcatttgcatcaacggcaaatgggatccggaaccgaattgcaccattctcgatctaga (SEQ ID NO:5);

human SCR13:

ggtaccgataaacttaagaagtgcaaatcatcaaatttaattatacttgaggaacatttaaaaaacaagaaggaattcgatcataattctaacataaggtacagatgtagaggaaaagaaggatggatacacacagtctgcataaatggaagatgggatccagaagtgaactgctcaatgggtctaga (SEQ ID NO:25);

mouse SCR18-20:

atcgatgaaagataacagctgcgttgatccgccgcatgttccgaatgcgaccattgtgacccgcaccaaaaacaaatatctgcacggcgatcgtgtgcgttatgaatgcaacaaaccgctggaactgtttggtcaggttgaagtgatgtgcgaaaacggcatctggaccgaaaaaccgaaatgccgtgatagcaccggtaaatgtggtccgccgccgccgattgataatggcgatatcaccagcctgagcctgccggtttatgaaccgctgagcagcgtggaatatcagtgccagaaatattatctgctgaaaggcaaaaaaaccatcacctgcaccaacggtaaatggagcgaaccgccgacctgtctgcatgcgtgtgtgattccggaaaacatcatggaaagccacaacatcattctgaaatggcgccacaccgaaaaaatctatagccacagcggcgaagatattgaattcggctgtaaatatggctattacaaagcgcgtgatagcccgccgtttcgtaccaaatgcatcaacggcaccattaactatccgacctgcgtgcgtctaga (SEQ ID NO:7);

and
human SCR18-20:

gaattcgacacctcctgtgtgaatccgcccacagtacaaaatgcttatatagtgtcgagacagatgagtaaatatccatctggtgagaga gtacgttatcaatgtaggagcccttatgaaatgtttggggatgaagaagtgatgtgtttaaatggaaactggacggaaccacctcaatgc aaagattctacaggaaaatgtgggccccctccacctattgacaatggggacattacttcattcccgttgtcagtatatgctccagcttcatc agttgagtaccaatgccagaacttgtatcaacttgagggtaacaagcgaataacatgtagaaatggataatggtcagaaccaccaaaat gcttacatccgtgtgtaatatcccgagaaattatggaaaattataacatagcattaaggtggacagccaaacagaagctttattcgagaac aggtgaatcagttgaatttgtgtgtaaacggggatatcgtctttcatcacgttctcacacattgcgaacaacatgttgggatgggaaactgg agtatccaacttgtgcaaaaagacctctaga (SEQ ID NO:29).

The above described polynucleotides encoding SCR2, SCR7, SCR9, SCR13 and human SCR18-20 were cloned into pPICZalphaA (Invitrogen) via the EcoRI (5-prime) and XbaI (3-prime) cloning sites. The mouse SCR18-20 was cloned into pPICZalphaC (Invitrogen) via the EcoRI (5-prime) and the ClaI (3-prime) cloning site. The correct reading frame was confirmed by sequencing. The vectors were transfected into pichia pastoris yeast strain X-33 according to the protocol of the manufacturer (Invitrogen) and positively selected using Zeocin. Positive yeast clones were cultured at 30°C for 96h and expression was induced by adding repeatedly 1% Methanol (each 24h). Expressing clones were analysed by dot blotting.

The resulting complement factor H-derived short consensus repeat (fH-SCR) polypeptides are defined by the following amino acid sequences:

Mouse SCR2:

STKKPCGHPGDTPFGSFRLAVGSQFEFGAKVVYTCDDGYQLLGEIDYRECGADGWIN DIPLCEILE (SEQ ID NO:2);

Mouse SCR7:

STKVRKCVFHYVENGDSAYWEKVYVQGQSLKVQCYNGYSLQNGQDTMTCTENGW SPPPKCIIL (SEQ ID NO:4);

Human SCR7:

EFLRKCYFPYLENGYNQNYGRKFVQGKSIDVACHPGYALPKAQTTVTCMENGWSPT PRCIPL (SEQ ID NO:22);

Human SCR9:

EFKSCDIPVFMNARTKNDFTWFKLNDTLDYECHDGYESNTGSTTGSIVCGYNGWSDL PICYPL (SEQ ID NO:24);

Mouse SCR13:

STKATDQLEKCRVLKSTGIEAIKPKLTEFTHNSTMDYKCRDKQEYERSICINGKWDPE PNCTIL (SEQ ID NO:6);

Human SCR13:

GTDKLKKCKSSNLIILEEHLKNKKEFDHNSNIRYRCRGKEGWIHTVCINGRWDPEVN CSMGL (SEQ ID NO:26);

Mouse SCR18-20:

KDNSCVDPPHVPNATIVTRTKNKYLHGDRVRYECNKPLELFGQVEVMCENGIWTEK PKCRDSTGKCGPPPPIDNGDITSLSLPVYEPLSSVEYQCQKYYLLKGKKTITCTNGKW SEPPTCLHACVIPENIMESHNIILKWRHTEKIYSHSGEDIEFGCKYGYYKARDSPPFRT KCINGTINYPTCV (SEQ ID NO:8);

and
Human SCR18-20:

EFDTSCVNPPTVQNAYIVSRQMSKYPSGERVRYQCRSPYEMFGDEEVMCLNGNWTE PPQCKDSTGKCGPPPPIDNGDITSFPLSVYAPASSVEYQCQNLYQLEGNKRITCRNGQ WSEPPKCLHPCVISREIMENYNIALRWTAKQKLYSRTGESVEFVCKRGYRLSSRSHTL RTTCWDGKLEYPTCAKRPL (SEQ ID NO:30).

The SCRs were purified from the yeast supernatant via NiNTA-columns as recommended by the manufacturer (Qiagen). Fractions positive in slot blots (see Figure 1) were dialysed against PBS.

Monoclonal antibody clone #48; (Ab #48; Chesebro et al. (1981) Virology 112(1): 131-44) that specifically recognizes the envelope protein of Friend Murine Leukaemia Virus (FV) was purified from the supernatant of hybridoma cells by G-Sepharose (Amersham) according to the protocol of the manufacturer.

Equimolar amounts of fH-SCR and Ab#48 antibody molecules were cross-linked using Sulfo-SMPB as recommended by the manufacturer (Pierce). The resulting short consensus repeat-antibody constructs (SCR-Ab) were purified by spin filters (Zeba). The successful cross-linking of the fH-SCR with the Ab#48 antibody molecules was analyzed by western blotting (see Figure 2).

**Example 2: SCR binding assay**

To show that the generated SCRs bind to negatively charges surfaces, supernatants of the transfected and induced yeast strains was applied to a heparin column. For this, 20 ml hSCR18-20 were centrifuged, the resulting supernatant (SN) diluted 1:2 with $dH_2O$ and sterile filtered (0,2μm sterile filter). The treated SN was applied to heparin affinity chromatography using a 1 ml HiTrap heparin column (GE healthcare) and a Pharmacia FPLC-System according to the following protocol:

Column: HiTrap Heparin HP (Amersham)

| | |
|---|---|
| Buffer A: | 1/3 PBS |
| Buffer B: | 1xPBS + 1 M NaCl |
| flow-rate: | 1ml/min |
| load: | 3x40ml prepared supernatant (1:2 $H_2O$) |
| wash: | buffer A |
| Gradient: | linear up to 100% buffer B → hold for 10 min |
| equilibration: | buffer A |

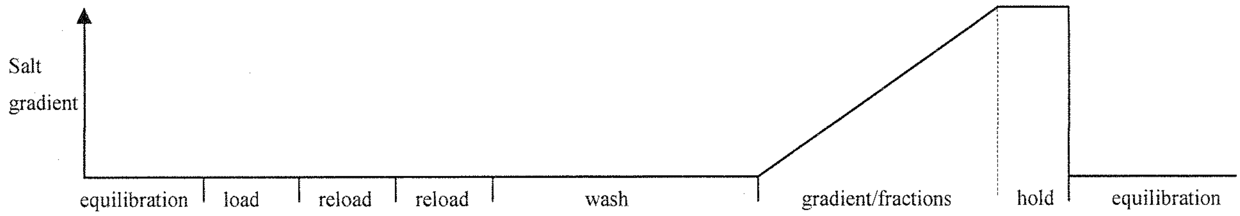


The flow-through was collected and 2x reloaded. The column was washed extensively with low salt buffer (1/3 PBS) and bound proteins were eluted using a linear salt gradient, ranging from 50mM to 1 M NaCl (high salt buffer: PBS with 1 M NaCl) in a total volume of 100 ml. Individual fractions of 2 ml were collected, and the presence of hSCR18-20 was assayed by SlotBlotting (polyclonal serum goat-anti-humanFH 1:1.000 (Quidel); rabbit-anti-goat HRP conjugated 1:2.000 (Dako) (Figure 3). To check the purity of the collected fractions, silver staining of a SDS-PAGE gel (15%) under reducing conditions was performed (Figure 4). The fractions, positive in the slot blot showed the expected band around 32kd on the SDS-PAGE gel, the smear around 32 is due to the glycosylation of the SCR.

**Example 3: In vitro lysis by fH depleted serum**

Isolated FV virus was incubated with normal mouse serum (NMS), in RPMI 1640 without any supplement as input virus control or in NMS containing less than 5% fH, designated as fH-depleted serum (Δ-fH-serum). All sera were diluted 1:10 in RPMI 1640. The RNA of remaining FV (which was not lysed by mouse complement) was isolated and amplified by reverse transcriptase real time-PCR. Virus was isolated by centrifugation (Hermle Z382K, Rotor 220.87V01, 1h/25.000 g/4°C) and quantified by Real-time RT-PCR. For this, viral RNA was isolated with the Viral RNA Mini Kit (Qiagen). 5 μl of eluted RNA either from in vitro assays or revealed from serum was taken as template for Real-time RT-PCR according to the following protocol: The PCR-Mix contained RT-PCR Reaction Mix, iScript Reverse Transcriptase, nuclease-free water (iScript One-Step RT-PCR Kit, BioRad), F-MuLV env-specific fluorogenic PCR probe (5'-FAM ACT CCC ACA TTG ATT TCC CCG, Metabion), upstream primer 5'-AAGTCTCCCCCCGCCTCTA-3' (SEQ ID NO: 17) and downstream primer 5"-AGTGCCTGGTAAGCTCCCTGT-3" (SEQ ID NO: 18). The viral RNA was transcribed to cDNA using the following PCR cycle profile: 30 min 45°C, 15 sec 95°C, 30 sec 60°C (iCycler, BioRad). Real-time PCR and RT-PCR amplifications were performed in 25 μl reaction mixture with BioRad iScript One-Step RT-PCR Reaction Mix (BioRad), using an iCycler (BioRad). Comparisons between groups were made using differences in critical threshold values ($C_t$ value). Experiments were performed in duplicates. An increase of about 3.3 in the Ct value corresponds to a reduction of about 1 log in viral titer; see following Table 3.

| Sample | Ct value | mean |
|---|---|---|
| input virus | 24.1 | |
| | 25.9 | 25.0 |
| NMS | 24.6 | |
| | 24.4 | 24.5 |
| fH-depleted NMS | 32.2 | |
| | 33.8 | 33.0 |

Thus fH depleted serum reduces the amount of FV at around 3 logs, since the Ct value of the control sera increased from Ct 24.5 to 33.0.

Similarly, Mouse Mammary Tumour Virus (MMTV), a further murine retrovirus, is efficiently lysed by complement and the viral titre is reduced for about five logs; see Figure 5 and the following Table 4:

| Sample | Ct value | mean |
|---|---|---|
| input virus | 18.7 | |
| | 19 | 18.85 |

(continued)

| Sample | Ct value | mean |
|---|---|---|
| NMS | 20 | |
| | 20.4 | 20.2 |
| fH-depleted NMS | 37.3 | |
| | 35.7 | 36.5 |

The Ct values were determined by RT-PCR according to the following protocol: MMTV was incubated with NMS or fH-depleted serum (1:10 final dilution) in RPMI 1640 without any supplement at 37°C for 15min in the presence of RNAse. Lysis was stopped by the addition of the AVL buffer from the viral RNA isolation kit (Qiagen) to inactivate the RNAse. RNA isolation was performed as described in the kit by the manufacturer. To quantify the amount of MMTV after lysis, real time RT-PCR was performed using the QRT-PCR kit from Stratagene at 45°C for 30 min followed by PCR of the cDNA in the presence of SybrGreen. The following primers were used:

MMTV for: 5'-TCTTTTGCGCACAACCCATCAA-3' (SEQ ID NO: 19)

MMTV rev: 5'-AAGGCCATGTTTGTTAAGGGC-3' (SEQ ID NO: 20)

The cDNA was amplified using the following PCR cycle profile: 15 sec 95°C, 30 sec 60°C for 50 cycles (iCycler, BioRad). Experiments were performed in duplicate.

**Example 4: In vitro lysis induced by SCR-Ab constructs**

The capacity of the herein described SCR-Ab constructs to enhance complement dependent lysis was first demonstrated using an in vitro lysis assay. Therefore, $10^3$ spleen focus forming units (SFFU) of Friend virus were mixed with the construct containing SCR7, SCR13, SCR18-20 or SCR 2, the uncoupled mixture of Ab#48 antibody and SCR7 (Ab#48 single / SCR single), the antibody alone (Ab#48 single) or SCR alone (SCR single) in a ten-fold excess and incubated with normal mouse serum (NMS) in a 1:10 dilution for 30min. As a control, fH-depleted mouse serum (fH depleted NMS) was used. Virus was isolated by centrifugation (Hermle Z382K, Rotor 220.87V01, 1h/25.000 g/4°C) and quantified by Real-time RT-FCR. For this, viral DNA was isolated with the Viral RNA Mini Kit (Qiagen). 5 μl of eluted RNA either from in vitro assays or revealed from serum was taken as template for Real-time RT-PCR according to the following protocol: The PCR-Mix contained RT-PCR Reaction Mix, iScript Reverse Transcriptase, nuclease-free water (iScript One-Step RT-PCR Kit, BioRad), F-MuLV env-specific fluorogenic PCR probe (5'-FAM ACT CCC ACA TTG ATT TCC CCG, Metabion), upstream primer 5'-AAGTCTCCCCCCGCCTCTA-3' (SEQ ID NO: 17) and downstream primer 5'-AGTGCCTGGTAAGCTCCCTGT-3' (SEQ ID NO: 18). The viral RNA was transcribed to cDNA using the following PCR cycle profile: 30 min 45°C, 15 sec 95°C, 30 sec 60°C (iCycler, BioRad). Real-time PCR and RT-PCR amplifications were performed in 25 μl reaction mixture with BioRad iScript One-Step RT-PCR Reaction Mix (BioRad), using an iCycler (BioRad). Experiments were performed in duplicates.

The results shown in the following Table 5 clearly indicate that FV is lysed only when the SCRs are coupled to an FV specific antibody molecule. As an example the cross-linked Ab#48 to SCR7 (SCR7-Ab#48) molecule is given in Figure 6. Even at low concentrations, the SCR-Ab construct reduced the viral titre similar as an fH-depleted mouse serum; see following Table 5:

| Sample | Ct value | mean |
|---|---|---|
| NMS | 17 | |
| | 17 | 17 |
| fH depleted NMS | 24,9 | |
| | 23,6 | 24.3 |
| Ab#48 single | 16,6 | |
| | 16,1 | 16.4 |

(continued)

| Sample | Ct value | mean |
|---|---|---|
| SCR7-Ab#48 | 23,2 | |
| | 23,4 | 23.3 |
| Ab#48 single/ SCR7 single | 16,1 | |
| | 16,5 | 16.3 |

In contrast, the mixture of uncoupled SCR (SCR single) and Ab#48 (Ab#48 single) was unable to induce complement-mediated lysis (CoML; Table 5). Constructs in which SCR13 or SCR18-20 were cross-linked to the Ab#48 behaved similar and induced CoML (data not shown).

**Example 5: In vivo assessment of SCR-Ab constructs.**

Furthermore, the herein described SCR-Ab constructs were tested in vivo using BALB/c mice as this mouse strain is highly susceptible to FV infection.

For that reason, 5μg of the respective SCR-Ab constructs was mixed with FV (500 SFFU/animal) for 30min on ice in a total volume of 500μl and applied to BALB/c mice (purchased from Charles River, Germany) via the tail vein. Control animals obtained 500 SFFU FV in 500μl PBS without any supplement.

After one week, animals were sacrificed and the following parameters were determined: infectious centres in the spleen (infectious centre assay) and infected cells in the spleen (FACS analysis).

Serial dilutions of spleen cells from infected mice were plated onto susceptible Mus dunni cells, co-cultivated for 3 days, fixed with ethanol, stained with F-MuLV envelope-specific Mab 720 (Dittmer (1998) J Virol 72: 6554-8), and developed with peroxidase-conjugated goat anti-mouse IgG and substrate to detect foci of infected cells.

The determination of infectious centres in the spleen revealed that the constructs which contained SCRs which bind to negatively charged surfaces were able to reduce the infection. While control constructs (SCR2-Ab#48) or infection in the absence of any Ab and SCR (positive control) induced no protection against FV infection, the SCR-Ab constructs containing SCR7, 13, or 18-20 or a mixture of SCR7,13, 18-20 (mix) reduced the infectious centres up to 3 logs; see Table 6 as listed herein below:

| | | infectious centres in IC-Assay | | | | |
|---|---|---|---|---|---|---|
| **Application** | **Nr** | **$10^5$** | **$10^4$** | **$10^3$** | **$10^2$** | **$10^1$** |
| no FV (negative control) | 1 | / | / | / | / | / |
| | 2 | / | / | / | / | / |
| | 3 | / | / | / | / | / |
| No Ab, no SCR (positive control) | 4 | Na | na | 86 | 5 | 5 |
| | 5 | Na | na | 60 | 11 | 5 |
| SCR2-Ab#48 | 8 | Na | na | 51 | 27 | 6 |
| | 9 | Na | na | 22 | 5 | 1 |
| SCR7-Ab#48 | 13 | 47 | 4 | / | / | / |
| | 14 | Na | 84 | 13 | 2 | / |
| SCR13-Ab#48 | 15 | Na | 29 | / | / | / |
| | 16 | 73 | 11 | 1 | / | / |
| SCR18-20-Ab#48 | 17 | 61 | 5 | / | 1 | / |
| | 18 | 17 | 5 | / | / | / |
| SCR7-Ab#48, SCR13-Ab#48 and SCR18-20-Ab#48 (Mix) | 10 | Na | 64 | 9 | / | / |
| | 11 | Na | 7 | / | / | / |
| | 12 | Na | 46 | 3 | / | / |

Thus, only when SCRs 7, 13 or 18-20 were coupled to the virus-specific Ab, the amount of infectious centres was drastically reduced (about 3 logs) when compared to the control SCR, the absence of any constructs; see Table 6. As expected, uninfected control mice (negative control) gave no FV-specific signal in this assay.

In a second set of experiments, a mixture of uncoupled SCRs and Ab#48 (Ab#48 single/ SCR7 single; equimolar amount of Ab and SCR, total 5μg) was applied to the animals via the tail vein. After 6h, BALB/c mice were infected with FV (500 SFFU) again via the tail vein. After one week, animals were sacrificed and the infection of the mice was determined as described above.

Titration of infectious centres in the spleen on a Reporter cell line (IC-Assay) and detection of infectious centres by FACS analysis of infected animals treated with a mixture of uncoupled SCRs and virus-specific Ab is shown in Table 7 as listed herein below:

| Nr | Application | infected cells in the spleen (FACS) | | infectious centres in IC-Assay (MW $10^4$) |
|---|---|---|---|---|
| 1 | no FV (negative control) | 0,74 | | |
| 2 | | 6,83 | | |
| 3 | | 10,77 | | |
| 4 | No Ab, no SCR (positive control) | 5,4 | 8,86 | 25 |
| 5 | | 5,91 | | |
| 6 | | 15,42 | | |
| 7 | | 3,5 | | |
| 8 | | 1,25 | | |
| 9 | Ab#48 single | 1,73 | 4,2 | 11,4 |
| 10 | | 2,42 | | |
| 11 | | 5,64 | | |
| 12 | | 9.32 | | |
| 13 | | 5,07 | | |
| 14 | Ab#48 single / SCR2 single | 3,62 | 4,76 | 12,6 |
| 15 | | 2,74 | | |
| 16 | | 3,08 | | |
| 17 | | 1,77 | | |
| 18 | | 10,08 | | |
| 19 | Ab#48 single / SCR7 single | 3,86 | 5,49 | 41 |
| 20 | | 5,33 | | |
| 21 | | 6,42 | | |
| 22 | | (-0,8) | | |
| 23 | | (-8,7) | | |
| 24 | Ab#48 single / SCR13 single | 5,87 | 4,77 (0,96) | 34,4 |
| 25 | | 3,8 | | |
| 26 | | 4,65 | | |

As expected, the mixture of uncoupled SCRs and Ab#48 had no effect. Neither the amount of infected cells in the spleen, nor the infectious centres in the IC assay were significantly reduced when compared to the control settings in the absence of any construct, the Ab#48 alone, Ab#48 together with control SCR2 or the uncoupled mixtures of SCRs 7, 13 or 18-20 and Ab #48; see Table 7.

To determine the amount of FV infected cells in the spleen FACS analyses was performed. Suspensions of spleen cells were incubated with biotinylated monoclonal antibody clone #34; (Ab #34; Chesebro et al. (1981) Virology 112(1): 131-44) recognizing F-MuLV glyco-Gag on the surface of infected cells. After washing step, spleen cells were incubated with FITC-conjugated streptavidine (DAKO). The fluorescence signal was analyzed with a Becton Dickinson FACScan flow cytometer using CellQuest software.

The viral load in the spleen is measured by measuring the percent of infected cells with FACS analysis and by titration of spleen cells using a reporter cell line. The FACS analysis showed that the SCR-Ab constructs reduced the amount of FV infected cells (0.5-2.5 of infected cells in the spleen) compared to the absence of the constructs (FV, 15% infected cells) or a control SCR coupled to the Ab#48 (7% infected cells, see Figure 7).

**Example 6: In vitro lysis induced by SCR-Ab constructs**

To determine the induction of CoML by the cross-linked SCR-3D6 constructs, HIV-1 was pre-incubated with the SCR-3D6 in RPMI- for about 10min on ice. NHS was added in a 1:10 dilution. Quickly 1μg/μl RNase A was added and the samples were incubated at 37°C for an hour. To compare the effect of the SCR-3D6 (constr.-SCR7 in Fig. 8) to the uncoupled compounds, some of the samples contained uncoupled SCR and Ab (SCR7+3D6) or the Ab alone, without the SCR (3D6; Fig. 8). As positive control 1% Igepal in RPMI- was added to determine 100% lysis. As negative controls one sample contained the input virus without any supplement and one sample consisted of input virus and NHS. After incubation the virus was pelleted for 60min at 15000rpm. The RNA of the virus pellet was isolated by a viral RNA-isolation kit (Qiagen) as recommended by the manufacturer and analysed by real-time RT-PCR using the Taqman-Kit from Biorad. The PCR-Mix contained iScript Reverse transcriptase, nuclease-free water, HIV-specific MGB-probe (5'-**FAM**-CTG CAG AAT GGG A-**mgb**-3', Applied Biosystems), upstream primer 5'-ATG TTA AAA GAG ACC ATC AAT GA-3' (SEQ ID NO:43; Metabion) downstream primer 5'-CTA TGT CAC TTC CCC TTG GT-3'(SEQ ID NO:44; Metabion) and the viral RNA template. The amplification profile started with 50°C for 10min, and 98°C for 5min. The following 50 cycles had 95°C,15sec, 54°C for 30sec. The reaction was performed in 40μl reaction mixture in duplicates.

**Example 7: In vitro lysis of HIV-1 induced by SCR9**

To determine the induction of CoML by SCR9, HIV-1 virus was incubated with the in RPMI-medium for about 10min on ice together with normal human serum (NHS) in a 1:10 dilution. Quickly 1 μg/μl RNase A was added and the samples were incubated at 37°C for an hour. To compare the effect of the SCR9 (SCR9 in Fig. 10) to the other SCRs, uncoupled SCR7 was included. Both SCRs were applied in a 300molar excess calculated to the content of fH in the system. As positive control 1% Igepal in RPMI-medium was added to determine 100% lysis. As negative controls one sample contained the input virus with heat inactivated serum (input control in Fig. 10) and one sample consisted of input virus and NHS. After incubation the virus was pelleted for 60min at 15000rpm. The RNA of the virus pellet was isolated by a viral RNA-isolation kit (Qiagen) as recommended by the manufacturer and analysed by real-time RT-PCR using the Taqman-Kit from Biorad. The PCR-Mix contained iScript Reverse transcriptase, nuclease-free water, HIV-specific MGB-probe (5'-**FAM**-CTG CAG AAT GGG A-**mgb**-3', Applied Biosystems), upstream primer 5'-ATG TTA AAA GAG ACC ATC AAT GA-3' (SEQ ID NO:43; Metabion) downstream primer 5'-CTA TGT CAC TTC CCC TTG GT-3' (SEQ ID NO:44; Metabion) and the viral RNA template. The amplification profile started with 50°C for 10min, and 98°C for 5min. The following 50 cycles had 95°C for 15sec, 54°C for 30sec. The reaction was performed in 40μl reaction mixture in duplicates. Considering Examples 4, 5 and 6, the coupling of SCR9 with a pathogen-specific binding molecule is considered to lead to an even higher activity.

**Example 8: HIV-1 Infection assay in the presence of SCR constructs**

After the lysis experiment as described above (initial viral concentration contained 40ng HIV-1 NL4-3 p24 equivalents) the pelleted samples (Hermle Z382K, Rotor 220.87V01, 1h/25.000 g/4°C) were resuspended and transferred to a u-bottom 96-well plate containing 100000 PBMCs in RPMI supplemented with IL-2 and 10% FCS. The plate was then incubated over night at 37°C. The next day the cells were washed and resuspended in fresh medium. 10μl of each sample were taken and lysed 1:10 in 1% Igepal and stored at -80°C for the p24 ELISA experiment. These samples were supposed to be day 0 of infection. Further samples were taken on day 5 and day 10 of infection. The amount of produced virus was determined by a p24-ELISA as described (Stoiber (1996) loc. cit.). The results of the HIV-1 infection assay are described in **Fig. 9**.

**Example 9: In vitro MHV-lysis-assay**

Mouse Hepatitis Virus A59 (MHV; 20μl of a stock with $3x10^8$ plaque forming Units), a member of the coronavirus family, was incubated with SCR13 or SCR2 (control-SCR, both in a 100-fold excess) before NMS was added in a 1:5 dilution. Samples were incubated for 30min at 37°C. The RNA of the lysed viruses was digested by RNAse. After centrifugation for 1h at 13000 rpm at 4°C, the supernatant was discarded and the pellet of the remaining non-lysed MHV was resuspended in RPMI without any supplement before the viral RNA was isolated with the QIAGEN kit as recommended by the manufacturer. Isolated RNA was amplified by RT-PCR (upstream primer 5'-TCCTGGTTTTCTGGCATTACCCAG-3' (SEQ ID NO:45), downstream primer 5'-CTGAGGCAATACCGTGCCGGGCGC-3' (SEQ ID NO:46)) using the following profile:

| | | | |
|---|---|---|---|
| Cycle 1 | 1x | 50,0°C | for 10 min |
| Cycle 2 | 1x | 94,0°C | for 5 min |
| Cycle 3 | 35x | 94,0°C | for 30 sec |
| | | 58,0°C | for 30 sec |
| | | 72,0°C | for 40 sec |

Amplified viral cDNA was applied to an agarose gel electrophoresis and visualized by ethidium-bromide. The expected band at 380bp is highlighted by an arrow in Fig 10. While control construct or NMS were unable to reduce the viral RNA, SCR13 induced the complete destruction of MHV. This experiment shows that SCR13 is effective not only by retroviruses (HIV, SIV, FV, MMTV) but also with viruses from other virus families. Considering Examples 4, 5 and 6, the coupling of SCR13 with a pathogen-specific binding molecule is considered to lead to an even higher activity.

**Example 10: Cell-Lysis of tumour cells by SCR7-Ab combinations**

SOK3-cells (1x10$^6$/ml) were incubated with an antibody specific for an extracellular domain of HER2/neu, a mAb against the tumour cell (1:100 dilution in PBS) and, after washing, exposed to normal human serum (NHS, 1:10 in RPM; I30min at 37°C) as a source of complement. The samples contained in addition SCR7 (data 12), or as control SCR2 (data 11). Samples contained a 60-fold molar excess considering that the serum concentration of fH is 500μg/ml. After washing again, a FITC-labelled antibody against C3c was added (1:500) for 30min on ice followed by a further washing step. The, samples were incubated with propidium-iodine (PI), washed and fixed with PBS supplemented with 3% formaldehyde. The deposition of C3 (FL-1-H) and the PI-staining (FL2H) were determined by FACS analysis and analysed by FACs Diva software (BD Bioscience). Considering Examples 4, 5 and 6, the coupling of SCR7 with a tumour cell-specific binding molecule is considered to lead, in accordance with this invention, to an even higher activity.

**Example 11: Destruction of a Human B lymphocyte Burkitt's lymphoma cell line by SCR-Ab combinations**

Raji cells (5×10$^5$) were incubated with the SCR7 or SCR9, respectively, at 37°C for 1h (in a 50-fold excess compared to fH (shown as 7-50 and 9-50 in Fig. 13). When indicated, cells were prior incubated with an anti-CD20 antibody (ahCD20; Dako) and cross-linked with an anti-IgG (mIgG; Dako; second and third data block of Fig. 13). Both antibodies were used in a 1:50 dilution in RPMI. After washing, NHS (1:10 in RPMI) was added and the samples were again incubated for 1h. At some of the samples NHS as omitted (middle block of Fig. 13) or substituted by heat-inactivated NHS (hi). Samples were washed again and incubated with 500μl RPMI containing 10% FCS overnight cell in a cell culture incubator (37°C 5% $CO_2$). Next, cells were washed again and dead cells were stained by propidium-iodine (PI) to gate on the viable cells in the FACS analysis. The amount of viable cells was counted by FACS (1min) and analysed by the FACS Diva software (BD Bioscience). Considering Examples 4, 5 and 6, the coupling of SCR7 or SCR9 with a tumour cell-specific binding molecule is considered to lead, in accordance with this invention, to an even higher activity.

**Example 12: Preparation of artificial SCR (aSCR)**

The codon-optimized plasmid comprising the genetic sequences encoding for an illustrative artificial short consensus repeats (aSCR*i*; SEQ ID NO: 32) was purchased from GeneArt. The aSCR*i* was cloned into pPICZalphaC (Invitrogen) via the ClaI (5-prime) and the NotI (3-prime) cloning site. The correct reading frame was confirmed by sequencing. The vectors were transfected into pichia pastoris yeast strain X-33 according to the protocol of the manufacturer (Invitrogen) and positively selected using Zeocin. Positive yeast clones were cultured at 30°C for 96h and expression was induced by adding repeatedly 0.5% Methanol (each 12h). Expressing clones were analysed by Western blotting.

The binding of the illustrative artificial SCR (aSCR*i*) to heparin was analyzed with the following modification: The bound protein was eluted by a high salt buffer (PBS containing 500mM NaCl) not by a gradient but by direct application of this buffer. The fraction was dialyzed against PBS and after concentration analyzed by Western blot. As the aSCR*i* does not represent a fH sequence, an polyclonal serum goat-anti-humanFH was substituted by a direct POX-labelled anti-HIS antibody (1:2000, Sigma) which recognizes the his-tag at the C-terminus of the aSCR*i*. The blot in **Fig. 14** shows two broad band representing concentrated monomeric (15-18kD) and dimeric aSCRs (30-36kD), the smear is the due to the glycosylation of the protein. The bound protein was released from the heparin column after elution with high salt buffer (eluate), no aSCR was found in the flow trough.

**Example 13: Complement-mediated lysis induced by artificial SCR (aSCR)**

To determine the induction of complement-mediated lysis (CoML) by artificial SCR, HIV-1 was incubated in RPMI-medium for about 10min on ice together with normal human serum (NHS) in a 1:10 dilution and the monoclonal HIV-specific Antibody 2G12 (Polymun, Vienna) in a 1:500 dilution. Samples were then incubated at 37°C for an hour. The aSCR having SEQ ID NO: 31 was applied in an equimolar amount and in a 120molar excess calculated to the content of fH in the system. As positive control 1% Igepal in RPMI-medium was added to determine 100% lysis. As negative controls one sample contained the input virus with heat inactivated serum (hiNHS in Fig. 15) and one sample consisted of input HIV-1 and NHS and 2G12 (NHS in Fig. 15). After incubation, the virus was pelleted for 60min at 15000rpm. Due to CoML, the intraviral p24 protein is released in the supernatant and can be used as read out for the induction of lysis. Using an equimolar amount of the aSCR in relation to fH, no increased CoML was observable (aSCR-1; Fig. 15) Already at a 20-fold excess, substantial CoML was induced (not shown). With increasing concentration of the aSCR, CoML of HIV was further enhanced and resulted in a 120-fold excess nearly 100% (aSCR-120; Fig. 15). Thus, the aSCR is capable of efficiently inducing CoML when coupled or linked to a pathogen-specific Ab. Considering Examples 4, 5 and 6, the coupling of artificial SCR with a pathogen-specific binding molecule is considered to lead to an even higher activity.

## Claims

**1.** A short consensus repeat-antibody construct (SCR-Ab) comprising

(a) a complement factor H-derived short consensus repeat (fH-derived SCR); and
(b) an antibody molecule that specifically recognizes or binds to a cancer cell, malignant cell or tumour cell,

wherein said fH-derived SCR comprises a polypeptide that is capable of binding heparin, wherein said complement factor H-derived short consensus repeat (fH-derived SCR) and said antibody molecule are covalently linked or comprised in a single chain multi-functional polypeptide and wherein said fH-derived SCR is selected from the group consisting of:

(i) a polypeptide encoded by the amino acid sequence comprised in SEQ ID NO: 4, SEQ ID NO: 6, SEQ ID NO: 8, SEQ ID NO: 22, SEQ ID NO: 24, SEQ ID NO: 26, SEQ ID NO: 30 or SEQ ID NO: 32;
(ii) a polypeptide encoded by the amino acid sequence that is at least 60% identical to the amino acid sequence comprised in SEQ ID NO: 4, SEQ ID NO: 6, SEQ ID NO: 8, SEQ ID NO: 22, SEQ ID NO: 24, SEQ ID NO: 26, SEQ ID NO: 30 or SEQ ID NO: 32 and wherein said polypeptide is capable of binding heparin;
(iii) a polypeptide encoded by the polynucleotide sequence as comprised in SEQ ID NO: 3, SEQ ID NO: 5, SEQ ID NO: 7, SEQ ID NO: 21, SEQ ID NO: 23, SEQ ID NO: 25, SEQ ID NO: 29 or SEQ ID NO: 31;
(iv) a polypeptide encoded by the complementary sequence of a polynucleotide that is able to hybridize with the polynucleotide as comprised in SEQ ID NO: 3, SEQ ID NO: 5, SEQ ID NO: 7, SEQ ID NO: 21, SEQ ID NO: 23, SEQ ID NO: 25, SEQ ID NO: 29 or SEQ ID NO: 31 and wherein said polypeptide is capable of binding heparin; and
(v) a polypeptide encoded by a nucleic acid molecule which is at least 60% identical to the nucleic acid sequence as comprised in SEQ ID NO: 3, SEQ ID NO: 5, SEQ ID NO: 7, SEQ ID NO: 21, SEQ ID NO: 23, SEQ ID NO: 25, SEQ ID NO: 29 or SEQ ID NO: 31 and wherein said polypeptide is capable of binding heparin.

**2.** The short consensus repeat-antibody construct (SCR-Ab) according to claim 1 wherein said fH-derived SCR is selected from the group consisting of SCR7, SCR9, SCR13 and SCR18-20 or a functional fragment of said fH-derived SCR7, SCR9, SCR13 and SCR18-20 or is an artificial SCR (aSCR).

**3.** The short consensus repeat-antibody construct (SCR-Ab) of claim 1 or 2, wherein said cancer cell, malignant cell or tumour cell is selected from the group consisting of breast cancer cells, Burkitt's lymphoma cells, multiple myeloma cells, colorectal cancer cells, metastatic colorectal cancer cells, Non-Hodgkin's Lymphoma cells, lung cancer cells, chronic lymphocytic leukaemia cells, micro-metastases and residual tumour cells.

**4.** The short consensus repeat-antibody construct (SCR-Ab) of any one of claims 1 to 3, wherein said antibody molecule is a monoclonal antibody recognizing an epitope selected from the group consisting of CD9, CD 19, CD20, CD22, CD30, CD33, CD40, CD46, CD55, CD56, CD138, erbB1, HER2/neu, IGFR, MUC-1, TAG-72, TAL-6, TRAILR and VEGFR.

**5.** The short consensus repeat-antibody construct (SCR-Ab) of any one of claims 1 to 4, wherein said fH-derived SCR comprises the polypeptide encoded by the amino acid sequence comprised in SEQ ID NO: 4 or SEQ ID NO: 22.

**6.** A polynucleotide encoding the short consensus repeat-antibody construct (SCR-Ab) of any one of claims 1 to 5.

**7.** A vector comprising the polynucleotide of claim 6.

**8.** A cell transfected with the polynucleotide of claim 6 or the vector of claim 7.

**9.** A method for the preparation of the short consensus repeat-antibody construct (SCR-Ab) of any one of claims 1 to 5 which comprises

(a) cultivating a cell of claim 8 and isolating said polypeptide from the culture; or
(b) coupling said fH-derived SCR with said antibody molecule.

**10.** The method of claim 9, wherein said fH-derived SCR is coupled with said antibody molecule by

(a) using sulfosuccinimidyl 4-[p-maleimidophenyl]butyrate (Sulfo-SMPB) chemical cross-linking; or
(b) forming a biotin-streptavidin complex.

**11.** A composition comprising the short consensus repeat-antibody construct (SCR-Ab) of any one of claims 1 to 5.

**12.** The composition of claim 11 which is

(a) a pharmaceutical composition, optionally further comprising a pharmaceutically acceptable carrier; or
(b) a diagnostic composition, optionally further comprising suitable means for detection.

**13.** The short consensus repeat-antibody construct (SCR-Ab) of any one of claims 1 to 5 or the composition of claim 11 or 12 for use in the prevention, treatment or amelioration of a cancerous disease.

**14.** The short consensus repeat-antibody construct or the composition of claim 13 for the use of claim 13, wherein said a cancerous disease is breast cancer, Burkitt's lymphoma, multiple myeloma, colorectal cancer, metastatic colorectal cancer, Non-Hodgkin's Lymphoma, lung cancer or chronic lymphocytic leukaemia or is derived from micro-metastases or residual tumours.

**15.** Kit comprising the short consensus repeat-antibody construct (SCR-Ab) of any one of claims 1 to 5, the polynucleotide of claim 6, the vector of claim 7 or the composition of claim 11 or 12.

## Patentansprüche

**1.** Kurzes Consensus-Repeat-Antikörper-Konstrukt (short consensus repeat-antibody construct; SCR-Ab), umfassend:

(a) ein vom Komplementfaktor H abgeleitetes kurzes Consensus-Repeat (fH-derived SCR); und
(b) ein Antikörper-Molekül, das eine Krebszelle, eine maligne Zelle oder Tumorzelle spezifisch erkennt oder an sie bindet,

wobei das vom Komplementfaktor H abgeleitete SCR (fH-derived SCR) ein Polypeptid umfasst, das fähig ist, Heparin zu binden, wobei das vom Komplementfaktor H abgeleitete kurze Consensus-Repeat (fH-derived SCR) und das Antikörper-Molekül kovalent verknüpft sind oder in einem multifunktionellen Einzelketten-Polypeptid umfasst sind, und wobei das vom Komplementfaktor H abgeleitete SCR (fH-derived SCR) ausgewählt ist aus der Gruppe bestehend aus:

(i) einem Polypeptid, das von der in SEQ ID NO:4, SEQ ID NO:6, SEQ ID NO:8, SEQ ID NO:22, SEQ ID NO:24, SEQ ID NO:26, SEQ ID NO:30 oder SEQ ID NO:32 umfassten Aminosäuresequenz codiert wird;
(ii) einem Polypeptid, das von der Aminosäuresequenz codiert wird, die mindestens 60% identisch ist mit der in SEQ ID NO:4, SEQ ID NO:6, SEQ ID NO:8, SEQ ID NO:22, SEQ ID NO:24, SEQ ID NO:26, SEQ ID NO:30 oder SEQ ID NO:32 umfassten Aminosäuresequenz, und wobei das Polypepetid fähig ist, Heparin zu binden;
(iii) einem Polypeptid, das von der wie in SEQ ID NO:3, SEQ ID NO:5, SEQ ID NO:7, SEQ ID NO:21, SEQ ID NO:23, SEQ ID NO:25, SEQ ID NO:29 oder SEQ ID NO:31 umfassten Polynucleotidsequenz codiert wird;
(iv) einem Polypeptid, das von der komplementären Sequenz eines Polynucleotids codiert wird, das fähig ist, mit dem wie in SEQ ID NO:3, SEQ ID NO:5, SEQ ID NO:7, SEQ ID NO:21, SEQ ID NO:23, SEQ ID NO:25, SEQ ID NO:29 oder SEQ ID NO:31 umfassten Polynucleotid zu hybridisieren, und wobei das Polypeptid fähig ist, Heparin zu binden; und
(v) einem Polypeptid, das von einem Nucleinsäuremolekül codiert wird, das mindestens 60% identisch ist mit der in SEQ ID NO:3, SEQ ID NO:5, SEQ ID NO:7, SEQ ID NO:21, SEQ ID NO:23, SEQ ID NO:25, SEQ ID NO:29 oder SEQ ID NO:31 umfassten Nucleinsäuresequenz, und wobei das Polypeptid fähig ist, Heparin zu binden.

**2.** Kurzes Consensus-Repeat-Antikörper-Konstrukt (SCR-Ab) nach Anspruch 1, wobei das vom Komplementfaktor H abgeleitete SCR (fH-derived SCR) aus der Gruppe bestehend aus SCR7, SCR9, SCR13 und SCR18-20 oder einem funktionellen Fragment des vom Komplementfaktor H abgeleiteten SCR7, SCR9, SCR13 und SCR18-20 ausgewählt ist oder ein künstliches SCR (artificial SCR; aSCR) ist.

**3.** Kurzes Consensus-Repeat-Antikörper-Konstrukt (SCR-Ab) nach Anspruch 1 oder 2, wobei die Krebszelle, maligne Zelle oder Tumorzelle ausgewählt ist aus der Gruppe bestehend aus Brustkrebszellen, Burkitt-Lymphom-Zellen, Zellen eines Multiplen Myeloms, Zellen eines kolorektalen Karzinoms, Zellen von Metastasen eines kolorektalen Karzinoms, Non-Hodgkin-Lymphomzellen, Lungenkrebszellen, Zellen chronisch lymphatischer Leukämie, Mikrometastasen und residualen Tumorzellen.

**4.** Kurzes Consensus-Repeat-Antikörper-Konstrukt (SCR-Ab) nach einem der Ansprüche 1 bis 3, wobei das Antikörper-Molekül ein monoclonaler Antikörper ist, der ein Epitop erkennt, das ausgewählt ist aus der Gruppe bestehend aus CD9, CD19, CD20, CD22, CD30, CD33, CD40, CD46, CD55, CD56, CD138, erbB1, HER2/neu, IGFR, MUC-1, TAG-72, TAL-6, TRAILR und VEGFR.

**5.** Kurzes Consensus-Repeat-Antikörper-Konstrukt (SCR-Ab) nach einem der Ansprüche 1 bis 4, wobei das vom Komplementfaktor H abgeleitete SCR (fH-derived SCR) das Polypeptid umfasst, das von der in SEQ ID NO:4 oder SEQ ID NO:22 umfassten Aminosäuresequenz codiert wird.

**6.** Polynucleotid, das das kurze Consensus-Repeat-Antikörper-Konstrukt (SCR-Ab) nach einem der Ansprüche 1 bis 5 codiert.

**7.** Vektor, der das Polynucleotid nach Anspruch 6 umfasst.

**8.** Zelle, die mit dem Polynucleotid nach Anspruch 6 oder dem Vektor nach Anspruch 7 transfiziert ist.

**9.** Verfahren zur Herstellung des kurzen Consensus-Repeat-Antikörper-Konstrukts (SCR-Ab) nach einem der Ansprüche 1 bis 5, das umfasst:

(a) Züchten einer Zelle nach Anspruch 8 und Isolieren des Polypeptids aus der Kultur; oder
(b) Koppeln des vom Komplementfaktor H abgeleiteten SCR (fH-derived SCR) mit dem Antikörper-Molekül.

**10.** Verfahren nach Anspruch 9, wobei das vom Komplementfaktor H abgeleitete SCR (fH-derived SCR) mit dem Antikörper-Molekül gekoppelt wird durch:

(a) Anwenden chemischer Vernetzung mittels Sulfosuccinimidyl-4-[p-maleimidophenyl]butyrat (Sulfo-SMPB); oder
(b) Bilden eines Biotin-Streptavidin-Komplexes.

**11.** Zusammensetzung, die das kurze Consensus-Repeat-Antikörper-Konstrukt (SCR-Ab) nach einem der Ansprüche 1 bis 5 umfasst.

**12.** Zusammensetzung nach Anspruch 11, die

(a) ein Arzneimittel, das gegebenenfalls des Weiteren einen pharmazeutisch verträglichen Träger umfasst; oder
(b) eine diagnostische Zusammensetzung, die gegebenenfalls des Weiteren geeignete Nachweismittel umfasst,

ist.

**13.** Kurzes Consensus-Repeat-Antikörper-Konstrukt (SCR-Ab) nach einem der Ansprüche 1 bis 5 oder Zusammensetzung nach Anspruch 11 oder 12 zur Verwendung bei der Vorbeugung, Behandlung oder Besserung einer Krebserkrankung.

**14.** Kurzes Consensus-Repeat-Antikörper-Konstrukt oder Zusammensetzung nach Anspruch 13 zur Verwendung nach Anspruch 13, wobei die Krebserkrankung Brustkrebs, Burkitt-Lymphom, Multiples Myelom, kolorektales Karzinom, metastasierendes kolorektales Karzinom, Non-Hodgkin-Lymphom, Lungenkrebs oder chronisch lymphatische Leukämie ist oder von Mikrometastasen oder residualen Tumoren herrührt.

**15.** Kit, umfassend das kurze Consensus-Repeat-Antikörper-Konstrukt (SCR-Ab) nach einem der Ansprüche 1 bis 5, das Polynucleotid nach Anspruch 6, den Vektor nach Anspruch 7 oder die Zusammensetzung nach Anspruch 11 oder 12.

## Revendications

**1.** Construction courte répétition consensus-anticorps (SCR-Ab) comprenant

(a) une courte répétition consensus dérivée du facteur H du complément (SCR dérivée de fH); et
(b) une molécule d'anticorps qui reconnaît ou se lie spécifiquement à une cellule cancéreuse, une cellule maligne ou une cellule tumorale,

où ladite SCR dérivée de fH comprend un polypeptide qui est capable de lier l'héparine, où ladite courte répétition consensus dérivée du facteur H du complément (SCR dérivée de fH) et ladite molécule d'anticorps sont liées de manière covalente ou comprises dans un polypeptide multifonctionnel à une seule chaîne et où ladite SCR dérivée de fH est choisie dans le groupe consistant en:

(i) un polypeptide codé par la séquence d'acides aminés comprise dans SEQ ID NO: 4, SEQ ID NO: 6, SEQ ID NO: 8, SEQ ID NO: 22, SEQ ID NO: 24, SEQ ID NO: 26, SEQ ID NO: 30 ou SEQ ID NO: 32;
(ii) un polypeptide codé par la séquence d'acides aminés qui est identique à raison d'au moins 60 % à la séquence d'acides aminés comprise dans SEQ ID NO: 4, SEQ ID NO: 6, SEQ ID NO: 8, SEQ ID NO: 22, SEQ ID NO: 24, SEQ ID NO: 26, SEQ ID NO: 30 ou SEQ ID NO: 32 et où ledit polypeptide est capable de lier l'héparine;
(iii) un polypeptide codé par la séquence polynucléotidique comprise dans SEQ ID NO: 3, SEQ ID NO: 5, SEQ ID NO: 7, SEQ ID NO: 21, SEQ ID NO: 23, SEQ ID NO: 25, SEQ ID NO: 29 ou SEQ ID NO: 31;
(iv) un polypeptide codé par la séquence complémentaire d'un polynucléotide qui est capable de s'hybrider avec le polynucléotide compris dans SEQ ID NO: 3, SEQ ID NO: 5, SEQ ID NO: 7, SEQ ID NO: 21, SEQ ID NO: 23, SEQ ID NO: 25, SEQ ID NO: 29 ou SEQ ID NO: 31 et où ledit polypeptide est capable de lier l'héparine; et
(v) un polypeptide codé par une molécule d'acide nucléique qui est identique à raison d'au moins 60 % à la séquence d'acide nucléique comprise dans SEQ ID NO: 3, SEQ ID NO: 5, SEQ ID NO: 7, SEQ ID NO: 21, SEQ ID NO: 23, SEQ ID NO: 25, SEQ ID NO: 29 ou SEQ ID NO: 31 et où ledit polypeptide est capable de lier l'héparine.

**2.** Construction courte répétition consensus-anticorps (SCR-Ab) selon la revendication 1 où ladite SCR dérivée de fH est choisie dans le groupe consistant en SCR7, SCR9, SCR13 et SCR18-20 ou un fragment fonctionnel de ladite SCR7, SCR9, SCR13 et SCR18-20 dérivée de fH ou est une SCR artificielle (aSCR).

**3.** Construction courte répétition consensus-anticorps (SCR-Ab) selon la revendication 1 ou 2, où ladite cellule cancéreuse, cellule maligne ou cellule tumorale est choisie dans le groupe consistant en les cellules de cancer du sein, les cellules de lymphome de Burkitt, les cellules de myélome multiple, les cellules de cancer colorectal, les cellules de cancer colorectal métastatique, les cellules de lymphome non hodgkinien, les cellules de cancer du poumon, les cellules de leucémie lymphoïde chronique, les micrométastases et les cellules de tumeurs résiduelles.

**4.** Construction courte répétition consensus-anticorps (SCR-Ab) selon l'une quelconque des revendications 1 à 3, où ladite molécule d'anticorps est un anticorps monoclonal reconnaissant un épitope choisi dans le groupe consistant en CD9, CD19, CD20, CD22, CD30, CD33, CD40, CD46, CD55, CD56, CD138, erbB1, HER2/neu, IGFR, MUC-1, TAG-72, TAL-6, TRAILR et VEGFR.

**5.** Construction courte répétition consensus-anticorps (SCR-Ab) selon l'une quelconque des revendications 1 à 4, où ladite SCR dérivée de fH comprend le polypeptide codé par la séquence d'acides aminés comprise dans SEQ ID NO: 4 ou SEQ ID NO: 22.

**6.** Polynucléotide codant la construction courte répétition consensus-anticorps (SCR-Ab) selon l'une quelconque des revendications 1 à 5.

**7.** Vecteur comprenant le polynucléotide selon la revendication 6.

**8.** Cellule transfectée avec le polynucléotide selon la revendication 6 ou le vecteur selon la revendication 7.

**9.** Procédé pour la préparation de la construction courte répétition consensus-anticorps (SCR-Ab) selon l'une quelconque des revendications 1 à 5 qui comprend

(a) la culture d'une cellule selon la revendication 8 et l'isolement dudit polypeptide à partir de la culture; ou
(b) le couplage de ladite SCR dérivée de fH avec ladite molécule d'anticorps.

**10.** Procédé selon la revendication 9, où ladite SCR dérivée de fH est couplée avec ladite molécule d'anticorps par

(a) l'utilisation du pontage chimique au 4-[p-maléimidophényl]butyrate de sulfosuccinimidyle (Sulfo-SMPB) ; ou
(b) la formation d'un complexe biotine-streptavidine.

**11.** Composition comprenant la construction courte répétition consensus-anticorps (SCR-Ab) selon l'une quelconque des revendications 1 à 5.

**12.** Composition selon la revendication 11 qui est

(a) une composition pharmaceutique, comprenant éventuellement en outre un vecteur pharmaceutiquement acceptable ; ou
(b) une composition diagnostique, comprenant éventuellement en outre des moyens de détection appropriés.

**13.** Construction courte répétition consensus-anticorps (SCR-Ab) selon l'une quelconque des revendications 1 à 5 ou composition selon la revendication 11 ou 12 pour une utilisation dans la prévention, le traitement ou l'amélioration d'une maladie cancéreuse.

**14.** Construction courte répétition consensus-anticorps ou composition selon la revendication 13 pour l'utilisation selon la revendication 13, où ladite une maladie cancéreuse est le cancer du sein, le lymphome de Burkitt, le myélome multiple, le cancer colorectal, le cancer colorectal métastatique, le lymphome non hodgkinien, le cancer du poumon ou la leucémie lymphoïde chronique, ou est dérivée de micrométastases ou de tumeurs résiduelles.

**15.** Kit comprenant la construction courte répétition consensus-anticorps (SCR-Ab) selon l'une quelconque des revendications 1 à 5, le polynucléotide selon la revendication 6, le vecteur selon la revendication 7 ou la composition selon la revendication 11 ou 12.

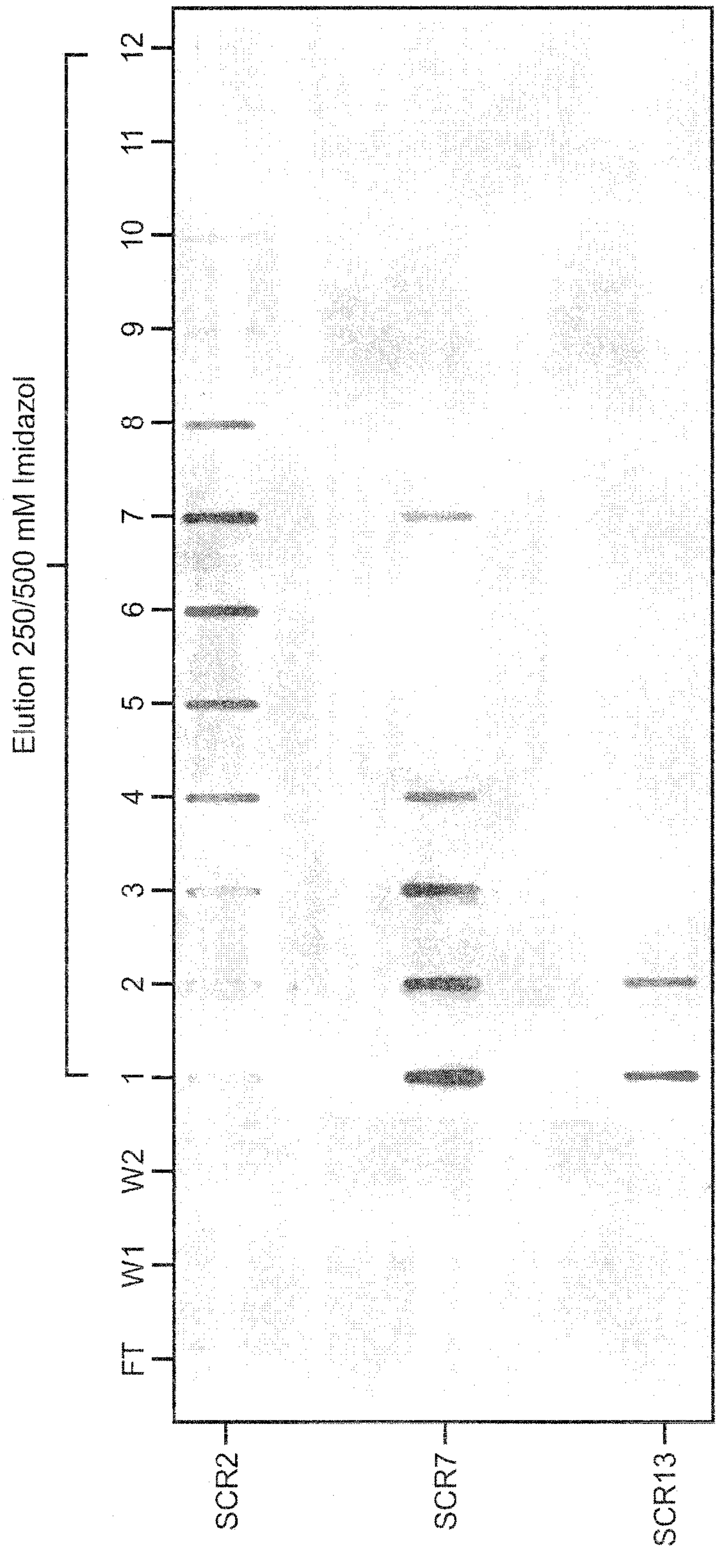
Elution 250/500 mM Imidazol
12
11
10
9
8
7
6
5
4
3
2
1
W2
W1
FT
SCR2
SCR7
SCR13

Figure 1

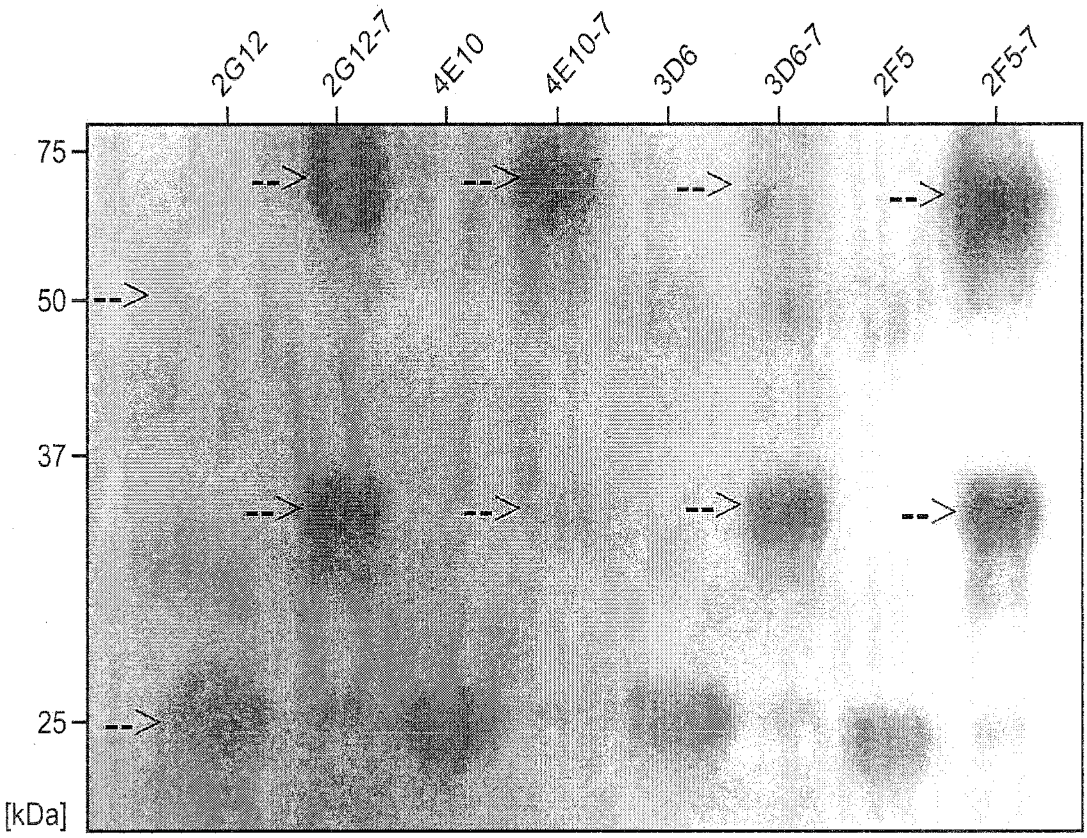


Figure 2

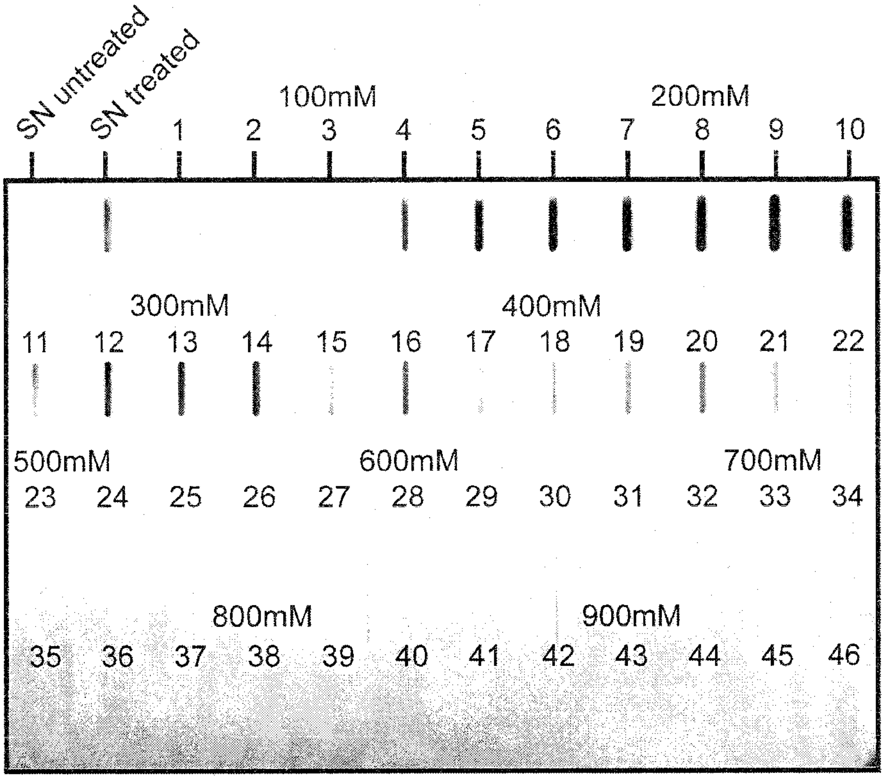


Figure 3

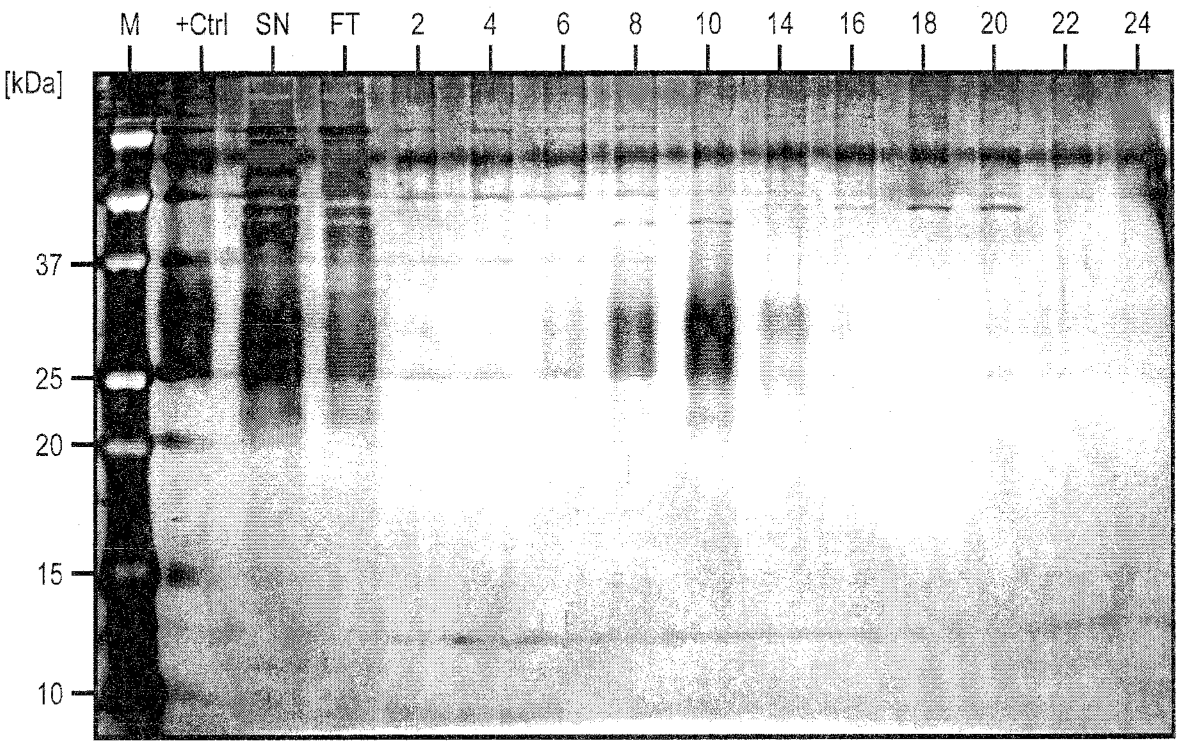


Figure 4

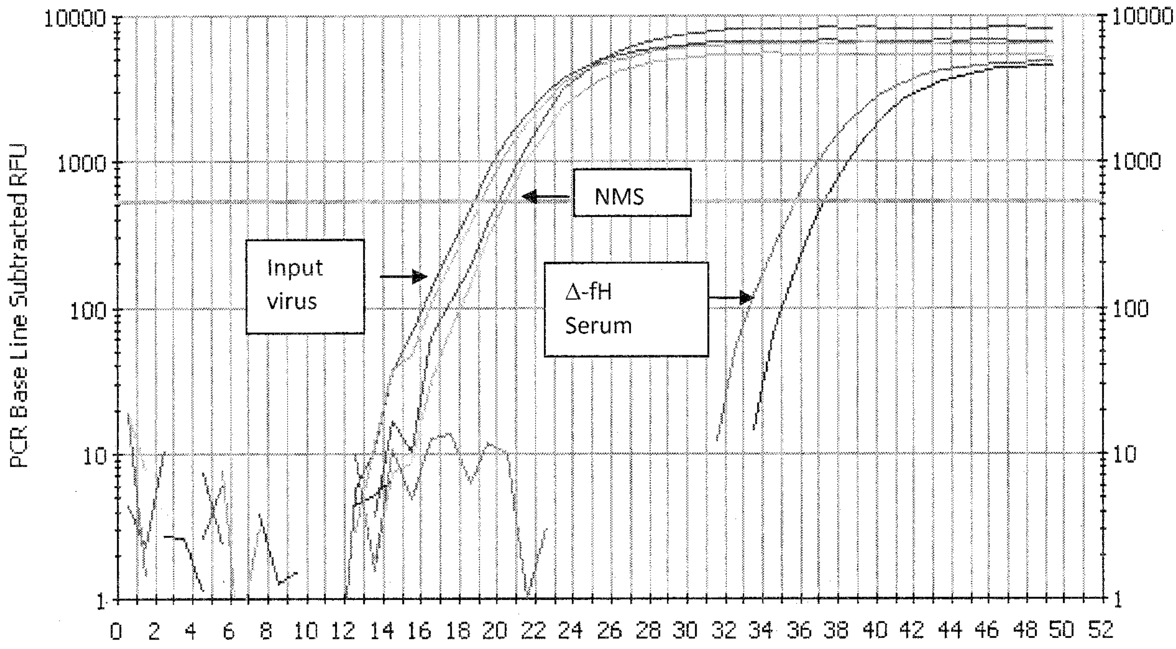


Figure 5

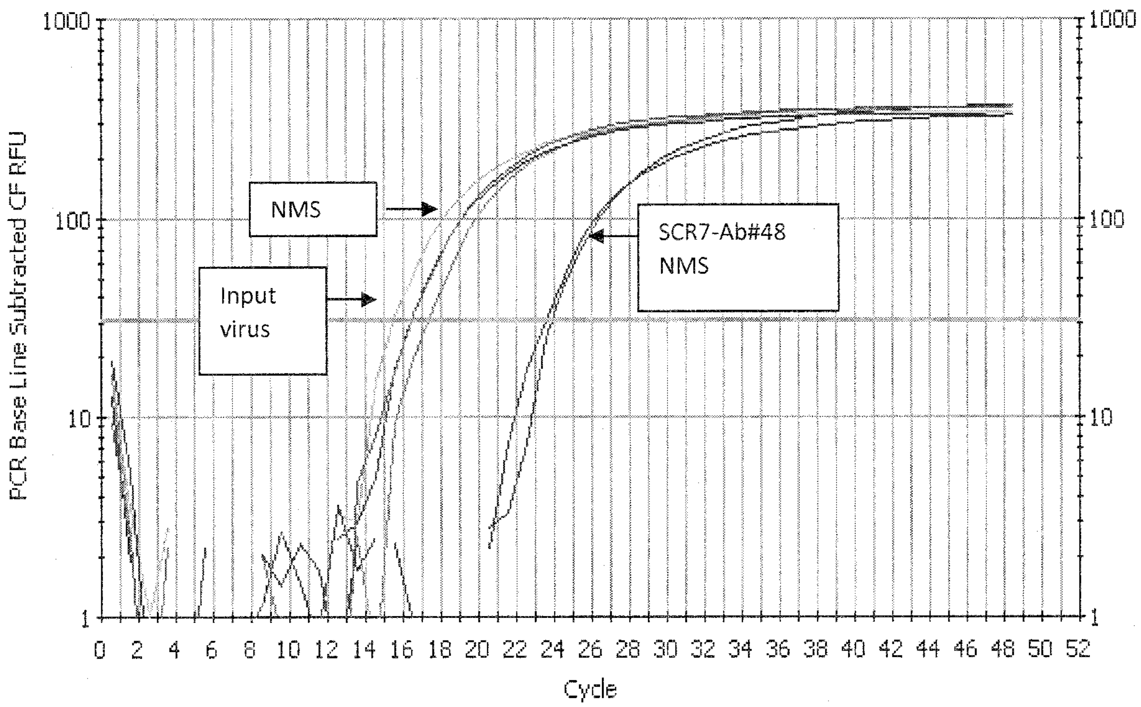


Figure 6

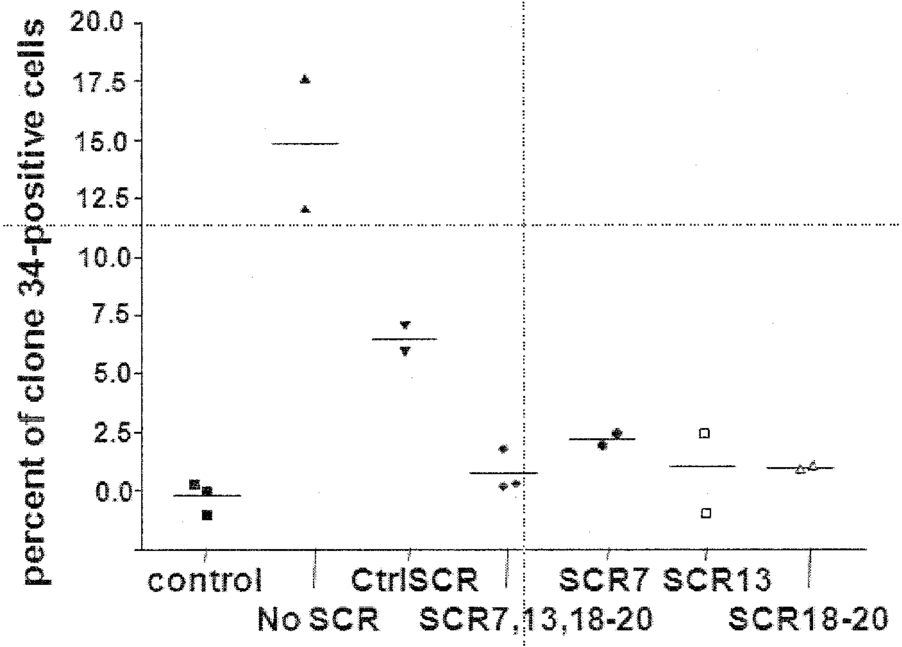


Figure 7

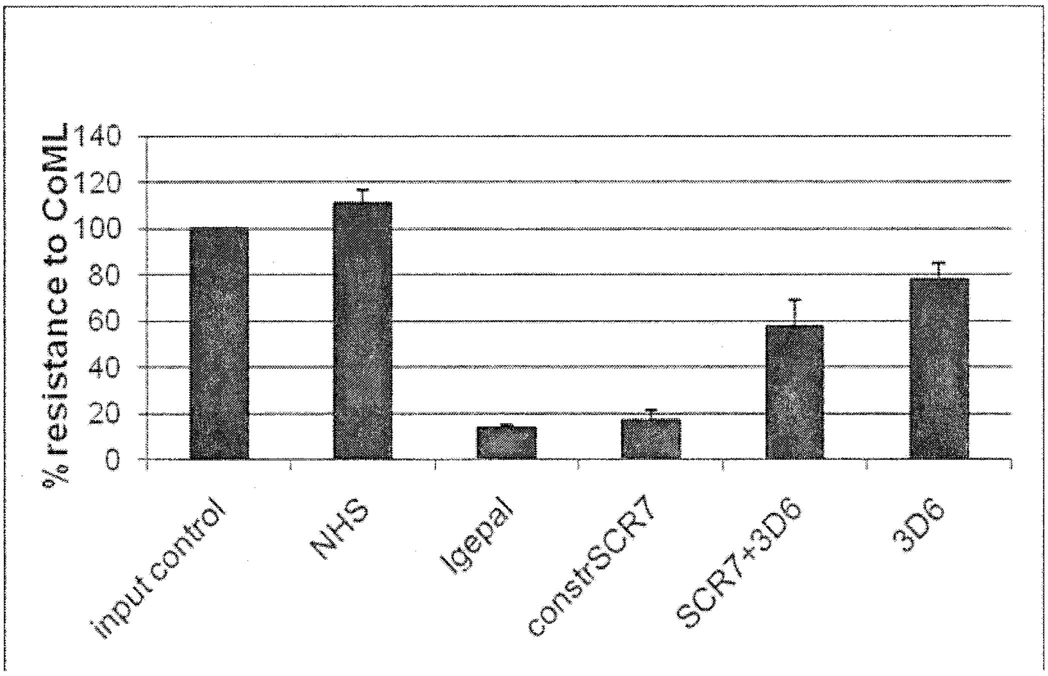


Figure 8

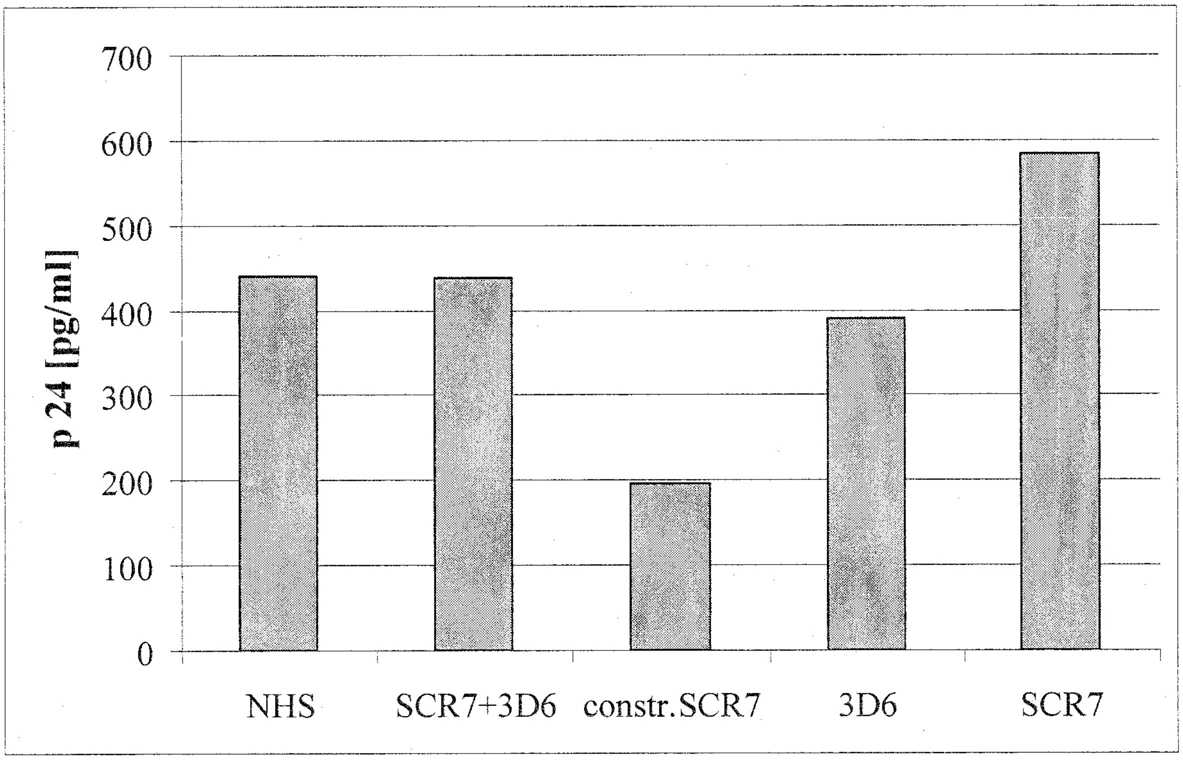


Figure 9

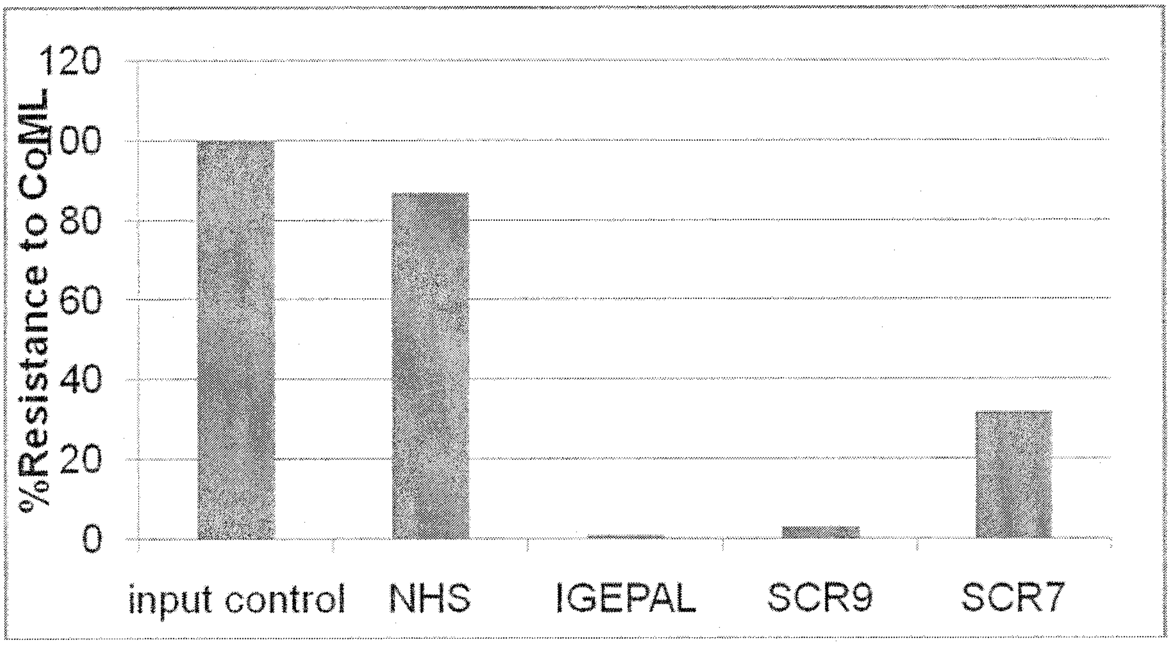


Figure 10

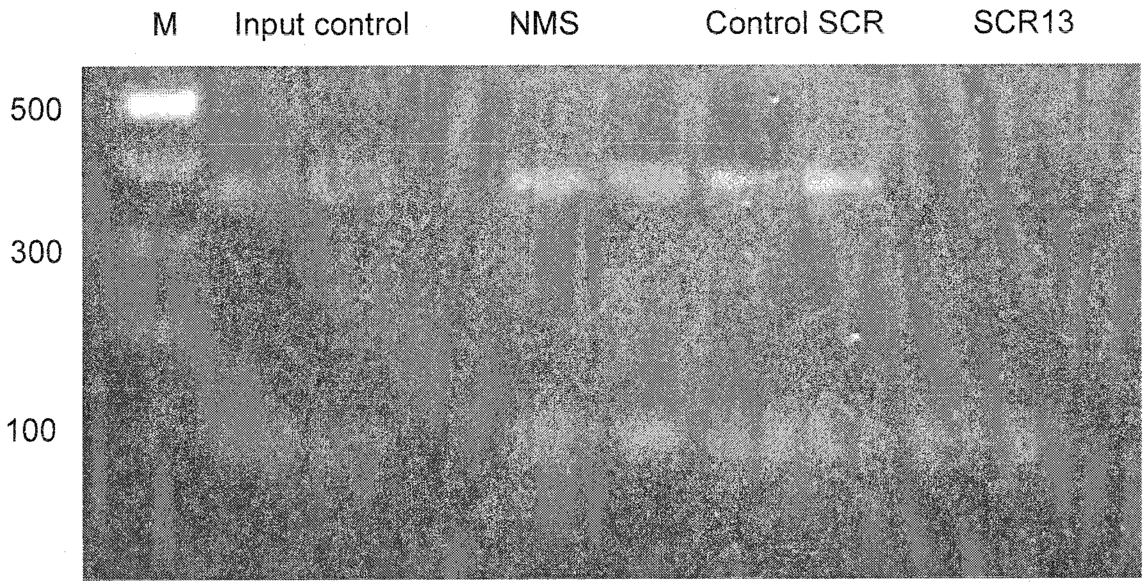


Figure 11

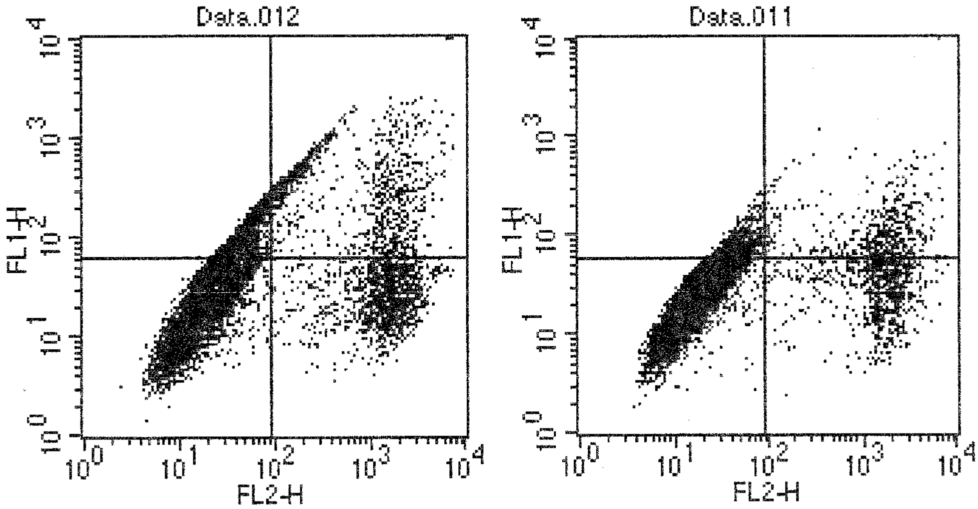


Figure 12

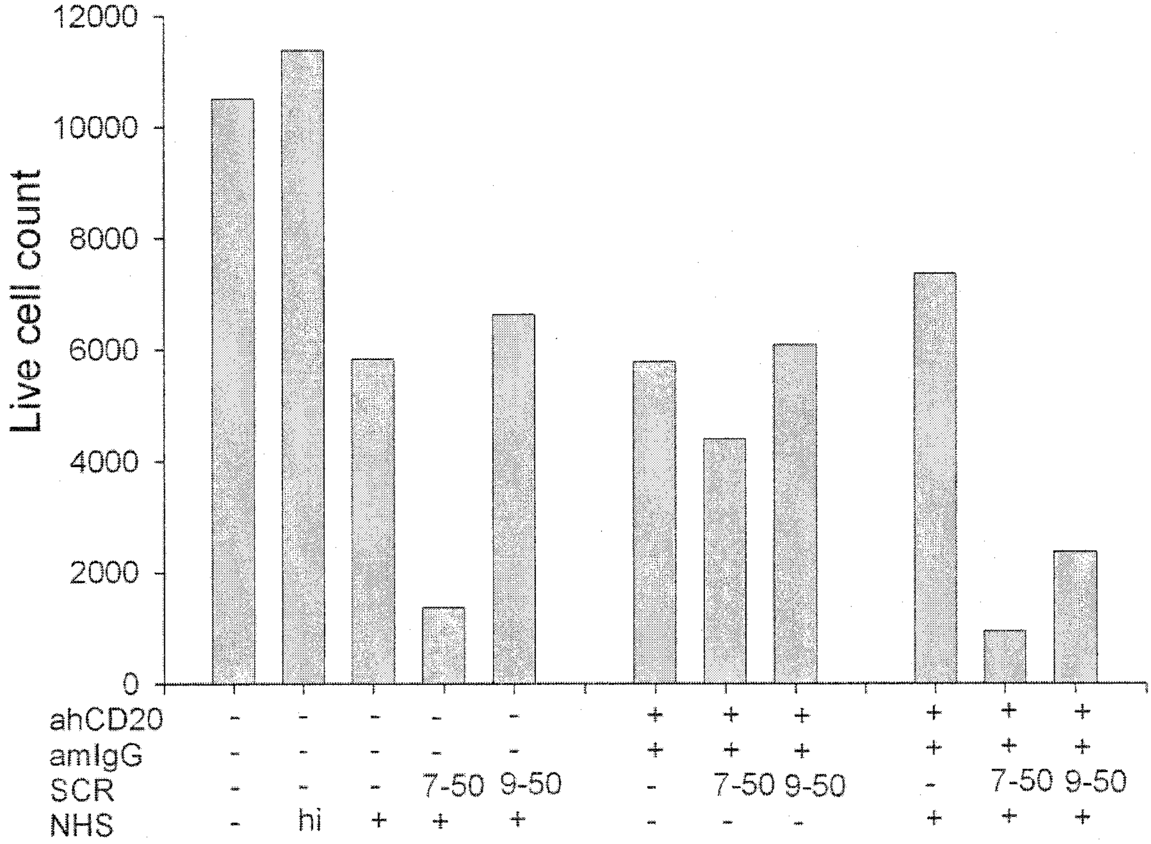

Live cell count
12000
10000
8000
6000
4000
2000
0
ahCD20 - - - - - + + + + + +
amIgG - - - - - + + + + + +
SCR - - - 7-50 9-50 - 7-50 9-50 - 7-50 9-50
NHS - hi + + + - - - + + +


Figure 13

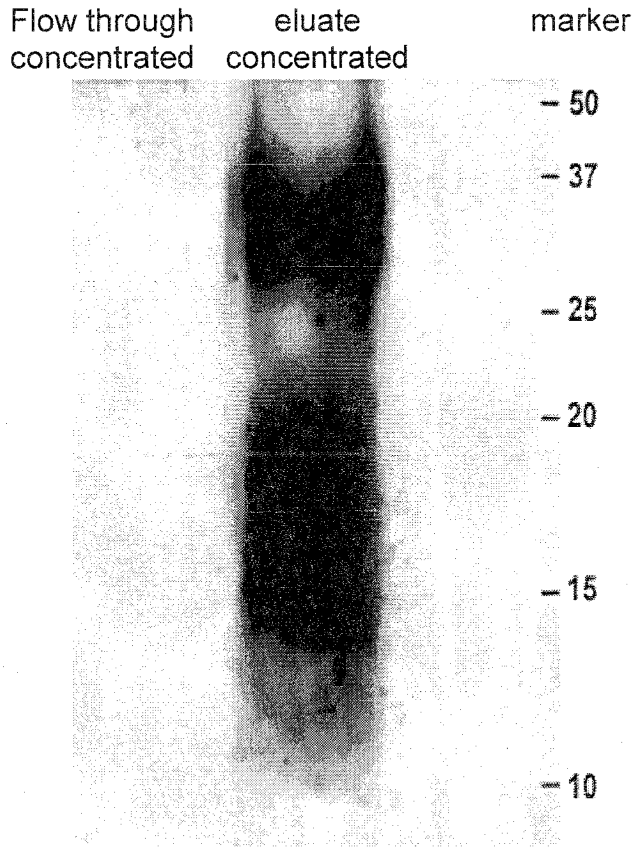


Figure 14

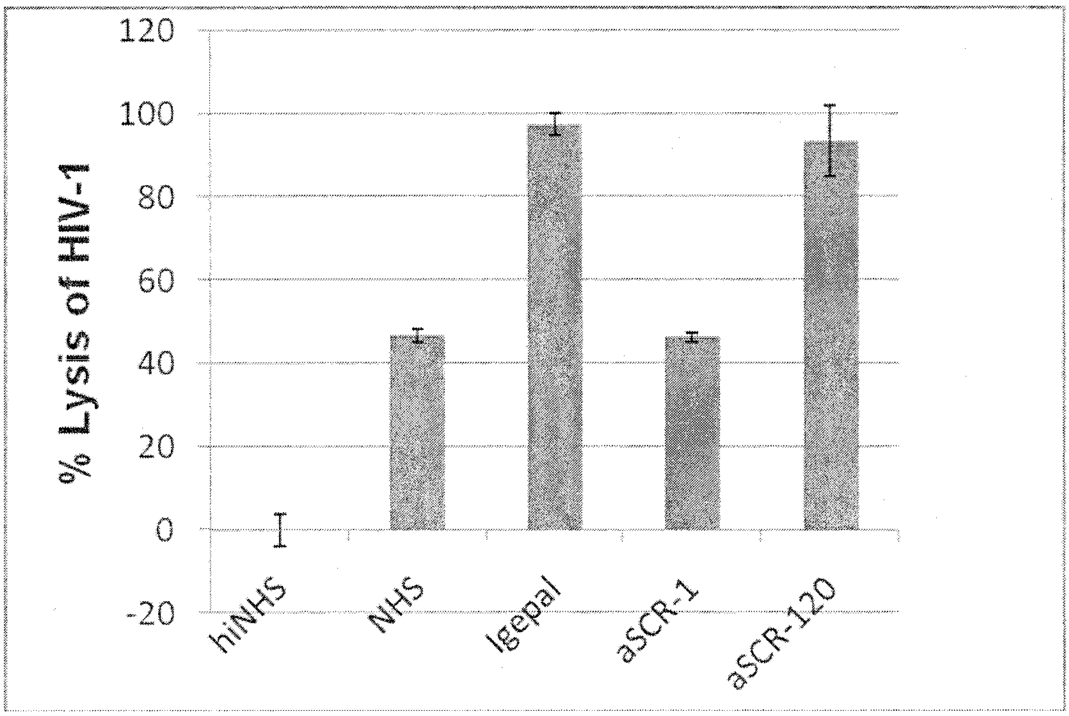


Figure 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 200268059 A1
- US 2006246066 A1
- EP 0854150 A1
- WO 0024782 A
- US 20040071710 A1
- WO 03022879 A2
- EP 0355140 B1
- US 20030157112 A1
- US 4271145 A
- WO 0220565 A
- WO 9429469 A
- WO 9700957 A
- US 5580859 A
- US 5589466 A
- US 4394448 A
- US 2007086985 A
- US 2007082388 A
- US 2007071770 A
- US 2007003522 A
- US 2007048285 A
- EP 1757703 A
- WO 2004111248 A
- WO 2007022030 A
- WO 2007018562 A
- EP 07008954 A

### Non-patent literature cited in the description


- **TAKEUCHI.** *J Virol.,* 1994, vol. 68, 8001-7
- **COOPER.** *J Exp Med.,* 1976, vol. 144, 970-84
- **BARTHOLOMEW.** *J Immunol.,* 1978, vol. 12.1, 1748-51
- **BARTHOLOMEW.** *J Exp Med.,* 1978, vol. 147, 844-53
- **SHERWIN.** *Int J Cancer.,* 1978, vol. 21 (1), 6-11
- **JENSEN.** *Hamatol Bluttransfus,* 1979, vol. 23, 501-3
- **KOBILINSKY.** *Infect Immun.,* 1980, vol. 29 (1), 165-70
- **DIERICH.** Immunology of HIV infection. Plenum Press, 1996, 365-376
- **EBENBICHLER.** *J Exp Med.,* 1991, vol. 174, 1417-24
- **STOIBER.** *Immunol Reviews,* 2001, vol. 180, 168-76
- **SAIFUDDIN.** *AIDS Res Hum Retroviruses,* 1995, vol. 11, 115-22
- **STOIBER.** *Annu Rev Immunol.,* 1997, vol. 15, 649-674
- **SULLIVAN.** *J Immunol,* 1996, vol. 157, 1791-1798
- **DIERICH.** *Nature Med,* 1996, vol. 2, 153-155
- **FRANK.** *AIDS,* 1996, vol. 10, 1611-20
- **MONTEFIORI.** *Virology,* 1994, vol. 205, 82-92
- **SAIFUDDIN.** *J Exp Med.,* 1995, vol. 182, 501-509
- **SCHMITZ.** *J Clin Invest.,* 1995, vol. 96, 1520-6
- **MARSCHANG.** *Eur J Immunol.,* 1995, vol. 25, 285-290
- **TAKEFMAN.** *Virology,* 1998, vol. 46, 370-378
- **SPEAR.** *Immunology,* 1991, vol. 73, 377-82
- **STOIBER.** *J Exp Med.,* 1996, vol. 183, 307-310
- *SCR,* vol. 7 (9), 18-20
- **CHENG.** *Mol Immunol.,* 2006, vol. 43, 972-9
- **REITER.** *Journal of Immunology,* 1989, vol. 142, 2771-2777
- **REITER.** *Targeted Diagnosis and Therapy,* 1989, vol. 2, 119-135
- **YEFENOF.** *Journal of Immunology,* 1990, vol. 144, 1538-1543
- **PINTÉR.** *Expert Opinion of Investigational Drugs,* 2000, vol. 9, 199-205
- **REICHERT.** *Nat Rev Drug Discov,* 2007, vol. 6, 349-356
- **HOLZ.** *Recent Results Cancer Res,* 1998, vol. 146, 214-218
- **DURRANT.** *Curr Opin Investig Drugs,* 2001, vol. 2, 959-966
- **BJØRGE.** *Br J Cancer,* 2005, vol. 9, 895-905
- **PRODINGER.** Complement. In: Fundamental Immunology. Lippincott-Raven Publishers, 2004
- **AJONA.** *J Immunol,* 2007, vol. 178, 5991-5997
- **FEDARKO.** *J Biol Chem,* 2000, vol. 275 (22), 16666-16672
- **KINDERS.** *Clin Cancer Res,* 1998, vol. 4, 2511-2520
- **SMITH.** *J Virol,* 2000, vol. 74, 5659-5666
- **GHEBREMARIAM.** *Ann N Y Acad Sci,* 2005, vol. 1056, 113-122

- **SAMBROOK ; RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridization, a practical approach. IRL Press, 1985
- **THOMPSON.** *Nucl. Acids Res.,* 1994, vol. 2, 4673-4680
- **BRUTLAG.** *Comp. App. Biosci.,* 1990, vol. 6, 237-245
- **ALTSCHUL.** *Nucl. Acids Res.,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410
- **HENIKOFF.** *Proc. Natl. Acad. Sci.,* 1989, vol. 89, 10915
- **SCHLESINGER.** *Virology,* 1983, vol. 125, 8-17
- **CHESEBRO et al.** *Virology,* 1981, vol. 112 (1), 131-44
- **GIRAUDON ; WILD.** *J. Gen Virol,* 1981, vol. 54, 325
- **HOFFMAN.** *PNAS,* 1980, vol. 77, 2979-2983
- **HARLOW ; LANE.** Antibodies: a laboratory manual. Cold Spring Harbor Laboratory Press, 1988
- **GOLD.** *Ann. Rev. Biochem,* 1995, vol. 64, 763-797
- **KOHL.** *PNAS,* 2003, vol. 100, 1700-1705
- **FORRER.** *FEBS letters,* 2003, vol. 539, 2-6
- **HANES.** *PNAS,* 1997, vol. 94, 4937-4942
- **SAMBROOK.** Molecular Cloning: A Laboratory Manual. Cold Spring Harbor Laboratory, 1989
- **SCOPES.** Protein Purification. Springer-Verlag, 1982
- **MACK.** *PNAS,* 1995, vol. 92, 7021-7025
- **GIORDANO.** *Nature Medicine,* 1996, vol. 2, 534-539
- **SCHAPER.** *Circ. Res.,* 1996, vol. 79, 911-919
- **ANDERSON.** *Science,* 1992, vol. 256, 808-813
- **ISNER.** *Lancet,* 1996, vol. 348, 370-374
- **MUHLHAUSER.** *Circ. Res.,* 1995, vol. 77, 1077-1086
- **ONODUA.** *Blood,* 1998, vol. 91, 30-36
- **VERZELETTI.** *Hum. Gene Ther.,* 1998, vol. 9, 2243-2251
- **VERMA.** *Nature,* 1997, vol. 389, 239-242
- **ANDERSON.** *Nature,* 1998, vol. 392, 25-30
- **WANG.** *Gene Therapy,* 1997, vol. 4, 393-400
- **WANG.** *Nature Medicine,* 1996, vol. 2, 714-716
- **LI.** *Ann NY Acad Sci,* 2006, vol. 1082, 172-9
- Remington's Pharmaceutical Sciences. 1980
- **CONNOR.** *J. Exp. Med.,* 1997, vol. 185, 621-628
- **DITTMER.** *J Virol,* 1998, vol. 72, 6554-8

SEQUENCE LISTING

<110> Medizinische Universität Innsbruck

<120> Complement factor H-derived short consensus repeat-antibody constructs

<130> M3077 EP/1 S3

<150> EP 07 00 8954.5
<151> 2007-05-03

<160> 46

<170> PatentIn version 3.3

<210> 1
<211> 212
<212> DNA
<213> artificial sequence

<220>
<221> source
<223> /note="Description of artificial sequence: mouse SCR2"

<400> 1

```
gaattctcga ccaaaaaacc gtgtggtcat ccgggtgata ccccgtttgg tagctttcgt      60
ctggcggttg gtagccagtt tgaatttggc gcgaaagtgg tgtatacctg cgatgatggc     120
tatcagctgc tgggcgaaat tgattatcgt gaatgcggtg cggatggctg gattaacgat     180
attccgctgt gcgaaatcct cgagggtcta ga                                   212
```

<210> 2
<211> 66
<212> PRT
<213> artificial sequence

<220>
<221> source
<223> /note="Description of artificial sequence: mouse SCR2"

<400> 2

```
Ser Thr Lys Lys Pro Cys Gly His Pro Gly Asp Thr Pro Phe Gly Ser
1               5                   10                  15

Phe Arg Leu Ala Val Gly Ser Gln Phe Glu Phe Gly Ala Lys Val Val
            20                  25                  30

Tyr Thr Cys Asp Asp Gly Tyr Gln Leu Leu Gly Glu Ile Asp Tyr Arg
        35                  40                  45

Glu Cys Gly Ala Asp Gly Trp Ile Asn Asp Ile Pro Leu Cys Glu Ile

              50                   55                   60

          Leu Glu
          65
```

<210> 3
<211> 203
<212> DNA
<213> artificial sequence

<220>
<221> source
<223> /note="Description of artificial sequence: mouse SCR7"

<400> 3

```
gaattctcga ccaaagtgcg caaatgtgtg ttccactacg tggaaaacgg tgatagcgcg      60

tactgggaaa aagtgtatgt tcagggccag agcctgaaag tgcagtgcta taacggctat     120

agcctgcaga atggccagga taccatgacc tgcaccgaaa atggttggag cccgccgccg     180

aaatgtatta ttctcgatct aga                                              203
```

<210> 4
<211> 63
<212> PRT
<213> artificial sequence

<220>
<221> source

<223> /note="Description of artificial sequence: mouse SCR7"

<400> 4

```
Ser Thr Lys Val Arg Lys Cys Val Phe His Tyr Val Glu Asn Gly Asp
1               5                   10                  15

Ser Ala Tyr Trp Glu Lys Val Tyr Val Gln Gly Gln Ser Leu Lys Val
            20                  25                  30

Gln Cys Tyr Asn Gly Tyr Ser Leu Gln Asn Gly Gln Asp Thr Met Thr
        35                  40                  45

Cys Thr Glu Asn Gly Trp Ser Pro Pro Pro Lys Cys Ile Ile Leu
    50                  55                  60
```

<210> 5
<211> 206
<212> DNA
<213> artificial sequence

<220>
<221> source
<223> /note="Description of artificial sequence: mouse SCR13"

<400> 5

```
gaattctcga ccaaagcgac cgatcagctg gaaaaatgcc gcgttctgaa aagcaccggc     60

atcgaagcga ttaaaccgaa actgaccgaa tttacccaca acagcaccat ggattacaaa    120

tgccgcgata aacaggaata tgaacgcagc atttgcatca acggcaaatg ggatccggaa    180

ccgaattgca ccattctcga tctaga                                         206
```

<210> 6
<211> 64
<212> PRT
<213> artificial sequence

<220>
<221> source
<223> /note="Description of artificial sequence: mouse SCR13"

<400> 6

```
Ser Thr Lys Ala Thr Asp Gln Leu Glu Lys Cys Arg Val Leu Lys Ser
1               5                   10                  15

Thr Gly Ile Glu Ala Ile Lys Pro Lys Leu Thr Glu Phe Thr His Asn
            20                  25                  30

Ser Thr Met Asp Tyr Lys Cys Arg Asp Lys Gln Glu Tyr Glu Arg Ser
        35                  40                  45

Ile Cys Ile Asn Gly Lys Trp Asp Pro Glu Pro Asn Cys Thr Ile Leu
    50                  55                  60
```

<210> 7
<211> 573
<212> DNA
<213> artificial sequence

<220>
<221> source
<223> /note="Description of artificial sequence: mouse SCR18-20"

<400> 7

```
atcgatgaaa gataacagct gcgttgatcc gccgcatgtt ccgaatgcga ccattgtgac     60
ccgcaccaaa aacaaatatc tgcacggcga tcgtgtgcgt tatgaatgca acaaaccgct    120
ggaactgttt ggtcaggttg aagtgatgtg cgaaaacggc atctggaccg aaaaaccgaa    180
atgccgtgat agcaccggta aatgtggtcc gccgccgccg attgataatg gcgatatcac    240
cagcctgagc ctgccggttt atgaaccgct gagcagcgtg gaatatcagt gccagaaata    300
ttatctgctg aaaggcaaaa aaaccatcac ctgcaccaac ggtaaatgga gcgaaccgcc    360
gacctgtctg catgcgtgtg tgattccgga aaacatcatg gaaagccaca acatcattct    420
gaaatggcgc cacaccgaaa aaatctatag ccacagcggc gaagatattg aattcggctg    480
taaatatggc tattacaaag cgcgtgatag cccgccgttt cgtaccaaat gcatcaacgg    540
caccattaac tatccgacct gcgtgcgtct aga                                 573
```

<210> 8
<211> 186
<212> PRT
<213> artificial sequence

<220>
<221> source
<223> /note="Description of artificial sequence: mouse SCR18-20"

<400> 8

```
Lys Asp Asn Ser Cys Val Asp Pro Pro His Val Pro Asn Ala Thr Ile
1               5                   10                  15

Val Thr Arg Thr Lys Asn Lys Tyr Leu His Gly Asp Arg Val Arg Tyr
            20                  25                  30

Glu Cys Asn Lys Pro Leu Glu Leu Phe Gly Gln Val Glu Val Met Cys
        35                  40                  45

Glu Asn Gly Ile Trp Thr Glu Lys Pro Lys Cys Arg Asp Ser Thr Gly
    50                  55                  60

Lys Cys Gly Pro Pro Pro Pro Ile Asp Asn Gly Asp Ile Thr Ser Leu
65                  70                  75                  80

Ser Leu Pro Val Tyr Glu Pro Leu Ser Ser Val Glu Tyr Gln Cys Gln
                85                  90                  95

Lys Tyr Tyr Leu Leu Lys Gly Lys Lys Thr Ile Thr Cys Thr Asn Gly
            100                 105                 110

Lys Trp Ser Glu Pro Pro Thr Cys Leu His Ala Cys Val Ile Pro Glu
        115                 120                 125

Asn Ile Met Glu Ser His Asn Ile Ile Leu Lys Trp Arg His Thr Glu
    130                 135                 140

Lys Ile Tyr Ser His Ser Gly Glu Asp Ile Glu Phe Gly Cys Lys Tyr
145                 150                 155                 160

Gly Tyr Tyr Lys Ala Arg Asp Ser Pro Pro Phe Arg Thr Lys Cys Ile
                165                 170                 175

Asn Gly Thr Ile Asn Tyr Pro Thr Cys Val
            180                 185
```

<210> 9
<211> 24
<212> DNA
<213> artificial sequence

<220>
<221> source
<223> /note="Description of artificial sequence: 5' mouse SCR2 primer"

<400> 9
aaaaagaatt ctcgaccaaa aaac 24

<210> 10

<211> 21
<212> DNA
<213> artificial sequence

<220>
<221> source
<223> /note="Description of artificial sequence: 3' mouse SCR2 primer"

<400> 10
aaaaagtcta gaccctcgag g 21

<210> 11
<211> 23
<212> DNA
<213> artificial sequence

<220>
<221> source
<223> /note="Description of artificial sequence: 5' mouse SCR7 primer"

<400> 11
aaaaagaatt ctcgaccaaa gtg 23

<210> 12
<211> 24
<212> DNA
<213> artificial sequence

<220>
<221> source
<223> /note="Description of artificial sequence: 3' mouse SCR7 primer"

<400> 12
aaaactctag atcgagaata atac 24

<210> 13
<211> 23
<212> DNA
<213> artificial sequence

<220>
<221> source
<223> /note="Description of artificial sequence: 5' mouse SCR13 primer"

<400> 13
aaaaagaatt ctcgaccaaa gcg 23

<210> 14
<211> 23
<212> DNA
<213> artificial sequence

<220>
<221> source
<223> /note="Description of artificial sequence: 3' mouse SCR13 primer"

<400> 14
aaaaatctag atcgagaatg gtg 23

<210> 15
<211> 28
<212> DNA
<213> artificial sequence

<220>
<221> source
<223> /note="Description of artificial sequence: 5' mouse SCR18-20 primer"

<400> 15
aaatcgatga aagataacag ctgcgttg 28

<210> 16
<211> 26
<212> DNA
<213> artificial sequence

<220>
<221> source
<223> /note="Description of artificial sequence: 3' mouse SCR18-20 primer"

<400> 16
aatctagacg cacgcaggtc ggatag 26

<210> 17
<211> 19
<212> DNA
<213> artificial sequence

<220>
<221> source
<223> /note="Description of artificial sequence: FV-plasma-titre upstream primer"

<400> 17
aagtctcccc ccgcctcta 19

<210> 18
<211> 21
<212> DNA
<213> artificial sequence

<220>
<221> source
<223> /note="Description of artificial sequence: FV-plasma-titre downstream primer"

<400> 18
agtgcctggt aagctccctg t 21

<210> 19
<211> 22
<212> DNA
<213> artificial sequence

<220>
<221> source
<223> /note="Description of artificial sequence: MMTV-quantification forward primer"

<400> 19

tcttttgcgc acaacccatc aa 22

<210> 20
<211> 21
<212> DNA
<213> artificial sequence

<220>
<221> source
<223> /note="Description of artificial sequence: MMTV-quantification reverse primer"

<400> 20
aaggccatgt ttgttaaggg c 21

<210> 21
<211> 188
<212> DNA
<213> artificial sequence

<220>
<221> source
<223> /note="Description of artificial sequence: human SCR7"

<400> 21

gaattcctca gaaaatgtta ttttccttat ttggaaaatg gatataatca aaattatgga 60

agaaagtttg tacagggtaa atctatagac gttgcctgcc atcctggcta cgctcttcca 120

aaagcgcaga ccacagttac atgtatggag aatggctggt ctcctactcc cagatgcatc 180

cctctaga 188

<210> 22
<211> 62
<212> PRT
<213> artificial sequence

<220>
<221> source
<223> /note="Description of artificial sequence: human SCR7"

<400> 22

```
Glu Phe Leu Arg Lys Cys Tyr Phe Pro Tyr Leu Glu Asn Gly Tyr Asn
1               5                   10                  15

Gln Asn Tyr Gly Arg Lys Phe Val Gln Gly Lys Ser Ile Asp Val Ala
            20                  25                  30

Cys His Pro Gly Tyr Ala Leu Pro Lys Ala Gln Thr Thr Val Thr Cys
        35                  40                  45

Met Glu Asn Gly Trp Ser Pro Thr Pro Arg Cys Ile Pro Leu
    50                  55                  60
```

<210> 23
<211> 191
<212> DNA
<213> artificial sequence

<220>
<221> source
<223> /note="Description of artificial sequence: human SCR9"

<400> 23

```
gaattcaaat cttgtgatat cccagtattt atgaatgcca gaactaaaaa tgacttcaca     60

tggtttaagc tgaatgacac attggactat gaatgccatg atggttatga aagcaatact    120

ggaagcacca ctggttccat agtgtgtggt tacaatggtt ggtctgattt acccatatgt    180

tatcctctag a                                                         191
```

<210> 24
<211> 63
<212> PRT
<213> artificial sequence

<220>
<221> source
<223> /note="Description of artificial sequence: human SCR9"

<400> 24

```
Glu Phe Lys Ser Cys Asp Ile Pro Val Phe Met Asn Ala Arg Thr Lys
1               5                   10                  15

Asn Asp Phe Thr Trp Phe Lys Leu Asn Asp Thr Leu Asp Tyr Glu Cys
            20                  25                  30

His Asp Gly Tyr Glu Ser Asn Thr Gly Ser Thr Thr Gly Ser Ile Val
        35                  40                  45

Cys Gly Tyr Asn Gly Trp Ser Asp Leu Pro Ile Cys Tyr Pro Leu
    50                  55                  60
```

<210> 25
<211> 188
<212> DNA
<213> artificial sequence

<220>
<221> source
<223> /note="Description of artificial sequence: human SCR13"

<400> 25

```
ggtaccgata aacttaagaa gtgcaaatca tcaaatttaa ttatacttga ggaacattta     60

aaaaacaaga aggaattcga tcataattct aacataaggt acagatgtag aggaaaagaa    120

ggatggatac acacagtctg cataaatgga agatgggatc cagaagtgaa ctgctcaatg    180

ggtctaga                                                             188
```

<210> 26
<211> 62
<212> PRT
<213> artificial sequence

<220>
<221> source
<223> /note="Description of artificial sequence: human SCR13"

<400> 26

```
Gly Thr Asp Lys Leu Lys Lys Cys Lys Ser Ser Asn Leu Ile Ile Leu
1               5                   10                  15

Glu Glu His Leu Lys Asn Lys Lys Glu Phe Asp His Asn Ser Asn Ile
            20                  25                  30

Arg Tyr Arg Cys Arg Gly Lys Glu Gly Trp Ile His Thr Val Cys Ile
        35                  40                  45

Asn Gly Arg Trp Asp Pro Glu Val Asn Cys Ser Met Gly Leu
    50                  55                  60
```

<210> 27
<211> 357
<212> DNA
<213> artificial sequence

<220>
<221> source
<223> /note="Description of artificial sequence: human SCR16-17"

<400> 27

```
gaattcggcc ttccttgtaa atctccacct gagatttctc atggtgttgt agctcacatg      60
tcagacagtt atcagtatgg agaagaagtt acgtacaaat gttttgaagg ttttggaatt     120
gatgggcctg caattgcaaa atgcttagga gaaaaatggt ctcaccctcc atcatgcata     180
aaaacagatt gtctcagttt acctagcttt gaaaatgcca tacccatggg agagaagaag     240
gatgtgtata aggcgggtga gcaagtgact tacacttgtg caacatatta caaaatggat     300
ggagccagta atgtaacatg cattaatagc agatggacag gaaggccaac atgcaga        357
```

<210> 28
<211> 121
<212> PRT
<213> artificial sequence

<220>
<221> source
<223> /note="Description of artificial sequence: human SCR16-17"

<400> 28

```
Glu Phe Gly Leu Pro Cys Lys Ser Pro Pro Glu Ile Ser His Gly Val
1               5                   10                  15

Val Ala His Met Ser Asp Ser Tyr Gln Tyr Gly Glu Glu Val Thr Tyr
            20                  25                  30
```

```
Lys Cys Phe Glu Gly Phe Gly Ile Asp Gly Pro Ala Ile Ala Lys Cys
        35                  40                  45

Leu Gly Glu Lys Trp Ser His Pro Pro Ser Cys Ile Lys Thr Asp Cys
    50                  55                  60

Leu Ser Leu Pro Ser Phe Glu Asn Ala Ile Pro Met Gly Glu Lys Lys
65                  70                  75                  80

Asp Val Tyr Lys Ala Gly Glu Gln Val Thr Tyr Thr Cys Ala Thr Tyr
                85                  90                  95

Tyr Lys Met Asp Gly Ala Ser Asn Val Thr Cys Ile Asn Ser Arg Trp
            100                 105                 110

Thr Gly Arg Pro Thr Cys Arg Gly Leu
        115                 120
```

<210> 29
<211> 575
<212> DNA
<213> artificial sequence

<220>
<221> source
<223> /note="Description of artificial sequence: human SCR18-20"

<400> 29

```
gaattcgaca cctcctgtgt gaatccgccc acagtacaaa atgcttatat agtgtcgaga      60

cagatgagta aatatccatc tggtgagaga gtacgttatc aatgtaggag cccttatgaa     120

atgtttgggg atgaagaagt gatgtgttta aatggaaact ggacggaacc acctcaatgc     180

aaagattcta caggaaaatg tgggccccct ccacctattg acaatgggga cattacttca     240

ttcccgttgt cagtatatgc tccagcttca tcagttgagt accaatgcca gaacttgtat     300

caacttgagg gtaacaagcg aataacatgt agaaatggat aatggtcaga accaccaaaa     360

tgcttacatc cgtgtgtaat atcccgagaa attatggaaa attataacat agcattaagg     420

tggacagcca aacagaagct ttattcgaga acaggtgaat cagttgaatt tgtgtgtaaa     480

cggggatatc gtctttcatc acgttctcac acattgcgaa caacatgttg ggatgggaaa     540

ctggagtatc caacttgtgc aaaaagacct ctaga                                575
```

<210> 30
<211> 191
<212> PRT
<213> artificial sequence

<220>
<221> source
<223> /note="Description of artificial sequence: human SCR18-20"

<400> 30

```
Glu Phe Asp Thr Ser Cys Val Asn Pro Pro Thr Val Gln Asn Ala Tyr
1               5                   10                  15

Ile Val Ser Arg Gln Met Ser Lys Tyr Pro Ser Gly Glu Arg Val Arg
            20                  25                  30

Tyr Gln Cys Arg Ser Pro Tyr Glu Met Phe Gly Asp Glu Glu Val Met
        35                  40                  45

Cys Leu Asn Gly Asn Trp Thr Glu Pro Pro Gln Cys Lys Asp Ser Thr
    50                  55                  60

Gly Lys Cys Gly Pro Pro Pro Pro Ile Asp Asn Gly Asp Ile Thr Ser
65                  70                  75                  80

Phe Pro Leu Ser Val Tyr Ala Pro Ala Ser Ser Val Glu Tyr Gln Cys
                85                  90                  95

Gln Asn Leu Tyr Gln Leu Glu Gly Asn Lys Arg Ile Thr Cys Arg Asn
            100                 105                 110

Gly Gln Trp Ser Glu Pro Pro Lys Cys Leu His Pro Cys Val Ile Ser
        115                 120                 125

Arg Glu Ile Met Glu Asn Tyr Asn Ile Ala Leu Arg Trp Thr Ala Lys
    130                 135                 140

Gln Lys Leu Tyr Ser Arg Thr Gly Glu Ser Val Glu Phe Val Cys Lys
145                 150                 155                 160

Arg Gly Tyr Arg Leu Ser Ser Arg Ser His Thr Leu Arg Thr Thr Cys
                165                 170                 175

Trp Asp Gly Lys Leu Glu Tyr Pro Thr Cys Ala Lys Arg Pro Leu
            180                 185                 190
```

<210> 31
<211> 345
<212> DNA
<213> artificial sequence

<220>

<221> source
<223> /note="Description of artificial sequence: aSCR"

<400> 31

```
atcgatgtta attaacctag gtgcgcacaa gtctggttct tcctccggta gaaagaaata     60
tggttccaag agaaagaagt ctggatcctc ttctggaaga aagaaatacg gtagtaagag    120
aaaaaaatcc ggttcctcct ccggaagtac tagaaaaaag tacggaagta aaagaaagaa    180
gtccggtagt tcctcaggaa gaaagaagta cggttcaaaa agaaaaaaga gtggatcctc    240
ctctggtagt actcgatcga ctagttcgcg aattgaaggt agaggttctg gtcatcatca    300
tcaccatcac ggttctggac atcaccacca tcatcatgcg gccgc                    345
```

<210> 32
<211> 114
<212> PRT
<213> artificial sequence

<220>
<221> source
<223> /note="Description of artificial sequence: aSCR"

<400> 32

```
Ser Met Leu Ile Asn Leu Gly Ala His Lys Ser Gly Ser Ser Ser Gly
1               5                   10                  15

Arg Lys Lys Tyr Gly Ser Lys Arg Lys Lys Ser Gly Ser Ser Ser Gly
            20                  25                  30

Arg Lys Lys Tyr Gly Ser Lys Arg Lys Lys Ser Gly Ser Ser Ser Gly
        35                  40                  45

Ser Thr Arg Lys Lys Tyr Gly Ser Lys Arg Lys Lys Ser Gly Ser Ser
    50                  55                  60

Ser Gly Arg Lys Lys Tyr Gly Ser Lys Arg Lys Lys Ser Gly Ser Ser
65                  70                  75                  80

Ser Gly Ser Thr Arg Ser Thr Ser Ser Arg Ile Glu Gly Arg Gly Ser
                85                  90                  95

Gly His His His His His His Gly Ser Gly His His His His His His
            100                 105                 110

Ala Ala
```

<210> 33
<211> 23
<212> DNA
<213> artificial sequence

<220>
<221> source
<223> /note="Description of artificial sequence: 5' human SCR7 primer"

<400> 33
aaaaagaatt cctcagaaaa tgt 23

<210> 34
<211> 20
<212> DNA
<213> artificial sequence

<220>
<221> source
<223> /note="Description of artificial sequence: 3' human SCR7 primer"

<400> 34
aaaatctaga gggatgcatc 20

<210> 35
<211> 24
<212> DNA
<213> artificial sequence

<220>
<221> source
<223> /note="Description of artificial sequence: 5' human SCR9 primer"

<400> 35
aaagaattca aatcttgtga tatc 24

<210> 36
<211> 23
<212> DNA
<213> artificial sequence

<220>
<221> source
<223> /note="Description of artificial sequence: 3' human SCR9 primer"

<400> 36
aaaatctaga ggataacata tgg 23

<210> 37
<211> 19
<212> DNA
<213> artificial sequence

<220>
<221> source
<223> /note="Description of artificial sequence: 5' human SCR13 primer"

<400> 37

gattgggtac cgacaagtt 19

<210> 38
<211> 18
<212> DNA
<213> artificial sequence

<220>
<221> source
<223> /note="Description of artificial sequence: 3' human SCR13 primer"

<400> 38
ggagctctag acccatgg 18

<210> 39
<211> 20
<212> DNA
<213> artificial sequence

<220>
<221> source
<223> /note="Description of artificial sequence: 5' human SCR16-17 primer"

<400> 39
aaagaattcg gccttccttg 20

<210> 40
<211> 21
<212> DNA
<213> artificial sequence

<220>
<221> source
<223> /note="Description of artificial sequence: 3' human SCR16-17 primer"

<400> 40
aatctagagg tctgcatgtt g 21

<210> 41
<211> 21
<212> DNA
<213> artificial sequence

<220>
<221> source
<223> /note="Description of artificial sequence: 5' human SCR18-20 primer"

<400> 41
aaaagaattc gacacctcct g 21

<210> 42
<211> 23
<212> DNA
<213> artificial sequence

<220>
<221> source
<223> /note="Description of artificial sequence: 3' human SCR18-20 primer"

<400> 42
aaaatctaga ggtctttttg cac 23

<210> 43
<211> 23
<212> DNA
<213> artificial sequence

<220>
<221> source
<223> /note="Description of artificial sequence: HIV-1-quantification upstream primer"

<400> 43
atgttaaaag agaccatcaa tga 23

<210> 44
<211> 20
<212> DNA
<213> artificial sequence

<220>
<221> source
<223> /note="Description of artificial sequence: HIV-1-quantification downstream primer"

<400> 44
ctatgtcact tccccttggt 20

<210> 45
<211> 24
<212> DNA
<213> artificial sequence

<220>
<221> source
<223> /note="Description of artificial sequence: MHV-quantification upstream primer"

<400> 45
tcctggtttt ctggcattac ccag 24

<210> 46
<211> 24
<212> DNA
<213> artificial sequence

<220>
<221> source
<223> /note="Description of artificial sequence: MHV-quantification downstream primer"

<400> 46
ctgaggcaat accgtgccgg gcgc 24